(19) Europäisches Patentamt
European Patent Office
Office européen des brevets

(11) EP 1 464 645 A1

(12) **EUROPEAN PATENT APPLICATION**
published in accordance with Art. 158(3) EPC

(43) Date of publication:
**06.10.2004 Bulletin 2004/41**

(21) Application number: **02788851.0**

(22) Date of filing: **17.12.2002**

(51) Int Cl.[7]: **C07D 487/04**, C07D 487/22, A61K 31/407, A61K 31/409, A61K 31/4196, A61K 31/422, A61K 31/4439, A61K 31/497, A61K 31/5377, A61K 31/541, A61P 25/00, A61P 35/00, A61P 43/00

(86) International application number:
**PCT/JP2002/013172**

(87) International publication number:
**WO 2003/051883 (26.06.2003 Gazette 2003/26)**

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(30) Priority: **18.12.2001 JP 2001384081**

(71) Applicant: **KYOWA HAKKO KOGYO CO., LTD.**
**Chiyoda-ku, Tokyo 100-8185 (JP)**

(72) Inventors:
- **KANAI, Fumihiko,**
  **c/o Kyowa Hakko Kogyo Co., Ltd.**
  **Tokyo 100-8185 (JP)**
- **MURAKATA, Chikara,**
  **c/o Kyowa Hakko Kogyo Co., Ltd.**
  **Sunto-gun, Shizuoka 411-8731 (JP)**
- **TSUJITA, Tetsuya,**
  **c/o Kyowa Hakko Kogyo Co., Ltd.**
  **Sunto-gun, Shizuoka 411-8731 (JP)**
- **YAMASHITA, Y.,**
  **c/o Kyowa Hakko Kogyo Co., Ltd.**
  **Sunto-gun, Shizuoka 411-8731 (JP)**
- **MIZUKAMI, Tamio,**
  **c/o Kyowa Hokko Kogyo Co., Ltd.**
  **Chiyoda-ku, Tokyo 100-8185 (JP)**
- **AKINAGA, Shiro,**
  **c/o Kyowa Hokko Kogyo Co., Ltd.**
  **Chiyoda-ku, Tokyo 100-8185 (JP)**

(74) Representative: **Tanner, James Percival et al**
**D. Young & Co,**
**21 New Fetter Lane**
**London EC4A 1DA (GB)**

(54) **INDOLE DERIVATIVE**

(57) There is provided an indole derivative represented by the above formula (I) or a pharmaceutically acceptable salt thereof which is useful for the treatment of malignant tumor or a brain neurodegenerative disease:

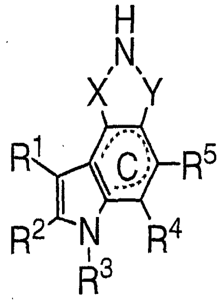

(I)

(wherein C ring represents a benzene ring or a cyclohexene ring; X and Y are the same or different and each represent $-CH_2-$, -CH(OH)-, $-CH(OR^X)-$, $-CH(SR^Y)-$ or carbonyl; $R^1$ and $R^2$ are the same ordifferent and each represent a hydrogen atom, substituted or unsubstituted lower alkyl, substituted or unsubstituted lower alkenyl or substituted or unsubstituted lower alkanoyl or $R^1$ and $R^2$ form a benzene ring together two carbon atoms each being adjacent thereto; and $R^3$, $R^4$ and $R^5$ are the same or different and each represent a hydrogen atom, substituted or unsubstituted lower alkyl, etc.)

EP 1 464 645 A1

## Description

### Technical Field

The present invention relates to indole derivatives or pharmaceutically acceptable salts thereof which are useful for the treatment of cancer or a brain neurodegenerative disease.

### Background of the Invention

Cyclin-dependent kinase 2 (CDK2) is an enzyme which forms a complex with cyclin E and catalyzes phosphorylation of specific residues of retinoblastoma (Rb) protein in the G1 phase of a cell cycle and it plays an important role in the progress of a cell cycle. CDK2 and cyclin E have been known to be highly expressed in breast cancer, lymphoma, etc. and their correlation to malignancy of cancer has been also reported [for example, Proceedings of the 57th Annual Meeting of the Japanese Cancer Association, page 20 (Presentation No. S06-4) (1998), etc.]. Therefore, CDK2 inhibitors are believed to be useful as anti-tumor agents.

On the other hand, CDK2 inhibitors have been reported to have an effect of suppressing the neuronal cell death caused by anti-tumor agents, cerebrovascular diseases, apoplexy, infarction, etc. (WO 99/43675). Therefore, CDK2 inhibitors are believed to be useful as therapeutic agents for a brain neurodegenerative disease.

With regard to compounds which inhibit CDK2, flavopiridol derivatives, olomoucine, roscovitine, purvalanol, UCN-01, etc. have been known [*Cancer Research*, Volume 56, page 2973 (1996); WO 97/42949; *Experimental Cell Research*, Volume 245, page 8 (1998); *Science*, Volume 281, page 533 (1998); and *Cancer Research*, Volume 57, page 1495 (1997)].

In addition, it has been known that, when cancer cells suffer from a damage of DNA by DNA-acting anti-tumor agents or antimetabolites, abrogating agents for accumulating action in G2 phase or S phase abrogate the action of repairing the damage of DNA (accumulating action at the G2 phase or S phase) by stopping the inherent cell cycle at the G2 phase or S phase of cancer cells, deprive of the chance for repairing the damage by promoting the progress of cell cycle and lead the cancer cells to apoptosis whereupon a synergic combination effect in the treatment of cancer is resulted [*Clinical Cancer Research*, Volume 2, page 791 (1996); *Cell Growth & Differentiation*, Volume 8, page 779 (1997); *Journal of National Cancer Institute*, Volume 88, page 956 (1996); and *Proceedings of American Association for Cancer Research*, Volume 39, page 70 (Presentation No. 476) (1998)].

With regard to the compounds having an abrogating action for the accumulating action at G2 phase and S phase as such, staurosporine derivatives, isogranulatimide [*Cancer Research*, Volume 58, page 5701 (1998)], caffeine [*Cancer Research*, Volume 55, page 1643 (1995)] and a peptide corresponding to from 211th to 221st amino acids form N-terminal of cdc 25C [*Cancer Research*, Volume 59, page 5887 (1999)] have been known. It has been further reported that, although K-252a has no abrogating action to accumulating action in the G2 phase, it has an abrogating action to accumulating action in the S phase [*Biochemical Pharmacology*, Volume 58, page 1713 (1999)].

Until now, with regard to indole derivatives, the compounds represented by the formula (II) are disclosed in the Japanese Published Unexamined Patent Application No. 202048/1993 or No. 178387/1992

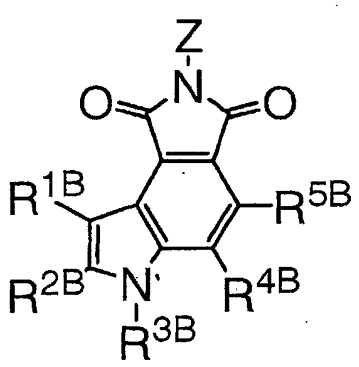

(II)

(wherein Z represents halogen, hydroxy, amino or lower alkyl which is substituted with halogen, hydroxy or amino; $R^{1B}$ and $R^{2B}$ are the same or different and each represent a hydrogen atom, carboxy, substituted or unsubstituted lower alkyl, substituted or unsubstituted lower alkenyl, substituted or unsubstituted lower alkanoyl or the like, or $R^{1B}$ and $R^{2B}$ form a benzene ring together with two carbon atoms adjacent to each of $R^{1B}$ and $R^{2B}$; $R^{3B}$ represents a hydrogen

atom, carbamoyl, substituted or unsubstituted lower alkyl, substituted or unsubstituted aralkyl, substituted or unsubstituted lower alkanoyl, substituted or unsubstituted aroyl or the like; (A) when $R^{3B}$ represents a hydrogen atom, substituted or unsubstituted lower alkyl, substituted or unsubstituted aralkyl, substituted or unsubstituted lower alkanoyl or substituted or unsubstituted aroyl, and when $R^{1B}$ and $R^{2B}$ are the same or different and each represent a hydrogen atom, substituted or unsubstituted lower alkyl, substituted or unsubstituted lower alkenyl or substituted or unsubstituted lower alkanoyl, then $R^{4B}$ and $R^{5B}$ are the same or different and each represent a hydrogen atom, hydroxy, substituted or unsubstituted lower alkyl, substituted or unsubstituted lower alkenyl, substituted or unsubstituted lower alkanoyl, substituted or unsubstituted aroyl, substituted or unsubstituted aryl or the like; and (B) when $R^{3B}$ represents a hydrogen atom, substituted or unsubstituted lower alkyl, carbamoyl, substituted or unsubstituted aralkyl, substituted or unsubstituted lower alkanoyl or substituted or unsubstituted aroyl, and when $R^{1B}$ and $R^{2B}$ form a benzene ring together with two carbon atoms adjacent to each of $R^{1B}$ and $R^{2B}$, then $R^{4B}$ and $R^{5B}$ are the same or different and each represent a hydrogen atom, substituted or unsubstituted lower alkyl, substituted or unsubstituted lower alkenyl, substituted or unsubstituted lower alkanoyl, substituted or unsubstituted aroyl, a substituted or unsubstituted aliphatic heterocyclic group, carbamoyloxy, amino or the like), and the compounds represented by the formula (III) are disclosed in US4,912,107 or US5,721,267.

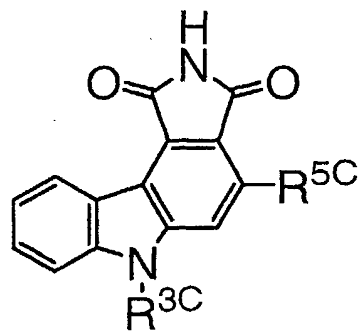

(III)

(wherein $R^{3C}$ represents a hydrogen atom, hydroxy, substituted or unsubstituted lower alkyl or the like; and $R^{5C}$ represents substituted or unsubstituted aryl or a substituted or unsubstituted heterocyclic group.)

Disclosure of the Invention

An object of the present invention is to provide indole derivatives or pharmaceutically acceptable salts thereof which are useful for the treatment of a malignant tumor or a brain neurodegenerative disease.
The present invention relates to the following (1) to (23).

(1) An indole derivative represented by the formula (I) or a pharmaceutically acceptable salt thereof:

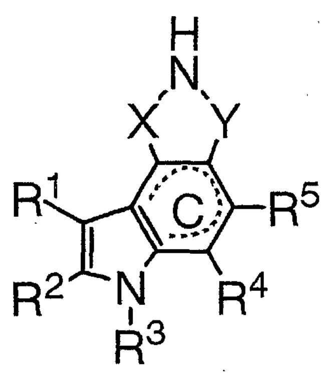

(I)

«wherein

C ring represents a benzene ring or a cyclohexene ring;
X and Y are the same or different and each represents $-CH_2-$, -CH(OH)-, $-CH(OR^X)-$ (wherein $R^X$ represents lower alkyl), $-CH(SR^Y)-$ (wherein $R^Y$ represents lower alkyl) or carbonyl;
$R^1$ and $R^2$ are the same or different and each represents a hydrogen atom, substituted or unsubstituted lower alkyl, substituted or unsubstituted lower alkenyl, or substituted or unsubstituted lower alkanoyl, or $R^1$ and $R^2$ form a benzene ring together with two carbon atoms, each being adjacent thereto, respectively; and
$R^3$ represents a hydrogen atom, substituted or unsubstituted lower alkyl, substituted or unsubstituted lower alkenyl, substituted or unsubstituted aralkyl, substituted or unsubstituted lower alkanoyl, substituted or unsubstituted aroyl, or is combined with $R^5$ to form, the formula (T)

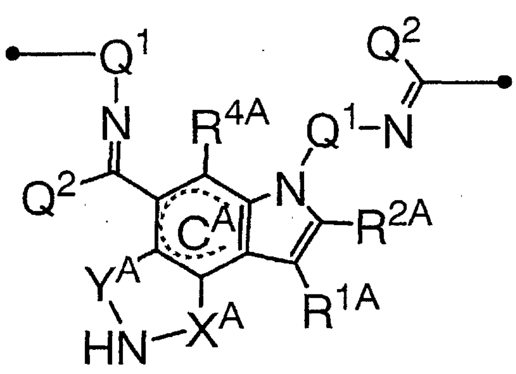

(T)

<wherein

$C^A$ ring represents a benzene ring or a cyclohexene ring;
$X^A$ and $Y^A$ are the same or different and each represents $-CH_2-$, -CH(OH)-, $-CH(OR^{XA})-$ (wherein $R^{XA}$ represents lower alkyl), $-CH(SR^{YA})-$ (wherein $R^{YA}$ represents lower alkyl) or carbonyl;
$R^{1A}$ and $R^{2A}$ are the same or different and each represents a hydrogen atom, substituted or unsubstituted lower alkyl, substituted or unsubstituted lower alkenyl, or substituted or unsubstituted lower alkanoyl, or $R^{1A}$ and $R^{2A}$ form a benzene ring together with two carbon atoms, each being adjacent thereto, respectively;
$R^{4A}$ represents a hydrogen atom, substituted or unsubstituted lower alkyl, substituted or unsubstituted lower alkenyl, substituted or unsubstituted lower alkanoyl, substituted or unsubstituted aroyl, substituted or unsubstituted aryl, a substituted or unsubstituted heterocyclic group, $-NR^6R^7$ [wherein $R^6$ and $R^7$ are the same or different and each represents a hydrogen atom, substituted or unsubstituted lower alkyl, lower alkoxycarbonyl or $-CONR^8R^9$ (wherein $R^8$ and $R^9$ are the same or different and each represents a hydrogen atom, substituted or unsubstituted lower alkyl, substituted or unsubstituted aryl, or a substituted or unsubstituted heterocyclic group)], $-COR^{10}$ {wherein $R^{10}$ represents hydroxy, lower alkoxy or $-NR^{11}R^{12}$ [wherein $R^{11}$ and $R^{12}$ are the same or different and each represents a hydrogen atom, substituted or unsubstituted lower alkyl, cycloalkyl, substituted or unsubstituted aralkyl, substituted or unsubstituted aryl, a substituted or unsubstituted heterocyclic group, lower alkylthio or an amino acid residue (wherein an amino group of the amino acid residue may be protected with a protective group) or $R^{11}$ and $R^{12}$ form a heterocyclic group together with the adjacent nitrogen atom]}, $-CO(NH)NR^{13}R^{14}$ [wherein $R^{13}$ and $R^{14}$ are the same or different and each represents a hydrogen atom or $-COR^{15}$ (wherein $R^{15}$ represents substituted or unsubstituted lower alkyl, substituted or unsubstituted aryl, or a substituted or unsubstituted heterocyclic group), or $R^{13}$ and $R^{14}$ form a heterocyclic group together with the adjacent nitrogen atom], $-CH=NOR^{16}$ (wherein $R^{16}$ represents a hydrogen atom, substituted or unsubstituted lower alkyl, substituted or unsubstituted lower alkenyl, substituted or unsubstituted aralkyl, substituted or unsubstituted aryl, or a substituted or unsubstituted heterocyclic group) or $-CH=NNR^{17}R^{18}$ [wherein $R^{17}$ and $R^{18}$ are the same or different and each represents a hydrogen atom, substituted or unsubstituted lower alkyl, substituted or unsubstituted aralkyl, substituted or unsubstituted aryl, a substituted or unsubstituted heterocyclic group or $-CONR^{11A}R^{12A}$ (wherein $R^{11A}$ and $R^{12A}$ are have same meanings as the above $R^{11}$ and $R^{12}$, respectively) or $R^{17}$ and $R^{18}$ form a heterocyclic group together with the

adjacent nitrogen atom];
$Q^1$ represents substituted or unsubstituted lower alkylene; and
$Q^2$ represents a hydrogen atom or substituted or unsubstituted lower alkyl>, provided that

(A): when $R^3$ represents a hydrogen atom, substituted or unsubstituted lower alkyl, substituted or unsubstituted lower alkenyl, substituted or unsubstituted aralkyl, substituted or unsubstituted lower alkanoyl, or substituted or unsubstituted aroyl, and also $R^1$ and $R^2$ are the same or different and each represents a hydrogen atom, substituted or unsubstituted lower alkyl, substituted or unsubstituted lower alkenyl or substituted or unsubstituted lower alkanoyl,

then $R^4$ and $R^5$ are the same or different and each represents a hydrogen atom, substituted or unsubstituted lower alkyl, substituted or unsubstituted lower alkenyl, substituted or unsubstituted lower alkanoyl, substituted or unsubstituted aroyl, substituted or unsubstituted aryl, a substituted or unsubstituted heterocyclic group, $-NR^{6A}R^{7A}$ (wherein $R^{6A}$ and $R^{7A}$ have the same meanings as the above $R^6$ and $R^7$, respectively), $-COR^{10A}$ (wherein $R^{10A}$ has the same meaning as the above $R^{10}$), $-CO(NH)NR^{13A}R^{14A}$ (wherein $R^{13A}$ and $R^{14A}$ have the same meanings as the above $R^{13}$ and $R^{14}$, respectively), $-CH=NOR^{16A}$ (wherein $R^{16A}$ has the same meaning as the above $R^{16}$) or $-CH=NNR^{17A}R^{18A}$ (wherein $R^{17A}$ and $R^{18A}$ have the same meanings as the above $R^{17}$ and $R^{18}$, respectively);

(B): when $R^3$ represents a hydrogen atom, substituted or unsubstituted lower alkyl, substituted or unsubstituted lower alkenyl, substituted or unsubstituted aralkyl, substituted or unsubstituted lower alkanoyl, or substituted or unsubstituted aroyl, and also when $R^1$ and $R^2$ form a benzene ring together with the two carbon atoms, each being adjacent thereto, respectively,

then $R^4$ and $R^5$ are the same or different and each represents a hydrogen atom, substituted or unsubstituted lower alkyl, substituted or unsubstituted lower alkenyl, substituted or unsubstituted lower alkanoyl, substituted or unsubstituted aroyl, a substituted or unsubstituted aliphatic heterocyclic group, oxazolinyl, $-NR^{6A}R^{7A}$ (wherein $R^{6A}$ and $R^{7A}$ have the same meanings as the above $R^6$ and $R^7$, respectively), $-COR^{10A}$ (wherein $R^{10A}$ has the same meaning as the above $R^{10}$), $-CO(NH)NR^{13A}R^{14A}$ (wherein $R^{13A}$ and $R^{14A}$ have the same meanings as the above $R^{13}$ and $R^{14}$, respectively), $-CH=NOR^{16A}$ (wherein $R^{16A}$ has the same meaning as the above $R^{16}$), or $-CH=NNR^{17A}R^{18A}$ (wherein $R^{17A}$ and $R^{18A}$ have the same meanings as the above $R^{17}$ and $R^{18}$, respectively); and

(C): when $R^3$ and $R^5$ are combined to form the formula (T), then $R^4$ represents a hydrogen atom, substituted or unsubstituted lower alkyl, substituted or unsubstituted lower alkenyl, substituted or unsubstituted lower alkanoyl, substituted or unsubstituted aroyl, substituted or unsubstituted aryl, a substituted or unsubstituted heterocyclic group, $-NR^{6A}R^{7A}$ (wherein $R^{6A}$ and $R^{7A}$ have the same meanings as the above $R^6$ and $R^7$, respectively), $-COR^{10A}$ (wherein $R^{10A}$ has the same meaning as the above $R^{10}$), $-CO(NH)NR^{13A}R^{14A}$ (wherein $R^{13A}$ and $R^{14A}$ have the same meanings as the above $R^{13}$ and $R^{14}$, respectively), $-CH=NOR^{16A}$ (wherein $R^{16A}$ has the same meaning as the above $R^{16}$) or $-CH=NNR^{17A}R^{18A}$ (wherein $R^{17A}$ and $R^{18A}$ have the same meanings as the above $R^{17}$ and $R^{18}$, respectively)>>.

(2) The indole derivative or the pharmaceutically acceptable salt thereof according to the above (1), wherein $R^3$ is a hydrogen atom, substituted or unsubstituted lower alkyl, substituted or unsubstituted lower alkenyl, substituted or unsubstituted aralkyl, substituted or unsubstituted lower alkanoyl or substituted or unsubstituted aroyl.
(3) The indole derivative or the pharmaceutically acceptable salt thereof according to the above (1), wherein $R^3$ is substituted or unsubstituted lower alkyl.
(4) The indole derivative or the pharmaceutically acceptable salt thereof according to any of the above (1) to (3), wherein $R^1$ and $R^2$ form a benzene ring together with the two carbon atoms, each being adjacent thereto, respectively.
(5) The indole derivative or the pharmaceutically acceptable salt thereof according to any of the above (1) to (3), wherein $R^1$ and $R^2$ are the same or different and each is a hydrogen atom, substituted or unsubstituted lower alkyl, substituted or unsubstituted lower alkenyl or substituted or unsubstituted lower alkanoyl.
(6) The indole derivative or the pharmaceutically acceptable salt thereof according to any of the above (1) to (5), wherein the C ring is a benzene ring.
(7) The indole derivative or the pharmaceutically acceptable salt thereof according to the above (1), wherein $R^3$ and $R^5$ are combined to form, together with the formula (T).
(8) An anti-tumor agent, which comprises the indole derivative or the pharmaceutically acceptable salt thereof according to any of the above (1) to (7) as an active ingredient.
(9) A therapeutic agent for a brain neurodegenerative disease, which comprises the indole derivative or the pharmaceutically acceptable salt thereof according to any of the above (1) to (7) as an active ingredient.
(10) An enhancer for activity of an anti-tumor agent, which comprises the indole derivative or the pharmaceutically

acceptable salt thereof according to any of the above (1) to (7) as an active ingredient.

(11) An inhibitor for a cyclin-dependent kinase 2 (CDK2), which comprises the indole derivative or the pharmaceutically acceptable salt thereof according to any of the above (1) to (7) as an active ingredient.

(12) An agent for abrogating accumulation action at G2 phase and/or S phase, which comprises the indole derivative or the pharmaceutically acceptable salt thereof according to any of the above (1) to (7) as an active ingredient.

(13) A pharmaceutical composition comprising the indole derivative or the pharmaceutically acceptable salt thereof according to any of the above (1) to (7) as an active ingredient.

(14) Use of the indole derivative or the pharmaceutically acceptable salt thereof according to any of the above (1) to (7) for the manufacture of an anti-tumor agent.

(15) Use of the indole derivative or the pharmaceutically acceptable salt thereof according to any of the above (1) to (7) for the manufacture of an enhancer for activity of an anti-tumor agent.

(16) Use of the indole derivative or the pharmaceutically acceptable salt thereof according to any of the above (1) to (7) for the manufacture of a therapeutic agent for a brain neurodegenerative disease.

(17) Use of the indole derivative or the pharmaceutically acceptable salt thereof according to any of the above (1) to (7) for the manufacture of an inhibitor for CDK2.

(18) Use of the indole derivative or the pharmaceutically acceptable salt thereof according to any of the above (1) to (7) for the manufacture of an agent for abrogating accumulation action at G2 phase and/or S phase.

(19) A method of treating a malignant tumor, which comprises administering an effective amount of the indole derivative or the pharmaceutically acceptable salt thereof according to any of the above (1) to (7).

(20) A method of treating a brain neurodegenerative disease, which comprises administering an effective amount of the indole derivative or the pharmaceutically acceptable salt thereof according to any of the above (1) to (7).

(21) A method of treating a disease in which CDK2 is concerned, which comprises administering an effective amount of the indole derivative or the pharmaceutically acceptable salt thereof according to any of the above (1) to (7).

(22) A method of enhancing activity for an anti-tumor agent which comprises administering an effective amount of the indole derivative or the pharmaceutically acceptable salt thereof according to any of the above (1) to (7).

(23) A method of abrogating accumulation action at G2 phase and/or S phase, which comprises administering an effective amount of the indole derivative or the pharmaceutically acceptable salt thereof according to any of the above (1) to (7).

Hereinafter, a compound which is represented by the formula (I) is referred to as Compound (I). The same is applied to compounds bearing other formula numbers as well.

In the definition of each group in the formula(I):

(i) Examples of the lower alkyl include $C_{1-9}$ straight or branched alkyl such as methyl, ethyl, propyl, isopropyl, butyl, isobutyl, sec-butyl, tert-butyl, pentyl, neopentyl, hexyl, heptyl, octyl and nonyl.

(ii) The lower alkylene has the same meaning as the above lower alkyl(i), wherein one hydrogen atom is removed.

(iii) A lower alkyl moiety in the lower alkoxy, lower alkylthio and lower alkoxycarbonyl has the same meaning as the above lower alkyl (i).

(iv) Examples of the cycloalkyl include $C_{3-6}$ cycloalkyl such as cyclopropyl, cyclobutyl, cyclopentyl and cyclohexyl.

(v) Examples of the lower alkenyl include $C_{2-6}$ alkenyl such as vinyl, allyl, butenyl, pentenyl and hexenyl.

(vi) Examples of the lower alkanoyl include $C_{1-8}$ straight orbranchedalkanoyl such as formyl, acetyl, propionyl, butyryl, isobutyryl,valeryl,isovaleryl,pivaloyl,hexanoyl,heptanoyl and octanoyl.

(vii) Examples of an aryl moiety in the aryl, aralkyl and aroyl include phenyl and naphthyl while an alkylene moiety for the aralkyl has the same meaning as the above lower alkylene (ii).

(viii) Examples of the aliphatic heterocyclic group include pyrrolidinyl, piperidinyl, piperazinyl, morpholinyl, thiomorpholinyl, oxazolinyl, dioxolanyl and tetrahydropyranyl.

Examples of the aromatic heterocyclic group include furyl, thienyl, pyrrolyl, pyridyl, imidazolyl, pyrazolyl, triazolyl, thiazolyl, oxazolyl, oxadiazolyl, pyrimidinyl, indolyl, benzothiazolyl, quinolyl, isoquinolyl and quinazolinyl.

Examples of the heterocyclic group include the groups which are mentioned in the above aliphatic heterocyclic group or aromatic heterocyclic group.

(ix) The heterocyclic group which is formed together with the adjacent nitrogen atom may contain an oxygen atom, a sulfur atom or an additional nitrogen atom and examples thereof include pyrrolidinyl, morpholino, thiomorpholino, N-methylpiperazinyl, pyrazolidinyl, piperidino, piperazinyl, homopiperazinyl, pyrrolyl, indolyl and isoindolyl.

(x) Examples of an amino acid moiety for the amino acid residue include α-amino acids such as glycine, alanine, proline, glutamic acid, lysine, serine, cysteine, phenylalanine and tyrosine. A protective group for the amino group of the amino acid residue may be any group so far as it is commonly used for a peptide synthesis and examples thereof include benzyloxycarbonyl and tert-butoxycarbonyl.

(xi) Substituent(s) for the substituted lower alkyl, substituted lower alkylene, substituted lower alkenyl and substituted lower alkanoyl is/are 1 to 3 substituent(s) which may be same or different and examples thereof are hydroxy, halogen, oxo, azido, lower alkoxy, aralkyloxy, substituted aralkyloxy [the substituent for the substituted aralkyloxy has the same meaning as the substituent (xiii) for a substituted aryl which is mentioned later], aryloxy, substituted aryloxy [the substituent for the substituted aryloxy has the same meaning as the substituent (xiii) for a substituted aryl which is mentioned later], mercapto, lower alkylthio, aralkylthio, substituted aralkylthio [the substituent for the substituted aralkylthio has the same meaning as the substituent (xiii) for substituted aryl which is mentioned later], arylthio, substituted arylthio [the substituent for the substituted arylthio has the same meaning as a substituent (xiii) for substituted aryl which is mentioned later], lower alkanoyl, aroyl, substituted aroyl [the substituent for the substituted aroyl has the same meanig as the substituent (xiii) for substituted aroyl which is mentioned later], carboxy, lower alkoxycarbonyl, aralkyloxycarbonyl, lower alkanoyloxy, p-toluenesulfonyloxy, methanesulfonyloxy, $-OCONR^{19}R^{20}$ (wherein $R^{19}$ and $R^{20}$ are the same or different and each represent a hydrogen atom, lower alkyl, aryl or heterocyclic group), aryl, substituted aryl [the substituent for the substituted aryl has the same meaning as the substituent (xiii) for substituted aryl which is mentioned later], a heterocyclic group, a substituted heterocyclic group [the substituent for the substituted heterocyclic group has the same meaning as the substituent (xiii) for a substituted heterocyclic group which is mentioned later], $-NR^{21}R^{22}$ <wherein $R^{21}$ and $R^{22}$ are the same or different and each represent a hydrogen atom, lower alkyl, substituted lower alkyl (an example of the substituent for substituted lower alkyl includes a heterocyclic group), hydroxy lower alkyl, di-lower alkylamino lower alkyl, cycloalkyl, aralkyl, aryl, substituted aryl [the substituent for the substituted aryl has the same meaning as the substituent (xiii) for substituted aryl which is mentioned later], a heterocyclic group, a substituted heterocyclic group [the substituent for the substituted heterocyclic group has the samemeaning as the substituent (xiii) for a substituted heterocyclic group which is mentioned later], $-COR^{23}$ {wherein $R^{23}$ represents lower alkyl, aryl, substituted aryl [the substituent for the substituted aryl has the same meaning as the substituent (xiii) for substituted aryl is mentioned later], a heterocyclic group or a substituted heterocyclic group [the substituent for the substituted heterocyclic group has the same meaning as the substituent (xiii) for a substituted heterocyclic group which is mentioned later]}, $-CONR^{24}R^{25}$ {wherein $R^{24}$ and $R^{25}$ are the same or different and each represent a hydrogen atom, hydroxy, lower alkyl, aralkyl, aryl, substituted aryl [the substituent for the substituted aryl has the same meaning as the substituent (xiii) for substituted aryl which is mentioned later], a heterocyclic group or a substituted heterocyclic group [the substituent for the substituted heterocyclic group has the same meaning as the substituent (xiii) for a substituted heterocyclic group which is mentioned later] or $R^{24}$ and $R^{25}$ form a heterocyclic group together with the adjacent nitrogen atom}, lower alkoxycarbonyl or aralkyloxycarbonyl or $R^{21}$ and $R^{22}$ form a heterocyclic group together with the adjacent nitrogen atom>, $-OR^{26}$ (wherein $R^{26}$ represents a heterocyclic group) or $-CONR^{24A}R^{25A}$ (wherein $R^{24A}$ and $R^{25A}$ has the same meaning as the above $R^{24}$ and $R^{25}$, respectively).

The above lower alkoxy, lower alkylthio, lower alkanoyl, aroyl, lower alkoxycarbonyl, lower alkyl, aryl, a heterocyclic group, a heterocyclic group formed together with the adjacent nitrogen atom, cycloalkyl and aralkyl have the same meaning as the above lower alkoxy (iii), lower alkylthio (iii), lower alkanoyl (vi), aroyl (vii), lower alkoxycarbonyl (iii), lower alkyl (i), aryl (vii), a heterocyclic group (viii), a heterocyclic group (ix) formed together with the adjacent nitrogen atom, cycloalkyl (iv) and aralkyl (vii), respectively; an aryl moiety of the aralkyloxy, aryloxy, aralkylthio, arylthio and aralkyloxycarbonyl has the same meaning as the above aryl (vii); an alkylene moiety of the aralkyloxy, aralkylthio and aralkyloxycarbonyl has the same meaning as the above lower alkylene (ii); a lower alkanoyl moiety of the lower alkanoyloxy has the same meaning as the above lower alkanoyl (vi); a lower alkylene moiety of the hydroxy lower alkyl has the same meaning as the above lower alkylene (ii); two lower alkyl moieties and an alkylene moiety of the di-lower alkylamino lower alkyl have the same meaning as the above lower alkyl (i) and lower alkylene (ii), respectively; and examples of the halogen (xii) include fluorine, chlorine, bromine and iodine atoms.

(xiii) Substituent(s) for the substituted aryl, substituted aralkyl, substituted aroyl, substituted aliphatic heterocyclic group and substituted heterocyclic group is/are 1 to 3 substituent(s) which may be the same or different and examples thereof include hydroxy, lower alkyl, lower alkenyl, lower alkanoyl, aroyl, halogen, trifluoromethyl, nitro, lower alkoxy, aralkyloxy, lower alkanoyloxy, aroyloxy, $-OCONR^{19A}R^{20A}$ (wherein $R^{19A}$ and $R^{20A}$ are the same as the above $R^{19}$ and $R^{20}$, respectively), $-NR^{27}R^{28}$ [wherein $R^{27}$ and $R^{28}$ are the same or different and each represent a hydrogen atom, lower alkyl, lower alkanoyl, aroyl, lower alkoxycarbonyl, aralkyloxycarbonyl or $-CONR^{29}R^{30}$ (wherein $R^{29}$ and $R^{30}$ have the same or different and each represent a hydrogen atom, lower alkyl or aryl)], carboxy, lower alkoxycarbonyl, $-CONR^{29A}R^{30A}$ (wherein $R^{29A}$ and $R^{30A}$ have the same meaning as the above $R^{29}$ and $R^{30}$, respectively) or cyano.

The above lower alkyl, lower alkoxy, lower alkoxycarbonyl, lower alkenyl, lower alkanoyl, aroyl, halogen and aryl have the same meaning as the above lower alkyl (i), lower alkoxy (iii), lower alkoxycarbonyl (iii), lower alkenyl (v), lower alkanoyl (vi), aroyl (vii), halogen (xii) and aryl (vii), respectively; an aryl moiety of the aralkyloxy, aroyloxy and

aralkyloxycarbonyl has the same meaning as the above aryl (vii); an alkylene moiety of the aralkyloxy and aralkyloxycarbonyl has the same meaning as the above lower alkylene (ii); and a lower alkanoyl moiety of the lower alkanoyloxy has the same meaning as the above lower alkanoyl (vi).

Examples of the disease in which CDK2 is concerned include cerebrovascular disease, apoplexy, infarction, brain neurodegenerative disease and malignant tumor. Examples of the malignant tumor include breast cancer, lymphoma, osteosarcoma and bladder cancer.

Pharmaceutically acceptable salts of the compound (I) include pharmaceutically acceptable acid addition salts, metal salts, ammonium salts, organic amine addition salts, amino acid addition salts, etc. Examples of the pharmaceutically acceptable acid addition salts include an inorganic acid salt such as hydrochloride, sulfate and phosphate and an organic acid salt such as acetate, maleate, fumarate, tartrate, citrate, lactate, aspartate, glutamate and succinate; examples of the pharmaceutically acceptable metal salts include an alkali metal salt such as sodium salt and potassium salt, an alkaline earth metal salt such as magnesium salt and calcium salt, aluminum salt and zinc salt; examples of the pharmaceutically acceptable ammonium salts include salts of ammonium and tetramethylammonium; examples of the pharmaceutically acceptable organic amine addition salts include addition salts of morpholine and piperazine; and examples of the pharmaceutically acceptable amino acid addition salts include addition salts of lysine, glycine and phenylalanine.

A process for producing the compound (I) is illustrated as hereunder.

When the defined group changes under the condition of the conducting process or is not appropriate for conducting the process in the following process for the production, it is subjected to the means which is commonly used in synthetic organic chemistry such as protection and deprotection of a functional group [e.g., "Protective Groups in Organic Synthesis" by T. W. Greene, John Wiley & Sons, Inc. (1981)] whereupon the object compound is able to be produced. If necessary, it is also possible to change the order of the reaction steps such as introduction of substituent(s).

The compound (I) is able to be produced by a series of reaction steps which is shown as follows.

Production Process 1

Among the compound (I), a compound (Ia) where C ring represents a cyclohexene ring, X and Y are carbonyl, $R^3$ represents $R^{3a}$ (wherein $R^{3a}$ represents a group defined as $R^3$ excluding the group which forms the above formula (T) together with $R^5$) and $R^5$ represents $R^{5a}$ (wherein $R^{5a}$ represents a group defined as $R^5$ excluding the group which forms the above formula (T) together with $R^3$) is able to be produced according to the following steps.

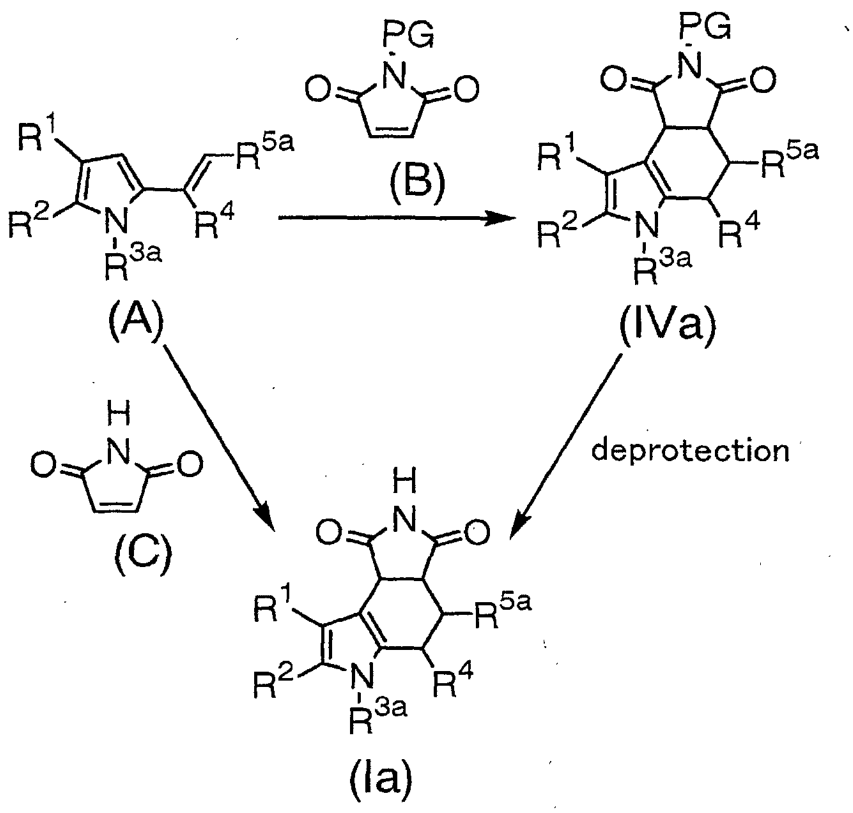

(wherein, PG represents a protective group such as trimethylsilyl and triphenylmethyl; and $R^1$, $R^2$, $R^{3a}$, $R^4$ and $R^{5a}$ each have the same meanings as defined above.)

Step 1

The compound (A) is allowed to react with the compound (B) or (C) in a solvent such as xylene, toluene or dichlorobenzene or without a solvent and, when the compound (B) having a protective group is used, the intermediate compound (IVa) is further subjected to deprotection according to a method according to a literature [e.g., "Protective Groups in Organic Synthesis" by T. W. Greene, John Wiley & Sons, Inc. (1981)], to give the compound (Ia).

The compound (B) or (C) is used in 1 to 20 equivalent(s) based on the compound (A). The reaction is usually carried out at the temperature between 60°C and 200°C and is completed within 1 minute to 48 hours.

Incidentally, the starting compound (A) is prepared, for example, by the following steps.

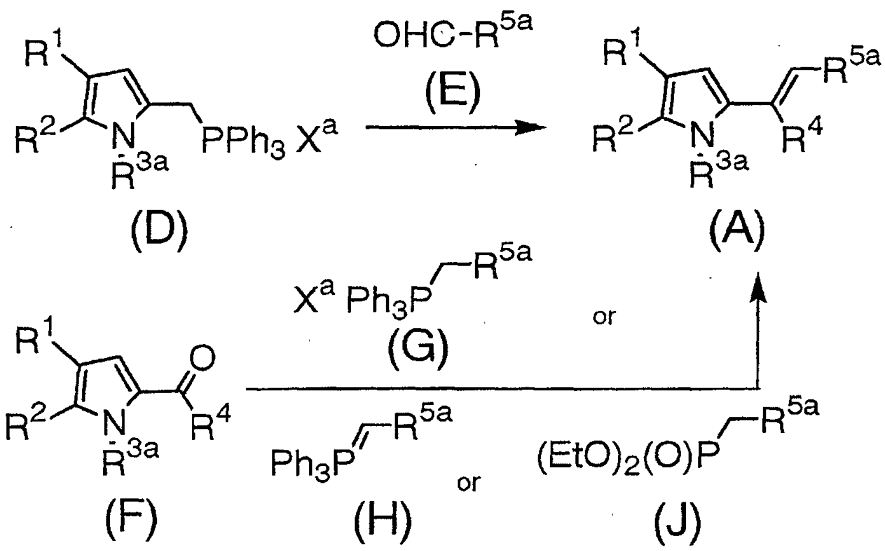

{wherein, PG, $R^1$, $R^2$, $R^{3a}$, $R^4$ and $R^{5a}$ each have the same meanings as defined above; Et represents ethyl; Ph represents phenyl; and $X^a$ represents halogen [the halogen has the same meaning as defined by the above halogen (xii)]}

The starting compound (A) is prepared by Wittig reaction of the compound (D) with the compound (E) or by Wittig reaction or Horner Emmons reaction of the compound (F) with the compound (G), (H) or (J) [e.g., *Canadian Journal of Chemistry*, volume 51, page 792 (1973); *Synthesis*, page 743 (1992); etc.]. The method for the preparation of the compounds (G), (H) and (J) is also described in the above literatures.

The compound (D) is prepared according to a known method [e.g., *The Journal of Organic Chemistry*, volume 52, page 19 (1987); *Canadian Journal of Chemistry*, volume 51, page 792 (1973); etc.] and the compound (F) is prepared according to a known method [e.g., *The Journal of Organic Chemistry*, volume 52, page 104 (1987); *Tetrahedron*, volume 50, page 6299 (1994)].

Production Process 2

In the compound (I), a compound (Ib) where C ring is a benzene ring, X and Y are carbonyl, $R^3$ is $R^{3a}$ (wherein $R^{3a}$ has the same meaning as defined above) and $R^5$ is $R^{5a}$ (wherein $R^{5a}$ has the same meaning as defined above) can be prepared from the above compound (Ia) or (IVa) according to the following steps.

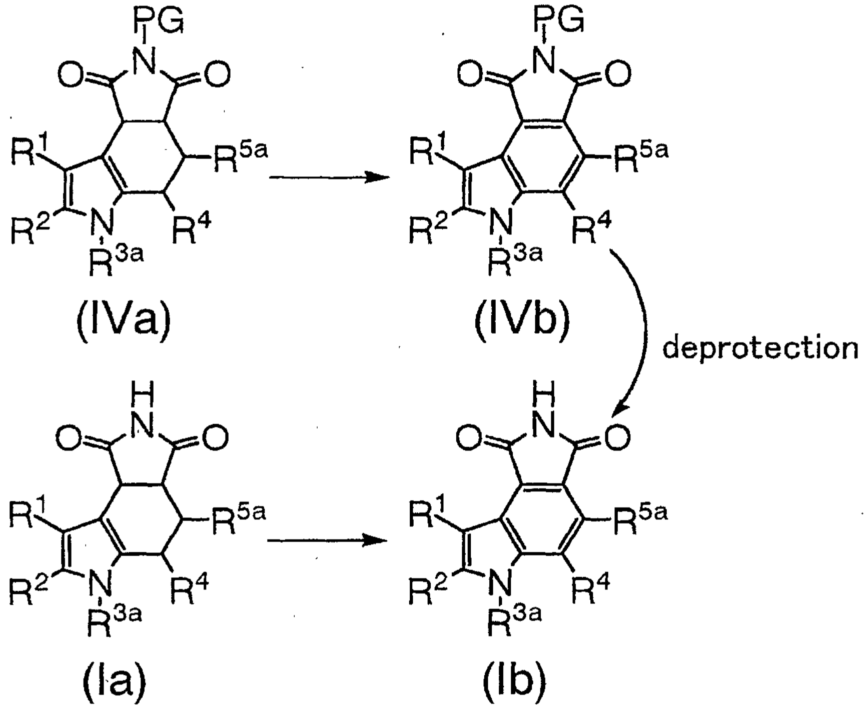


(wherein, PG, $R^1$, $R^2$, $R^{3a}$, $R^4$ and $R^{5a}$ each have the same meanings as defined above)

Step 2

A compound (Ia) or (IVa) which is prepared by the above production process 1 or by the production process 3 which is mentioned later is treated with an agent for removal of a hydrogen atom such as 2,3-dichloro-5,6-dicyano-1,4-benzoquinone (hereinafter, abbreviated as DDQ) or palladium carbon in a single solvent such as dichloromethane, ethyl acetate, toluene or dioxane or a mixed solvent thereof and, when there is a protective group, deprotection is carried out in the same manner as in the step 1, to give the compound (Ib).
The agent for removal of a hydrogen atom is used in 2 to 15 equivalents based on the compound (Ia) or (IVa).
The reaction is usually carried out at the temperature from -20°C to 180°C and is completed within 1 minute to 24 hours.

Production Process 3

Among compound (Ic) comprising the compound (Ia) prepared in the Production Process 1 and the compound (Ib) prepared in the Production Process 2, for example, each of the compounds which are mentioned as follows can also be produced from other compound (Ic) or the compound (IVa) prepared in the Production Process 1 and the compound (IVc) comprising the compound (IVb) prepared in the Production Process 2 by converting of functional group by the following steps.

Step 3-1

In the compound (Ic), a compound (Ic-2) where $R^{5a}$ is $R^{5a-2}$ {wherein $R^{5a-2}$ represents substituted aryl [the aryl has the same meaning as the aryl (vii) mentioned above and the substituent for the substituted aryl has the same meaning as the substituent (xiii) for the above substituted aryl] in which at least one of substituents is lower alkanoyloxy, aroyloxy or $-OCONR^{19A}R^{20A}$ (wherein $R^{19A}$ and $R^{20A}$ have the same meanings as defined above)} is prepared from a compound (Ic-1) or a compound (IVc-1) where $R^{5a}$ is $R^{5a-1}$ {wherein $R^{5a-1}$ represents substituted aryl in which at least one of substituents is hydroxy [the aryl has the same meaning as the above aryl (vii) and the substituent for the substituted aryl has the same meaning as the substituent (xiii) for the above substituted aryl]}.
The compound (Ic-2) is able to be prepared, by reacting the compound (Ic-1) or the compound (IVc-1) is

allowed to react with a carboxylic acid chloride such as acetyl chloride, valeryl chloride or benzoyl chloride or with an isocyanic acid derivative such as butyl isocyanate or phenyl isocyanate in the presence or absence of a base such as triethylamine or potassium carbonate in a single solvent such as dichloromethane, chloroform or tetrahydrofuran or a mixed solvent thereof and, if there is a protective group, carrying out deprotection in the same manner as in the step 1.

The carboxylic acid chloride or the isocyanic acid derivative is used in 1 to 30 equivalent(s) while the base is used in an amount of 0 to 250 equivalent(s) based on the compound (Ic-1) or the compound (IVc-1).

The reaction is usually carried out at the temperature of from -20°C to 60°C and is completed within 5 minutes to 24 hours.

Step 3-2

In the compound (Ic), a compound (Ic-4) where at least one of $R^1$ and $R^2$ is hydroxymethyl or $-CH_2NR^{21}R^{22}$ (wherein $R^{21}$ and $R^{22}$ each have the same meanings as defined above) is able to be prepared from a compound (Ic-3) or a compound (IVc-3) where at least one of $R^1$ and $R^2$ is formyl.

The compound (Ic-4) is able to be prepared, by treating the compound (Ic-3) or the compound (IVc-3) with a reducing agent such as sodium borohydride, sodium triacetoxyborohydride or sodium cyanoborohydride in the presence or absence of an acid such as acetic acid, in the presence or absence of $HNR^{21}R^{22}$ (wherein $R^{21}$ and $R^{22}$ each have the same meanings as defined above) in a single solvent such as 1,2-dichloroethane, tetrahydrofuran, methanol, ethanol or water or in a mixed solvent thereof and, if there is a protective group, carrying out deprotection in the same manner as in the step 1.

$HNR^{21}R^{22}$ is used in an amount of 0 to 20 equivalent(s) and the reducing agent is used in an amount of 1 to 20 equivalent(s) based on the compound (Ic-3) or the compound (IVc-3).

The reaction is usually carried out at the temperature of from -20°C to 40°C and is completed within 5 minutes to 24 hours.

Step 3-3

In the compound (Ic), a compound (Ic-6) where at least one of $R^1$, $R^2$, $R^4$ and $R^{5a}$ is substituted or unsubstituted lower alkenyl is prepared from a compound (Ic-5) or a compound (IVc-5) where at least one of $R^1$, $R^2$, $R^4$ and $R^{5a}$ is formyl.

A compound (Id) is able to be prepared, by reacting the compound (Ic-5) or the compound (IVc-5) is allowed to react with $R^{31}CH_2PPh_3X^b$ {wherein $R^{31}$ represents substitute or unsubstituted lower alkyl [the lower alkyl has the same meaning as the above lower alkyl (i) and the substituent for the substituted lower alkyl has the same meaning as the substituent (xi) for the above substituted lower alkyl] or a substituent (xi) which is defined as the substituent for the above substituted lower alkenyl and $X^b$ represents halogen [the halogen has the same meaning as in the above halogen (xii)] and Ph has the same meaning as defined above} or $R^{31}CH=PPh_3$ (wherein each of $R^{31}$ and Ph has the same meaning as defined above) in a single solvent such as 1,2-dichloroethane, toluene, tetrahydrofuran or N,N-dimethylformamide (DMF) or a mixed solvent thereof and in the presence or absence of a base and, if there is a protective group, carrying out deprotection in the same manner as in the case of step 1.

With regard to the base, n-butyl lithium, sodium hydride, potassium carbonate, etc. may be used.

The above $R^{31}CH_2PPh_3X^b$ or $R^{31}CH=PPh_3$ is used in an amount of 1 to 10 equivalent(s) and the base is used in an amount of 0 to 10 equivalent(s) based on the compound (Ic) or the compound (IVc).

The reaction is usually carried out at a temperature of from -20°C to 120°C and is completed within 5 minutes to 24 hours.

Step 3-4

In the compound (Ic), a compound (Ic-8) where at least one of $R^4$ and $R^{5a}$ is $-COR^{10B}$ (wherein $R^{10B}$ has the same meaning where hydroxy is removed from the definition for the above $R^{10}$) or $-CONHNR^{13}R^{14}$ (wherein $R^{13}$ and $R^{14}$ each have the same meanings as defined above) is able to be prepared from a compound (Ic-7) or a compound (IVc-7) where at least one of $R^4$ and $R^{5a}$ is carboxy.

The compound (Ic-8) is able to be prepared, by reacting the compound (Ic-7) or the compound (IVc-7) with thionyl chloride, phosphorus pentachloride, ethyl chloroformate, isobutyl chloroformate, diphenylphosphoric acid azide, etc. in the presence or absence of a base such as triethylamine, in a single solvent such as dichloromethane, 1,2-dihloroethane, tetrahydrofuran, 1,4-dioxane or DMF or a mixed solvent thereof, or with the corresponding lower alcohol [a lower alkyl moiety in the lower alcohol has the same meaning of the above lower alkyl (i) as defined above], $HNR^{11}R^{12}$ (wherein $R^{11}$ and $R^{12}$ each have the same meaning as defined above) or $H_2NNR^{13}R^{14}$ (wherein $R^{13}$ and $R^{14}$ each have the same meaning as defined above) in the co-presence of a dehydrating condensation agent such as dicyclohexyl

carbodiimide or water-soluble carbodiimide and, after the reaction, carrying out deprotection in the same manner as in the step 1 if there is a protective group.

Thionyl chloride, phosphorus pentachloride, ethyl chloroformate, isobutyl chloroformate, diphenylphosphoric acid azide, dicyclohexyl carbodiimide or water-soluble carbodiimide, lower alcohol, $HNR^{11}R^{12}$ and $H_2NNR^{13}R^{14}$ each are used in an amount of 1 to 20 equivalent(s) and the base is used in an amount of 0 to 20 equivalent(s) based on the compound (Ic-7) or the compound (IVc-7).

The reaction is usually carried out at the temperature of from -20°C to 80°C and is completed within 30 minutes to 24 hours.

Step 3-5

In the compound (Ic), a compound (Ic-10) where at least one of $R^4$ and $R^{5a}$ is lower alkyl substituted with $-OCONR^{19}R^{20}$ (wherein $R^{19}$ and $R^{20}$ each have the same meanings as defined above) [a lower alkylene moiety in the substituted lower alkyl has the same meaning as defined by the above lower alkylene (ii)] is prepared from a compound (Ic-9) or a compound (IVc-9) where at least one of $R^4$ and $R^{5a}$ is hydroxy lower alkyl [a lower alkylene moiety of the hydroxy lower alkyl has the same meaning as defined by the above lower alkylene (ii)].

The compound (Ic-10) is able to be prepared by reacting a compound (Ic-9) or a compound (IVc-9) with an isocyanic acid derivative such as butyl isocyanate or phenyl isocyanate in the presence or absence of a base such as triethylamine or potassium carbonate in a single solvent of dichloromethane, chloroform or tetrahydrofuran or a mixed solvent thereof and, if there is a protective group, carrying out deprotection in the same manner as in the step 1.

The isocyanic acid derivative is used in an amount of 1 to 10 equivalent(s) and the base is used in an amount of 0 to 10 equivalent(s) based on the compound (Ic-10) or the compound (IVc-10).

The reaction is usually carried out at -20 to 60°C and is completed within 5 minutes to 24 hours.

Step 3-6

In the compound (Ic), a compound (Ic-11) where at least one of $R^4$ and $R^{5a}$ is halogenated lower alkyl [halogen and lower alkylene moieties of the halogenated lower alkyl are the same as those defined by the above halogen (xii) and lower alkylene (ii), respectively] is prepared from a compound (Ic-9) or a compound (IVc-9) where at least one of $R^4$ and $R^{5a}$ is hydroxy lower alkyl.

A compound (Ic-11) is able to be prepared by reacting the compound (Ic-9) or the compound (IVc-9) is made to react with halogen such as bromine, chlorine or iodine in the presence of, for example, triphenylphosphine in a solvent such as DMF and, if there is a protective group, carrying out deprotection in the same manner as in the step 1.

Each of the triphenylphosphine or the like and halogen is used in an amount of 1 to 10 equivalent(s) based on the compound (Ic-9) or the compound (IVc-9).

Usually, the reaction is carried out at a temperature of from -20°C to 60°C and is completed within 5 minutes to 24 hours.

Step 3-7

In the compound (Ic), a compound (Ic-12) where at least one of $R^4$ and $R^{5a}$ is lower alkyl substituted with $-NR^{21}R^{22}$ (wherein $R^{21}$ and $R^{22}$ each have the same meanings as defined above) [a lower alkylene moiety of the substituted lower alkyl has the same meanings as defined in the above lower alkylene (ii) above] is prepared from a compound (Ic-11) or a compound (IVc-11) where at least one of $R^4$ and $R^{5a}$ is halogenated lower alkyl.

The compound (Ic-12) is able to be prepared, by reacting the compound (Ic-11) or the compound (IVc-11) with $HNR^{21}-R^{22}$ (wherein $R^{21}$ and $R^{22}$ each have the same meanings as defined above) in a single solvent such as dichloromethane and DMF or a mixed solvent thereof and, if there is a protective group, carrying out deprotection in the same manner as in the step 1.

$HNR^{21}R^{22}$ is used in an amount of 1 to 10 equivalent(s) based on the compound (Ic-11) or the compound (IVc-11).

Usually the reaction is carried out at the temperature of from 0°C to 150°C and is completed within 5 minutes to 24 hours.

Step 3-8

In the compound (Ic), a compound (Ic-14) where at least one of $R^4$ and $R^{5a}$ is 2-oxazolinyl is prepared from a compound (Ic-13) or a compound (IVc-13) where at least one of $R^4$ and $R^{5a}$ is hydroxyethylcarbamoyl.

The compound (Ic-14) is able to be prepared, by treating the compound (Ic-13) or the compound (IVc-13)

with a Burgess reagent ($CH_3OCONSO_2N(C_2H_5)_3$) according to *Tetrahedron Letters*, volume 33,, page 907 (1992) in a solvent such as tetrahydrofuran and, if there is a protective group, carrying out deprotection in the same manner as in the step 1.

The Burgess reagent is used in an amount of 0.1 to 10 equivalent(s) based on the compound (Ic-13) or the compound (IVc-13).

Usually, the reaction is carried out at a temperature of from 20°C to 60°C and is completed within 5 minutes to 24 hours.

Step 3-9

In the compound (Ic), a compound (Ic-15) where at least one of $R^4$ and $R^{5a}$ is amino or lower alkoxycarbonylamino [a lower alkyl moiety of the lower alkoxycarbonylamino has the same meaning as defined in the above lower alkyl (i)] is prepared from a compound (Ic-7) or a compound (IVc-7) where at least one of $R^4$ and $R^{5a}$ is carboxy.

The compound (Ic-15) is able to be prepared, by reacting the compound (Ic-7) or the compound (IVc-7) with diphenylphosphoric acid azide or the like in a single solvent such as tetrahydrofuran, ethanol or water or a mixed solvent thereof and then water or the corresponding lower alcohol [a lower alkyl moiety of the lower alcohol has the same meaning as defined in the above lower alkyl (i)] and, if there is a protective group, carrying out deprotection in the same manner as in the step 1.

Diphenylphosphoric acid azide is used in an amount of 1 to 10 equivalent(s) based on the compound (Ic-7) or the compound (IVc-7).

Usually, the reaction is carried out at a temperature of from 60°C to 100°C and is completed within 30 minutes to 24 hours.

Step 3-10

In the compound (Ic), a compound (Ic-16) where at least one of $R^4$ and $R^{5a}$ is tetrahydropyranyloxy lower alkyl [a lower alkylene moiety of the tetrahydropyranyloxy lower alkyl has the same meaning as defined in the above lower alkylene (ii)] is prepared from a compound (Ic-9) or a compound (IVc-9) where at least one of $R^4$ and $R^{5a}$ is hydroxy lower alkyl.

The compound (Ic-16) is able to be prepared, reacting the compound (Ic-9) or the compound (IVc-9) with dihydropyran in the presence of an acid catalyst such as pyridinium p-toluenesulfonate in a solvent such as dichloromethane and, if there is a protective group, carrying out deprotection in the same manner as in the step 1.

The acid catalyst is used in an amount of 0.1 to 1 equivalent and dihydropyran is used in an amount of 1 to 10 equivalent (s) based on the compound (Ic-9) or the compound (IVc-9).

Usually, the reaction is carried out at a temperature of from 20°C to 60°C and is completed within 30 minutes to 24 hours.

Step 3-11

In the compound (Ic), a compound (Ic-17) where at least one of $R^4$ and $R^{5a}$ is $-NHCONR^8R^9$ (wherein $R^8$ and $R^9$ each have the same meanings as defined above) is prepared from a compound (Ic-7) or a compound (IVc-7) where at least one of $R^4$ and $R^{5a}$ is carboxy.

The compound (Ic-17) is able to be prepared, reacting the compound (Ic-7) or the compound (IVc-7) with diphenylphosphoric acid azide or the like in a solvent such as DMF followed by being made to react with $HNR^8R^9$ (wherein $R^8$ and $R^9$ each have the same meanings as defined above) and, if there is a protective group, carrying out deprotection in the same manner as in the step 1.

Diphenylphosphoric acid azide or the like and $HNR^8R^9$ each are used in an amount of 1 to 10 equivalent(s) based on the compound (Ic-7) or the compound (IVc-7).

Usually, the reaction is carried out at a temperature of from 0°C to 100°C and is completed within 30 minutes to 24 hours.

Step 3-12

In the compound (Ic), a compound (Ic-19) where at least one of $R^4$ and $R^{5a}$ is formyl is prepared from a compound (Ic-18) or a compound (IVc-18) where at least one of $R^4$ and $R^{5a}$ is acetoxymethyl.

The compound (Ic-19) is able to be prepared, by treating the compound (Ic-18) or the compound (IVc-18) with an oxidizing agent such as DDQ in a single solvent such as dichloromethane or 1,4-dioxane or a mixed solvent thereof and, if there is a protective group, carrying out deprotection in the same manner as in the step 1.

The oxidizing agent is used in an amount of 1 to 10 equivalent(s) based on the compound (Ic-18) or the compound (IVc-18).

Usually, the reaction is carried out at a temperature of from 20°C to 120°C and is completed within 30 minutes to 24 hours.

Step 3-13

In the compound (Ic), a compound (Ic-20) where at least one of $R^4$ and $R^{5a}$ is $-CH_2NR^{21}R^{22}$ (wherein $R^{21}$ and $R^{22}$ each have the same meanings as defined above) is prepared from a compound (Ic-19) or a compound (IVc-19) where at least one of $R^4$ and $R^{5a}$ is formyl.

The compound (Ic-20) is able to be prepared, by treating the compound (Ic-19) or the compound (IVc-19) with a reducing agent such as sodium triacetoxyborohydride or sodium cyanoborohydride in the presence of $H_2NR^{21}R^{22}$ (wherein $R^{21}$ and $R^{22}$ each has the same meaning as defined above) in a single solvent such as 1,2-dichloroethane, tetrahydrofuran, methanol, ethanol or water or a mixed solvent thereof and, if there is a protective group, carrying out deprotection in the same manner as in the step 1.

$H_2NR^{21}R^{22}$ and the reducing agent each are used in an amount of 1 to 10 equivalent (s) based on the compound (Ic-19) or the compound (IVc-19).

Usually, the reaction is carried out at a temperature of from 0°C to 40°C and is completed within 5 minutes to 24 hours.

Step 3-14

In the compound (Ic), a compound (Ic-21) where at least one of $R^4$ and $R^{5a}$ is $-CH=NOR^{16}$ (wherein $R^{16}$ has the same meaning as defined above) or $-CH=NNR^{17}R^{18}$ (wherein $R^{17}$ and $R^{18}$ each have the same meanings as defined above) is prepared from a compound (Ic-19) or a compound (IVc-19) where at least one of $R^4$ and $R^{5a}$ is formyl.

The compound (Ic-21) is able to be prepared, by reacting the compound (Ic-19) or the compound (IVc-19) with $H_2NOR^{16}$ (wherein $R^{16}$ has the same meaning as defined above) or $H_2NNR^{17}R^{18}$ (wherein $R^{17}$ and $R^{18}$ each have the same meanings as defined above) in the presence or absence of a base such as triethylamine in a single solvent such as tetrahydrofuran, ethanol or DMF or a mixed solvent thereof and, if there is a protective group, carrying out deprotection in the same manner as in the step 1.

The base is used in an amount of 0 to 10 equivalent(s) and $H_2NOR^{16}$ or $H_2NNR^{17}R^{18}$ each are used in an amount of 1 to 10 equivalents based on the compound (Ic-19) or the compound (IVc-19).

Usually, the reaction is carried out at a temperature of from 0°C to 40°C and is completed within 5 minutes to 24 hours.

Step 3-15

In the compound (Ic), a compound (Ic-22) where at least one of $R^4$ and $R^{5a}$ is lower alkyl azide [a lower alkylene moiety of the lower alkyl azide has the same meaning of the lower alkylene (ii) defined above] is prepared from a compound (Ic-9) or a compound (IVc-9) where at least one of $R^4$ and $R^{5a}$ is hydroxy lower alkyl.

The compound (Ic-22) is able to be prepared, by reacting the compound (Ic-9) or the compound (IVc-9) with bis(p-nitrophenyl)phosphoric acid azide or the like in the presence of a base such as 1,8-diazabicyclo[5,4,0]undec-7-ene (DBU) in a solvent such as tetrahydrofuran and, if there is a protective group, carrying out deprotection in the same manner as in the step 1.

The base and bis(p-nitrophenyl)phosphoric acid azide or the like each are used in an amount of 1 to 10 equivalents based on the compound (Ic-9) or the compound (IVc-9).

Usually, the reaction is carried out at a temperature of from 0°C to 100°C and is completed within 30 minutes to 24 hours.

Step 3-16

In the compound (Ic), a compound (Ic-23) where at least one of $R^4$ and $R^{5a}$ is amino lower alkyl [a lower alkylene moiety of the amino lower alkyl has the same meaning of the above lower alkylene (ii) defined above] is prepared from a compound (Ic-22) or a compound (IVc-22) where at least one of $R^4$ and $R^{5a}$ is lower alkyl azide.

The compound (Ic-23) is able to be prepared, by treating the compound (Ic-22) or the compound (IVc-22) with a reducing agent such as triphenylphosphine or lithium aluminum hydride in a single solvent such as tetrahydrofuran, DMF, ethanol or water or a mixed solvent thereof or by subjecting the compound (Ic-22) or the compound (IVc-22) to a catalytic reduction in the presence of a catalyst such as palladium carbon in a single solvent such as tetrahydrofuran, DMF, ethanol or water or a mixed solvent thereof and, if there is a protective group, deprotection is carried out by the same manner as in the step 1.

The reducing agent is used in an amount of 1 to 10 equivalent(s) and the catalyst for the catalytic reduction is used in an amount of 5 to 100% by weight to the compound (Ic-22) or the compound (IVc-22).

Usually, the reaction is carried out at a temperature of from 0°C to 100°C and is completed within 30 minutes to 24 hours.

Step 3-17

In the compound (Ic), a compound (Ic-25) where $R^{3a}$ is hydroxypropyl is prepared from a compound (Ic-24) or a compound (IVc-24) where $R^{3a}$ is allyl.

The compound (Ic-25) is able to be prepared, by treating the compound (Ic-24) or the compound (IVc-24) with a reducing agent such as a boran-dimethyl sulfide complex, a 9-borabicyclononane dimer or diethyl boran in a solvent such as tetrahydrofuran and, if there is a protective group, carrying out deprotection in the same manner as in the step 1.

The reducing agent is used in an amount of 1 to 10 equivalent (s) to the compound (Ic-24) or the compound (IVc-24).

Usually, the reaction is carried out at a temperature of from -20°C to 40°C and is completed within 30 minutes to 24 hours.

Step 3-18

In the compound (Ic), a compound (Ic-27) where $R^{3a}$ is carboxymethyl is prepared from a compound (Ic-26) or a compound (IVc-26) where $R^{3a}$ is benzyloxycarbonylmethyl.

The compound (Ic-27) is able to be prepared, by subjecting the compound (Ic-26) or the compound (IVc-26) to a catalytic reduction in the presence of a reducing catalyst such as palladium carbon in a single solvent such as ethyl acetate, DMF, toluene, ethanol, tetrahydrofuran or water or a mixed solvent thereof and, if there is a protective group, carrying out deprotection in the same manner as in the step 1.

The reducing catalyst is used in an amount of 10 to 100 % by weight based on the compound (Ic-26) or the compound (IVc-26).

Usually, the reaction is carried out at a temperature of from 20°C to 120°C and is completed within 3 minutes to 72 hours.

Step 3-19

In the compound (Ic), a compound (Ic-29) where $R^{3a}$ is hydroxyethyl is prepared from a compound (Ic-28) or a compound (IVc-28) where $R^{3a}$ is carboxymethyl.

The compound (Ic-29) is able to be prepared, by treating the compound (Ic-28) or the compound (IVc-28) with a reducing agent such as boran dimethyl sulfide complex in a solvent such as tetrahydrofuran and, if there is a protective group, carrying out deprotection in the same manner as in the step 1.

The reducing agent is used in an amount of 0.5 to 10 equivalent(s) based on the compound (Ic-28) or the compound (IVc-28).

Usually, the reaction is carried out at a temperature of from -20°C to 60°C and is completed within 5 minutes to 24 hours.

Step 3-20

In the compound (Ic), a compound (Ic-31) where $R^{3a}$ is lower alkanoyloxy lower alkyl [a lower alkanoyl moiety and a lower alkylene moiety of the lower alkanoyloxy lower alkyl have the same meanings as defined in the above lower alkanoyl (vi) and lower alkylene (ii), respectively] is prepared from a compound (Ic-30) or a compound (IVc-30) where $R^{3a}$ is hydroxy lower alkyl [a lower alkylene moiety of the hydroxy lower alkyl has the same meaning as defined in the above lower alkylene (ii)].

The compound (Ic-31) is able to be prepared, by reacting the compound (Ic-30) or the compound (IVc-30) with an acylating agent such as acetic anhydride in the presence or absence of a base such as triethylamine in a single solvent such as dichloromethane or pyridine or a mixed solvent thereof and, if there is a protective group, carrying out deprotection in the same manner as in the step 1.

The base is used in an amount of 0 to 10 equivalent(s) and the acylating agent is used in an amount of 1 to 10 equivalent(s) based on the compound (Ic-30) or the compound (IVc-30).

Usually, the reaction is carried out at a temperature of from -20°C to 60°C and is completed within 5 minutes to 24 hours.

Step 3-21

In the compound (Ic), a compound (Ic-32) where $R^{3a}$ is halogenated lower alkyl [halogen moiety and a lower alkylene moiety in the halogenated lower alkyl has the same meanings as defined in the above halogen (xii) and lower alkylene (ii)] is prepared from a compound (Ic-30) or a compound (IVc-30) where $R^{3a}$ is hydroxy lower alkyl.
The compound (Ic-32) is able to be prepared, by reacting the compound (Ic-30) or the compound (IVc-30) with halogen such as bromine, chlorine or iodine in the presence of triphenylphosphine or the like in a solvent such as DMF and, if there is a protective group, and carrying out deprotection in the same manner as in the step 1.
Triphenylphosphine or the like and the halogen each are used in an amount of 1 to 10 equivalents based on the compound (Ic-30) or the compound (IVc-30).
Usually, the reaction is carried out at a temperature of from -20°C to 60°C and is completed within 5 minutes to 24 hours.

Step 3-22

In the compound (Ic), a compound (Ic-34) where $R^{3a}$ is lower alkyl substituted with $-NR^{21}R^{22}$ (wherein $R^{21}$ and $R^{22}$ each have the same meaning as defined above) [a lower alkylene moiety of the substituted lower alkyl has the same meaning as defined in the above lower alkylene (ii)] is prepared from a compound (Ic-33) or a compound (IVc-33) where $R^{3a}$ is a hydrogen atom.
The compound (Ic-34) is able to be prepared, by reacting the compound (Ic-33) or the compound (IVc-33) with $X^cR^{32}NR^{21}R^{22}$ {wherein $R^{21}$ and $R^{22}$ each have the same meanings as defined above; $X^c$ has the same meaning as the above $X^b$; and $R^{32}$ is lower alkylene [the lower alkylene has the same meaning as the above lower alkylene (ii)]} in the presence of a base such as potassium carbonate or potassium tert-butoxide in a single solvent such as dichloromethane or DMF or a mixed solvent thereof and, if there is a protective group, carrying out deprotection in by the same manner as in the step 1.
$X^cR^{32}NR^{21}R^{22}$ and the base each are used an amount of 1 to 10 equivalents based on the compound (Ic-33) or the compound (IVc-33).
Usually, the reaction is carried out at a temperature of from 0°C to 100°C and is completed within 5 minutes to 24 hours.

Step 3-23

In the compound (Ic), a compound (Ic-35) where $R^{3a}$ is 2,3-dihydroxypropyl is prepared from a compound (Ic-24) or a compound (IVc-24) where $R^{3a}$ is allyl.
The compound (Ic-35) is able to be prepared, treating the compound (Ic-24) or the compound (IVc-24) with an oxidizing agent such as osmium tetraoxide in the presence of a co-oxidizing agent such as N-methylmorpholine N-oxide in a single solvent such as acetone or water or a mixed solvent thereof and, if there is a protective group, carrying out deprotection in by the same manner as in the step 1.
The co-oxidizing agent is used in an amount of 1 to 10 equivalent(s) and the oxidizing agent is used in an amount of 0.01 to 1 equivalent based on the compound (Ic-24) or the compound (IVc-24).
Usually, the reaction is carried out at a temperature of from 0°C to 40°C and is completed within 5 minutes to 72 hours.

Step 3-24

In the compound (Ic), a compound (Ic-36) where $R^{3a}$ is 2,2-dimethyl-1,3-dioxolan-4-ylmethyl is prepared from a compound (Ic-35) or a compound (IVc-35) where $R^{3a}$ is 2,3-dihydroxypropyl.
The compound (Ic-36) is able to be prepared, by reacting the compound (Ic-35) or the compound (IVc-35) with 2,2-dimethoxypropane in the presence of an acidic catalyst such as p-toluenesulfonic acid in a solvent such as DMF and, if there is a protective group, carrying out deprotection in the same manner as in the step 1.
The acidic catalyst is used in an amount of 0.01 to 1 equivalent and 2,2-dimethoxypropane is used in an amount of 1 to 10 equivalents based on the compound (Ic-35) or the compound (IVc-35).
Usually, the reaction is carried out at a temperature of from 20°C to 100°C and is completed within 5 minutes to 72 hours.

Step 3-25

In the compound (Ic), a compound (Ic-37) where $R^{3a}$ is lower alkyl azide [a lower alkylene moiety in the lower alkyl azide has the same meaning as defined in the above lower alkylene (ii)] is prepared from a compound (Ic-30) or a compound (IVc-30) where $R^{3a}$ is hydroxy lower alkyl.

The compound (Ic-37) is able to be prepared, by reacting the compound (Ic-30) or the compound (IVc-30) with bis(p-nitrophenyl)phosphoric acid azide or the like in the presence of a base such as 1,8-azabicyclo[5,4,0]undec-7-ene (DBU) in a solvent such as tetrahydrofuran and, if there is a protective group, carrying out deprotection in the same manner as in the step 1.

The base and bis(p-nitrophenyl)phosphoric acid azide or the like each is used in an amount of 1 to 10 equivalents based on the compound (Ic-30) or the compound (IVc-30).

Usually, the reaction is carried out at a temperature of from 0°C to 100°C and is completed within 30 minutes to 24 hours.

Step 3-26

In the compound (Ic), a compound (Ic-38) where $R^{3a}$ is amino lower alkyl [a lower alkylene moiety in the amino lower alkyl has the same meaning as defined in the above lower alkylene (ii)] is prepared from a compound (Ic-37) or a compound (IVc-37) where $R^{3a}$ is lower alkyl azide.

The compound (Ic-38) is able to be prepared, by subjecting when the compound (Ic-37) or the compound (IVc-37) to a catalytic reduction in the presence of a reducing catalyst such as palladium carbon, lead-poisoned palladium-calcium carbonate (Lindlar catalyst) in a single solvent such as ethyl acetate, DMF, toluene, ethanol, tetrahydrofuran or water or a mixed solvent thereof and, if there is a protective group, carrying out deprotection in the same manner as in the step 1.

The reducing catalyst is used in an amount of 10 to 100% by weight based on the compound (Ic-37) or the compound (IVc-37).

Usually, the reaction is carried out at a temperature of from 20°C to 120°C and is completed within 3 minutes to 72 hours.

Step 3-27

In the compound (Ic), a compound (Ic-39) where $R^{3a}$ is lower alkyl substituted with -NHCOR$^{23}$ (where $R^{23}$ has the same meaning as defined above) [a lower alkylene moiety in the substituted lower alkyl has the same meaning as defined in the above lower alkylene (ii) above] is prepared from a compound (Ic-38) or a compound (IVc-38) where $R^{3a}$ is amino lower alkyl.

The compound (Ic-39) is able to be prepared, by reacting the compound (Ic-38) or the compound (IVc-38) with ClCOR$^{23}$ (wherein $R^{23}$ has the same meaning as defined above) in the presence of a base such as triethylamine in a single solvent such as dichloromethane, chloroform, DMF or tetrahydrofuran or a mixed solvent thereof or by reacting the compound (Ic-38) or the compound (IVc-38) with HOCOR$^{23}$ (wherein $R^{23}$ has the same meaning as defined above) in the co-presence of a dehydrating condensing agent such as dicyclohexylcarbodiimide or water-soluble carbodiimide in the presence of a base such as triethylamine in a single solvent such as dichloromethane, chloroform, DMF or tetrahydrofuran or a mixed solvent thereof and, if there is a protective group, carrying out deprotection in the same manner as in the step 1.

The base and ClCOR$^{23}$ or HOCOR$^{23}$ each are used in an amount of 1 to 10 equivalents based on the compound (Ic-38) or the compound (IVc-38).

Usually, the reaction is carried out at a temperature of from 0°C to 60°C and is completed within 3 minutes to 24 hours.

Step 3-28

In the compound (Ic), a compound (Ic-40) where $R^{3a}$ is di-(lower alkyl)amino lower alkyl [two lower alkyl moieties and a lower alkylene moiety in the di-(lower alkyl) amino lower alkyl have the same meanings as above defined in the above lower alkyl (i) and lower alkylene (ii), respectively] is prepared from a compound (Ic-38) or a compound (IVc-38) where $R^{3a}$ is amino lower alkyl.

The compound (Ic-40) is able to be prepared, by treating the compound (Ic-38) or the compound (IVc-38) with a reducing agent such as sodium triacetoxyborohydride or sodium cyanoborohydride in the presence of an aldehyde such as formaldehyde, acetaldehyde, propanal or octanal in a single solvent such as 1,2-dichloroethane, tetrahydrofuran, methanol, ethanol or water or a mixed solvent thereof and, if there is a protective group, carrying out deprotection in the same manner as in the step 1.

The aldehyde is used in an amount of 1 to 10 equivalents based on the compound (Ic-38) or the compound (IVc-38).

Usually, the reaction is carried out at a temperature of from 0°C to 40°C and is completed within 5 minutes to 24 hours.

Step 3-29

In the compound (Ic), a compound (Ic-41) where $R^{3a}$ is substituted or unsubstituted lower alkyl or substituted or unsubstituted aralkyl is prepared from a compound (Ic-33) or a compound (IVc-33) where $R^{3a}$ is a hydrogen atom.

The compound (Ic-41) is able to be prepared, by reacting the compound (Ic-33) or the compound (IVc-33) with a nucleophilic agent such as a substituted or unsubstituted lower alkyl halide [a halogen moiety and a lower alkylene moiety of the lower alkyl halide have the same meanings as above defined in the above halogen (xii) and lower alkylene (ii), respectively and the substituent of the substituted lower alkyl halide has the same meaning as the substituent (xi) in the above substituted lower alkyl] or a substituted or unsubstituted aralkyl halide [a halogen moiety and aralkyl moiety of the aralkyl halide have the same meanings as above defined in the above halogen (xii) and aralkyl (vii), respectively and a substituent of the substituted aralkyl halide has the same meaning as the substituent (xiii) in the above substituted aralkyl] in the presence of a base in a single solvent such as dichloromethane, DMF or water or a mixed solvent thereof and, if there is a protective group, carrying out deprotection in the same manner as in the step 1.

Examples of the base used include potassium carbonate, lithium hydroxide, potassium tert-butoxide and sodium hydride.

The nucleophilic agent and the base each are used in an amount of 1 to 10 equivalents based on the compound (Ic-33) or the compound (IVc-33).

Usually, the reaction is carried out at a temperature of from 0°C to 150°C and is completed within 5 minutes to 24 hours.

Production Process 4

In the compound (I), a compound (Id) where $R^3$ is $R^{3a}$ (wherein $R^{3a}$ has the same meaning as defined above), $R^5$ is $R^{5a}$ (wherein $R^{5a}$ has the same meaning as defined above) and X and Y are $X^a$ and $Y^a$, respectively [wherein, $X^a$ and $Y^a$ are the same or different and each represent carbonyl, $-CH_2-$, -CH(OH)- or $-CH(OR^x)-$ (wherein $R^x$ has the same meaning as defined above) and at least one of $X^a$ and $Y^a$ is $-CH_2-$, -CH(OH)- or $-CH(OR^x)-$] is able to be prepared according to the following step from the compound (Ic) which is prepared in any of the above production processes 1 to 3.

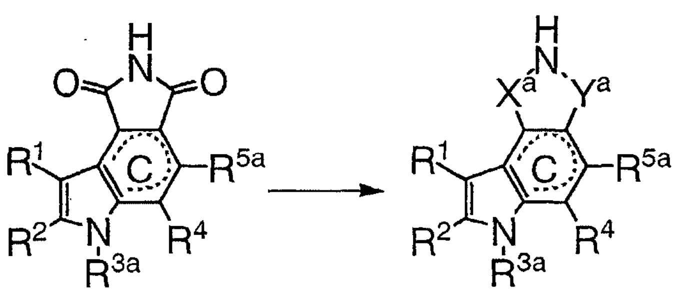

(Ic) (Id)

(wherein, each of $R^1$, $R^2$, $R^{3a}$, $R^4$, $R^{5a}$, $X^a$, $Y^a$ and C ring has the same meaning as defined above.)

Step 4

When the compound (Ic) is treated with a reducing agent such as sodium borohydride in a single solvent such as tetrahydrofuran or methanol or a mixed solvent thereof, a compound (Id-1) which is the compound (Id) where both $X^a$ and $Y^a$ are not $-CH(OR^x)-$ is able to be prepared.

Further, when the compound (Ic) is treated with a reducing agent such as sodium borohydride in a lower alcohol [a lower alkyl moiety of the lower alcohol has the same meaning as above defined in the lower alkyl (i)] solvent or in a mixed solvent of lower alcohol with tetrahydrofuran or the like, a compound (Id-2) which is the compound (Id)

where at least one of $X^a$ and $Y^a$ is -CH($OR^x$)- is prepared.

The reducing agent is used in an amount of 1 to 10 equivalents based on the compound (Ic).

The reaction is carried out at a temperature of from -78°C to 30°C and is completed within 3 to 24 hours.

It is also possible that, by subjecting the compound (Id) which is prepared in this step to a conversion of a functional group in the same manner as in the above production process 3, other compound (Id) is induced therefrom.

Production Process 5

In the compound (I), a compound (If) where $R^3$ and $R^5$ form a formula (T) together is able to be prepared according to the following step from a compound (Ie) which is the compound (Id) prepared in the production process 4 where $R^{3a}$ is lower alkyl azide [a lower alkylene moiety in the lower alkyl azide has the same meaning as defined in the above lower alkylene (ii)] and $R^{5a}$ is a substituted or unsubstituted lower alkanoyl or the compound (Ic-37) prepared in the production processes 1 and 2 or the step 3-25 of the production process 3.

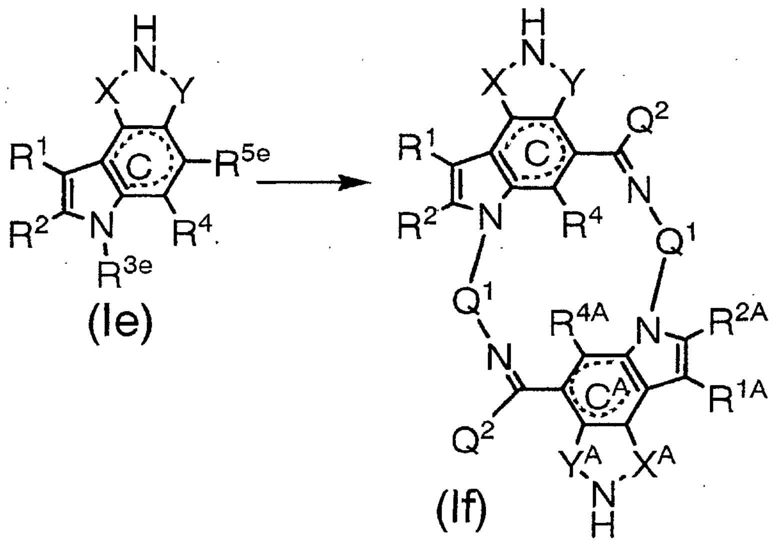

{wherein, $R^1$, $R^2$, $R^4$, X, Y, C ring, $Q^1$, $Q^2$, $R^{1A}$, $R^{2A}$, $R^{4A}$, $X^A$, $Y^A$ and $C^A$ ring each have the same meanings as defined above; $R^{3e}$ is lower alkyl azide [a lower alkylene moiety of the lower alkyl azide has the same meaning as defined in the above lower alkylene (ii)]; and $R^{5e}$ is substituted or unsubstituted lower alkanoyl [the alkanoyl has the same meaning as the above alkanoyl (vi) and a substituent of the substituted alkanoyl has the same meaning as defined in the substituent (xi) of the above substituted alkanoyl]}.

Step 5

The compound (If) is able to be prepared when the compound (Ie) is treated with triphenylphosphine or the like in a single solvent such as tetrahydrofuran, methanol, ethanol, water, chloroform, dichloromethane or DMF or a mixed solvent thereof or is subjected to a catalytic reduction in the presence of a reducing catalyst such as palladium carbon or lead-poisoned palladium-calcium carbonate (Lindlar catalyst).

Triphenylphosphine or the like is used in an amount of 1 to 20 equivalent(s) and the reducing catalyst is used in an amount of 5 to 100% by weight to the compound (Ie).

Usually, the reaction is carried out at a temperature of from -20°C to 100°C and is completed within 30 minutes to 48 hours.

It is also possible to prepare the compound (If) when an amino substance produced in the reduction of an azide group in the above step is treated with a silica gel column chromatography, a preparative thin layer chromatography, etc. or is treated with an acid such as hydrochloric acid, acetic acid, sulfuric acid, p-toluenesulfonic acid or 10-camphorsulfonic acid in a single solvent such as tetrahydrofuran, 1,4-dioxane, methanol, ethanol, water, chloroform, dichloromethane or DMF or a mixed solvent thereof.

It is also possible that, by subjecting the compound (If) which is prepared in this step is subjected to a conversion of a functional group by the same manner as in the above production process 3, other compound (If) is induced therefrom.

Production Process 6

In the compound (I), a compound (Ig) where at least one of X and Y is -CH(SR$^Y$)- (wherein R$^Y$ has the same meaning as defined above) is able to be prepared according to a process described in WO 89/7105 from the compound (Id) or the compound (If) prepared by the Production Process 4 or the Production Process 5 where at least one of X and Y is -CH(OH)-.

Besides the above steps, it is also possible to convert the functional group contained in the substituents of $R^1$, $R^2$, $R^3$, $R^4$ or $R^5$ or to convert X and Y in the compound (I) and the starting compound by other known method [ such as "Comprehensive Organic Transformations" by R. C. Larock, (1989)].

It is further possible that, by subjecting the compound (Ig) which is prepared in this step to a conversion of a functional group conversion in the same manner as in the above production process 3, to other compound (Ig) is induced therefrom.

When the above methods are appropriately combined and carried out, it is possible to prepare a compound (I) having a desired functional group at a desired position.

Isolation and purification of the products in the above production processes are able to be carried out by means of an appropriate combination of the methods which are commonly used in organic synthesis such as filtration, extraction, washing, drying, concentration, crystallization, various chromatographic means, etc. With regard to an intermediate, that may be also able to be subjected to the next reaction without any purification.

In some of the compound (I), there are isomers such as regioisomer, geometric isomer and optical isomer and any of the possible isomers and a mixture of any ratio of such isomers are also included in the present invention.

When a salt of a compound (I) is to be prepared, a salt of the compound (I) may be purified as it is in case such a salt is obtained, while, in case the compound (I) is obtained in a free form, then the compound (I) is dissolved or suspended in an appropriate solvent followed by adding acid or base to form a salt.

There are some cases where the compound (I) or a pharmaceutically acceptable salt is present in a form of an adduct with water or with various kinds of solvents and the such additives are also included in the present invention.

Specific examples of the compound (I) are shown in Table 1 to 8.

In the following tables, Ph and Ac means phenyl and acetyl, respectively.

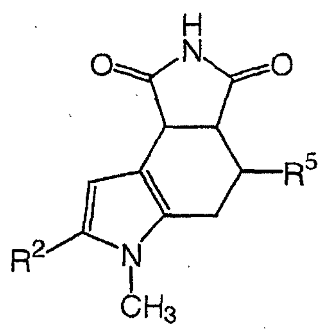

Table 1

| Compound No. | $R^2$ | $R^5$ |
|---|---|---|
| 1 | H | 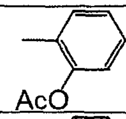 |
| 2 | —CHO | 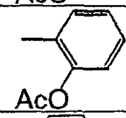 |
| 3 | H | 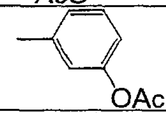 |
| 4 | —CHO | 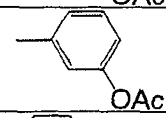 |
| 5 | H | 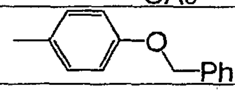 |

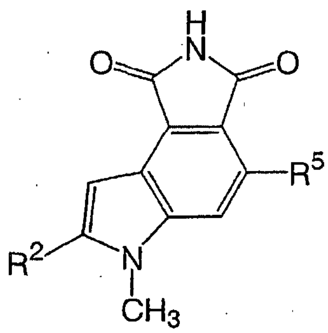

Table 2

| Compound No. | $R^2$ | $R^5$ |
|---|---|---|
| 6 | $-CHO$ | 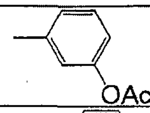 |
| 7 | $-CHO$ | 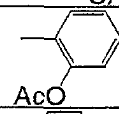 |
| 8 | $-CH=CHCO_2CH_3$ | 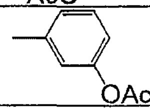 |
| 9 | 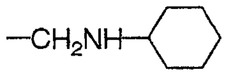 | 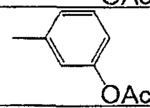 |
| 10 | $-CH_2OH$ | 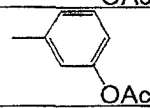 |
| 11 | $-CHO$ | 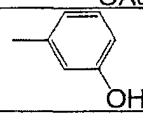 |
| 12 | $-CHO$ | 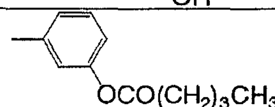 |
| 13 | $-CH_2NCH_3(CH_2)_2N(CH_3)_2$ | 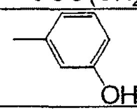 |
| 14 | $-CH_2NCH_3(CH_2)_2N(CH_3)_2$ | 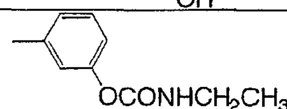 |
| 15 | $-CHO$ | 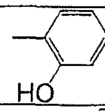 |
| 16 | $-CHO$ | 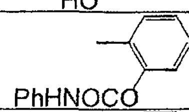 |
| 17 | $-CH_2NH(CH_2)_2OH$ | 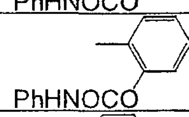 |
| 18 | 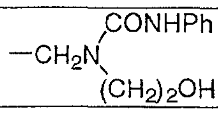 | 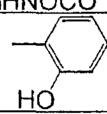 |

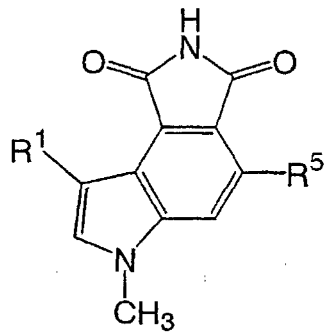

Table 3

| Compound No. | $R^1$ | $R^5$ |
|---|---|---|
| 19 | H | 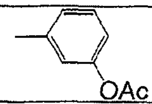 |
| 20 | -CHO | 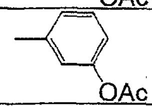 |
| 21 | -CHO | 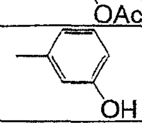 |
| 22 | -CHO | 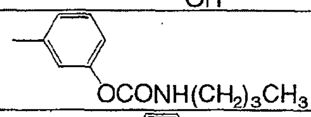 |
| 23 | $-CH_2NCH_3(CH_2)_2N(CH_3)_2$ | 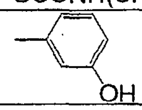 |

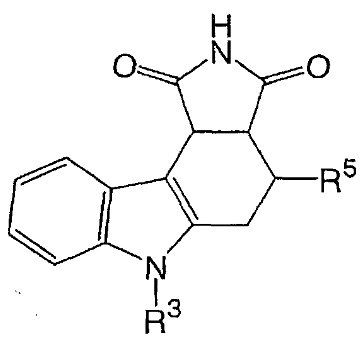

Table 4

| Compound No. | $R^3$ | $R^5$ |
|---|---|---|
| 24 | $-CH_3$ | $-CH_2OH$ |
| 25 | $-CH_3$ | $-(CH_2)_2OH$ |
| 26 | $-CH_2Ph$ | 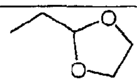 |

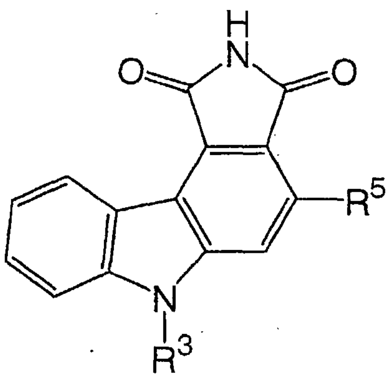

Table 5

| Compound No. | $R^3$ | $R^5$ |
| --- | --- | --- |
| 27 | $-CH_3$ | $-CO_2CH_3$ |
| 28 | $-CH_3$ | $-CO_2H$ |
| 29 | $-CH_3$ | $-CONHC(CH_3)_2CH_2OH$ |
| 30 | $-CH_3$ | 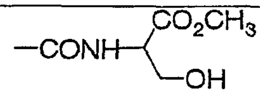 |
| 31 | $-CH_3$ | 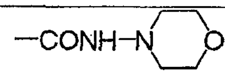 |
| 32 | $-CH_3$ | $-CONHNHAc$ |
| 33 | $-CH_3$ | 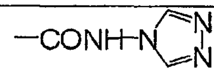 |
| 34 | $-CH_3$ | $-CH_2OH$ |
| 35 | $-CH_3$ | $-(CH_2)_2OH$ |
| 36 | $-CH_3$ | $-CH_2Br$ |
| 37 | $-CH_3$ | $-CH_2NH(CH_2)_2OH$ |
| 38 | $-CH_3$ | 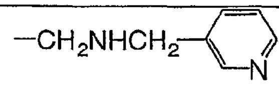 |
| 39 | $-CH_3$ | 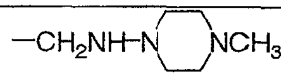 |
| 40 | $-CH_2CH=CH_2$ | $-CO_2CH_2CH_3$ |
| 41 | $-CH_2CH=CH_2$ | $-CO_2H$ |
| 42 | $-CH_2CH=CH_2$ | $-CONH(CH_2)_2OH$ |
| 43 | $-CH_2CH=CH_2$ | 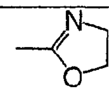 |

Table 5 (continued)

| Compound No. | $R^3$ | $R^5$ |
|---|---|---|
| 44 | $-CH_2CH=CH_2$ | $-NHCO_2CH_2CH_3$ |
| 45 | $-CH_2CH=CH_2$ | $-NH_2$ |
| 46 | $-CH_2CH=CH_2$ | $-CH_2OH$ |
| 47 | $-CH_2CH=CH_2$ | 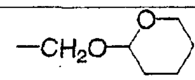 |
| 48 | $-CH_2CH=CH_2$ | $-CH_2N_3$ |
| 49 | $-CH_2CH=CH_2$ | $-CH_2NH_2$ |
| 50 | $-CH_2CH=CH_2$ | $-CHO$ |
| 51 | $-CH_2CH=CH_2$ | $-CH=NOH$ |
| 52 | $-CH_2CH=CH_2$ | $-CH=NOCH_3$ |
| 53 | $-CH_2CH=CH_2$ | 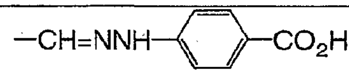 |
| 54 | $-CH_2CH=CH_2$ | $-CH=NNH(CH_2)_2OH$ |
| 55 | $-CH_2CH=CH_2$ | 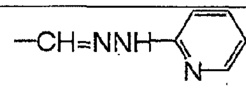 |
| 56 | $-CH_2CH=CH_2$ | $-CH=NNH_2$ |
| 57 | $-(CH_2)_3OH$ | $-CH_2OH$ |
| 58 | $-(CH_2)_3Br$ | $-CH_2Br$ |
| 59 | 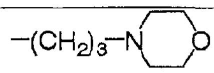 | 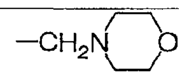 |
| 60 | $-(CH_2)_3OH$ | 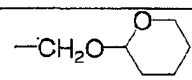 |

Table 5 (continued)

| Compound No. | $R^3$ | $R^5$ |
|---|---|---|
| 61 | $-(CH_2)_3N_3$ | 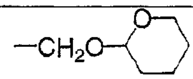 |
| 62 | $-(CH_2)_3NH_2$ | 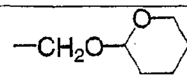 |
| 63 | $-(CH_2)_3NHCOPh$ | 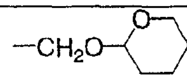 |
| 64 | $-(CH_2)_3N(CH_3)_2$ | $-CHO$ |
| 65 | $-(CH_2)_3N(CH_3)_2$ | $-CH=NOH$ |
| 66 | 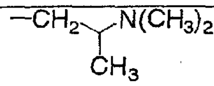 | $-CHO$ |
| 67 | 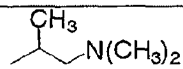 | $-CHO$ |
| 68 | 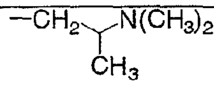 | $-CH=NOH$ |
| 69 | 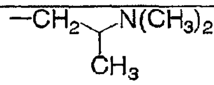 | $-CH=NNH_2$ |
| 70 | 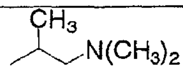 | $-CH=NOH$ |
| 71 | $-CH_2CO_2CH_2Ph$ | $-CO_2CH_2CH_3$ |
| 72 | $-CH_2CO_2H$ | $-CO_2H$ |
| 73 | 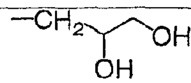 | $-CO_2CH_2CH_3$ |
| 74 | 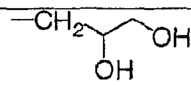 | $-CO_2H$ |
| 75 | 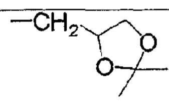 | $-CO_2H$ |
| 76 | 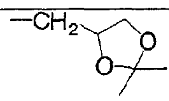 | 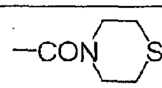 |

Table 5 (continued)

| Compound No. | $R^3$ | $R^5$ |
|---|---|---|
| 77 | $-CH_2Ph$ | 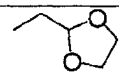 |
| 78 | $-CH_2Ph$ | 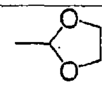 |
| 79 | 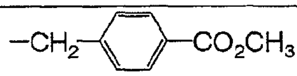 | 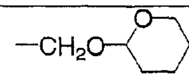 |
| 80 | 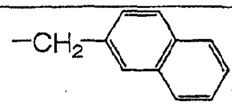 | 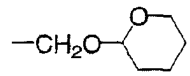 |
| 81 | 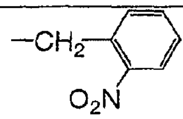 | 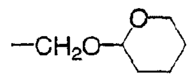 |
| 82 | 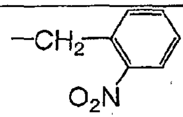 | $-CH_2OH$ |
| 83 | $-(CH_2)_3OH$ | $-CO_2CH_2CH_3$ |
| 84 | $-(CH_2)_3N_3$ | $-CO_2CH_2CH_3$ |
| 85 | $-(CH_2)_3NH_2$ | $-CO_2CH_2CH_3$ |
| 86 | $-CH_2CO_2H$ | $-CO_2CH_2CH_3$ |
| 87 | $-(CH_2)_2OH$ | $-CO_2CH_2CH_3$ |
| 88 | $-(CH_2)_2N_3$ | $-CO_2CH_2CH_3$ |
| 89 | $-(CH_2)_2NH_2$ | $-CO_2CH_2CH_3$ |

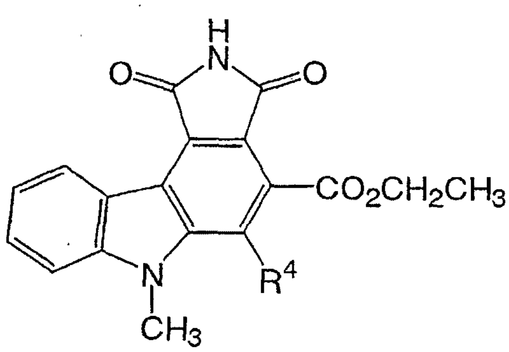

Table 6

| Compound No. | $R^4$ |
|---|---|
| 90 | $-(CH_2)_3OCH_2Ph$ |
| 91 | $-(CH_2)_3OH$ |
| 92 | $-(CH_2)_3Br$ |
| 93 | $-(CH_2)_3NHCH_2Ph$ |

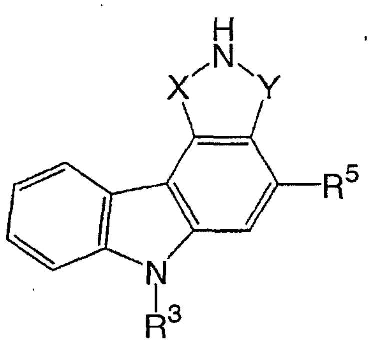

Table 7

| Compound No. | $R^3$ | $R^5$ | X | Y |
|---|---|---|---|---|
| 94 | $-CH_2CH=CH_2$ | $-CO_2CH_2CH_3$ | C=O | -CH-OH)- |
| 95 | $-CH_2CH=CH_2$ | $-CO_2CH_2CH_3$ | -CH(OH)- | C=O |
| 96 | $-(CH_2)_3NH_2$ | $-CO_2CH_2CH_3$ | C=O | $(CH(OCH_3)-$ |
| 97 | $-(CH_2)_3NH_2$ | $-CO_2CH_2CH_3$ | C=O | -CH-OH)- |
| 98 | $-(CH_2)_3NH_2$ | $-CO_2CH_2CH_3$ | -CH(OH)- | C=O |
| 99 | $-CH_2CH=CH_2$ | $-COCH_3$ | C=O | C=O |
| 100 | $-(CH_2)_3OH$ | $-CH(OH)CH_3$ | C=O | C=O |
| 101 | $-(CH_2)_3OH$ | $-COCH_3$ | C=O | C=O |
| 102 | $-(CH_2)_3N_3$ | $-COCH_3$ | C=O | C=O |
| 103 | $-(CH_2)_3NH_2$ | $-COCH_3$ | C=O | C=O |
| 104 | $-CH_2CH=CH_2$ | $-(COCH_2CH_2CH_3$ | C=O | C=O |
| 105 | $-(CH_2)_3OH$ | $-(COCH_2CH_2CH_3$ | C=O | C=O |
| 106 | $-(CH_2)_3OH$ | $-CH(OH)CH_2CH_2CH_3$ | C=O | C=O |
| 107 | $-(CH_2)_3N_3$ | $-(COCH_2CH_2CH_3$ | C=O | C=O |
| 108 | $-(CH_2)_3NH_2$ | $-(COCH_2CH_2CH_3$ | C=O | C=O |

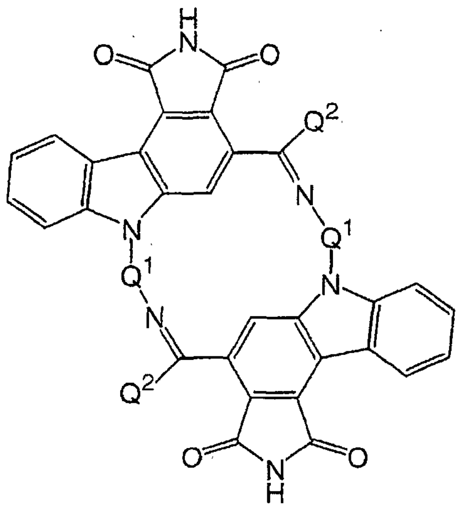

Table 8

| Compound No. | $Q^1$ | $Q^2$ |
|---|---|---|
| 109 | $-(CH_2)_3-$ | $-CH_3$ |

Now, toxicity and activity of the compound (I) are illustrated by way of Test Examples.

Test Example 1: CDK2 Inhibitory activity

A crude extract of cells of an insect, *Spodoptera frugiperda* (Sf-9) infected with human CDK2-cyclin E-expressing *baculovirus* was subjected to an affinity purification to give an enzyme solution. The enzyme solution (4 μL), 20 μL of reaction solution containing a DMSO solution in which a test compound was dissolved (final concentration: 0.1%) and 5 μL of a substrate solution [50 μmol/L of ATP (final concentration), 2 μCi of [$\gamma$-$^{32}$P]-ATP and 500 ng of glutathione S-transferase phosphate buffer (GST-RB)] were incubated at 30°C for 5 minutes. A sodium dodecylsulfate-polyacrylamide gel electrophoresis (SDS-PAGE) was carried out, then incorporation of radiation activity from [$\gamma$-$^{32}$P] -ATP into GST-RB was determined using an Image Analyzer BAS200 (FujiPhoto-Film) and an enzyme inhibition was calculated. CDK2 inhibitory activity (I) at various concentrations of a test compound was calculated by the following formula using radiation activity (C) of the group to which no test compound was added, radiation activity (S) of the group to which a test compound was added and radiation activity (B) of a background (a sample where the reaction time was 0 hour) and a concentration by which CDK2 activity was inhibited to an extent of 50% by each test compound was defined as $IC_{50}$.

$$I (\%) = [1 - (S - B)/(C - B)] \times 100$$

The result is shown in Table 9.

Table 9

| Compound No. | Enzyme Inhibitory Activity, $IC_{50}$ (μmol/L) |
|---|---|
| 12 | 5.0 |
| 36 | 2.3 |
| 45 | 4.9 |
| 46 | 3.3 |
| 47 | 4.0 |

Table 9 (continued)

| Compound No. | Enzyme Inhibitory Activity, $IC_{50}$ (μmol/L) |
|---|---|
| 48 | 0.43 |
| 50 | 0.66 |
| 54 | 2.0 |
| 64 | 7.0 |
| 65 | 2.8 |
| 66 | 0.25 |
| 68 | 0.20 |
| 69 | 0.96 |
| 79 | 2.0 |
| 80 | 2.4 |

Test Example 2: Cell growth inhibition against human osteosarcoma cells Saos-2 and U2OS cells

Each of Saos-2 and U2OS cells was made into $1.0 \times 10^4$ cells/mL using a Roswell Park Memorial Institute's medium (RPMI) 1640 (manufactured by Nissui) containing 10% of fetal bovine serum and penicillin/streptomycin and a Dulbecco's modified Eagle medium (DME) (manufactured by Nissui) containing 10% fetal bovine serum and penicillin/streptomycin each. Each of the prepared cells was dispensed in an amount of 0.1 mL per well to a 96-well microtiter plate (#167008 manufactured by Nunk). Each plate was incubated in a carbon dioxide gas incubator at 37°C for 20 hours. A solution of a test compound in DMSO prepared into 10 mmol/L was diluted with each medium for incubation and each 0.05 mL thereof was added to each well, subjected to stepwise dilution by means of a pipetting operation in the plate and incubated at 37°C for 72 hours in a carbon dioxide gas incubator.

A supernatant was removed from cell culture, each well was washed with 0.1 mL of a phosphate buffer (PBS buffer) twice and 0.1 mL of the above medium was added to each well. A cell proliferation kit II (manufactured by Boehringer Mannheim) was used for the measurement of cell numbers in each well. A reagent for color reaction was added, incubation was carried out in a carbon dioxide gas incubator at 37°C for 3 hours and absorbance at 490 nm and 650 nm was measured in a microplate reader (M-SP max 250; manufactured by Wako Pure Chemicals). A value where absorbance at 650 nm was deducted from that at 490 nm (difference absorbance) was calculated for each well and the difference absorbance obtained from non-treated cells and cells which were treated with a test compound of a known concentration was compared whereupon a concentration of the test compound which inhibited the cell growth to an extent of 50% was calculated and expressed as $IC_{50}$.

The results is shown in Table 10.

Table 10

| Compound No. | Cell Growth Inhibitory Activity, $IC_{50}$ (μmol/L) | |
|---|---|---|
| | Saos-2 | U2OS |
| 64 | 1.3 | 1.6 |
| 65 | 0.68 | 0.50 |
| 66 | 1.2 | 2.1 |
| 68 | 0.93 | 1.2 |
| 69 | 0.30 | 0.68 |

Test Example 3: Abrogating Action for the Cell Cycle Accumulation in G2 and S Phases

Each 5 mL of human epidermoid carcinoma cells A431 (hereinafter, referred to as A431 cells) prepared in $6 \times 10^4$ cells/mL in a DMEM medium (manufactured by Nissui; hereinafter, referred to as the medium A) containing 10% fetal bovine serum were disposed in a 10-cm laboratory dish (manufactured by Falcon; product no. 3303). The plate was incubated in a carbon dioxide gas incubator at 37°C for 24 hours, then cisplatin (manufactured by Sigma) where

its final concentration in the medium A was made 20 μmol/L was added and the mixture was incubated at 37°C for 1 hour in a carbon dioxide gas incubator.

After removing the medium, the cell was washed with PBS(-) [phosphate-buffered physiological saline solution (containing no calcium ion); manufactured by Dainippon Pharmaceutical], then the medium A was added thereto, the mixture was incubated at 37°C for 15 hours in a carbon dioxide gas incubator, a compound which was appropriately diluted with the medium A was added thereto and the mixture was incubated again in a carbon dioxide gas incubator at 37°C for 8 hours. After removal of the supernatant from the culture, the cell was washed with PBS(-), exfoliated with a 0.25% aqueous solution of trypsin (0.25% Trypsin manufactured by Gibco BRL) and a 0.02% aqueous solution of ethylenediaminetetraacetic acid (manufactured by Wako Pure Chemicals), fixed with a 70% aqueous solution of ethanol so as to make the cell concentration $10^6$ cells/mL and preserved in a cool chamber of 4°C. The fixed cells were subjected to a centrifugal separation to remove ethanol, washed with PBS(-) and treated with a 0.25 mg/mL ribonuclease A type 1-A (manufactured by Sigma) PBS(-) solution containing 0.1% Nonidet P-40 (manufactured by Nakarai Kagaku Yakuhin) at 37°C for 30 minutes and a 0.1% solution of propidium iodide (manufactured by Sigma) in NP-40/PBS(-) so as to make the final concentration 50 μg/mL followed by staining in ice for not shorter than 20 minutes.

A DNA histogram was measured using an EPICS ELITE Flow Cytometer (manufactured by Coulter) and distribution of cell cycle was analyzed using a Multicycle Program (manufactured by Phonenix Flow Systems).

The result is shown in Table 11.

Table 11

| | Cell Cycle Distribution (%) | | |
|---|---|---|---|
| | G1 | S | G2M |
| Untreated | 43.0 | 41.0 | 16.1 |
| Cisplatin (20 μmol/L) | 10.4 | 67.2 | 22.4 |
| Cisplatin (20 μmol/L) and the compound 70 (3 μmol/L) | 24.9 | 57.0 | 18.0 |
| Cisplatin (20 μmol/L) and the compound 85 (3 μmol/L) | 29.6 | 44.0 | 26.4 |
| Cisplatin (20 μmol/L) and the compound 89 (3 μmol/L) | 28.6 | 44.1 | 27.3 |
| Untreated | 39.4 | 44.7 | 15.9 |
| Cisplatin (20 μmol/L) | 2.2 | 80.6 | 17.2 |
| Cisplatin (20 μmol/L) and the compound 98 (10 μmol/L) | 15.2 | 66.7 | 18.1 |

In a cell cycle distribution in a group which was treated with cisplatin (20 μmol/L), there were noted increase in S phase, increase in G2 and M phase and decrease in G1 phase (inhibition to the progress to the next cell cycle or, in other words, induction of the accumulation in G2 or S phase) as compared with a cell cycle distribution in untreated groups.

When the compound 70, the compound 85 or the compound 89 (3 μmol/L each) or the compound 98 (10 μmol/L) was used together with cisplatin (20 μmol/L), there were noted decrease of S phase and increase of G1 phase (abrogation of the cell cycle accumulation in G2 or S phase).

Accordingly, it was suggested that the compounds of the present invention abrogated the cisplatin-induced cell cycle accumulation of cancer cells in G2 or S phase and potentiated the cell-killing affect of cisplatin.

The compound (I) or a pharmaceutically acceptable salt thereof is able to be used either solely as it is or in various pharmaceutical forms depending upon its pharmacological action and its object of administration.

The pharmaceutical preparation according to the present invention may contain the compound (I) or the pharmaceutically acceptable salt thereof in an amount which is effective as an active ingredient either solely or as a mixture with other active ingredient for the treatment. The pharmaceutical preparation as such may also be prepared by any method which has been well known in the technical filed of pharmaceutical preparation science by mixing the active ingredient with a pharmaceutically acceptable carrier or the like. The carrier may be in a broad range of forms depending upon the form of the preparation desirable for administration.

With regard to the administration route, it is preferred to use that which is most effective for the treatment and its examples are oral route and parenteral route such as an intravenous route.

Examples of the administration form are tablets, capsules, granules, syrups and injections.

In preparing the tablets, excipients such as lactose, glucose, sucrose, mannitol and methylcellulose, disintegrating agents such as starch, sodium alginate, carboxymethylcellulose calcium and crystalline cellulose, lubricants such as magnesium stearate and talc, binders such as gelatin, polyvinyl alcohol, polyvinylpyrrolidone, hydroxypropyl

cellulose and methylcellulose, surface-active agents such as sucrose fatty acid ester and sorbitol fatty acid ester, etc. may be used according to a conventional manner. Tablets where each tablet contains 1 to 200 mg of the active ingredient are suitable.

In preparing the granules, excipients such as lactose and sucrose, disintegrating agents such as starch, binders such as gelatin, etc. may be used according to a conventional manner.

In preparing the capsules, gelatin, water, sucrose, acacia, sorbitol, glycerol, crystalline cellulose, magnesium stearate, talc, etc. may be used according to a conventional manner. Capsules where each capsule contains 1 to 200 mg of the active ingredient is suitable.

In preparing the syrups, saccharides such as sucrose, water, ethanol, etc. may be used according to a conventional manner.

In preparing the injections, solvents such as water, physiological saline solution, vegetable oil (e.g., olive oil and peanut oil), ethyl oleate and propylene glycol, solubilizing agents such as sodium benzoate, sodium salicylate and urethane, isotonizing agents such as salt and glucose, preservatives such as phenol, cresol, p-hydroxybenzoates and chlorobutanol, antioxidants such as ascorbic acid and sodium pyrosulfite, etc. may be used according to a conventional manner.

The compound (I) or the pharmaceutically acceptable salt thereof is able to be administered either by an oral method or by a parenteral method such as injections and, although its effective dose and administrating frequency depend upon the dosage form and age, body weight, symptom, etc. of a patient, it is usually preferred to administer 0.1 to 50 mg/kg per day.

## Best Mode for Carrying Out the Invention

The present invention is now illustrated in detail as hereunder by way of Examples.

In a proton nuclear magnetic resonance spectrum ($^1$H-NMR) used in the Examples, exchangeable hydrogen may not be clearly observed depending upon the compound and the measuring condition. With regard to notation of multiplicity of signals, that which has been commonly used is used where "br" means a signal having an apparently broad width.

### Example 1: Compound 1

#### Step 1

2-[2-(2-Hydroxyphenyl)vinyl]-1-methylpyrrole (9.6 g, 48 mmol) was dissolved in 25 mL of pyridine, 4.75 mL (50.3 mmol) of acetic anhydride and 296.0 mg (2.423 mmol) of dimethylaminopyridine were added thereto and the mixture was stirred at room temperature for 30 minutes. Ice water was added to the reaction solution, the mixture was diluted with ether and the reaction solution was acidifiedwith 6 mol/L hydrochloric acid. The reaction solution was extracted with ether and the organic layer was washed with hydrochloric acid, a saturated aqueous solution of sodium hydrogen carbonate, water and a saturated saline solution successively and dried over magnesium sulfate. The solvent was evaporated from the organic layer to give 2-[2-(2-acetoxyphenyl)vinyl]-1-methylpyrrole.

#### Step 2

Maleimide (9.37 g, 96.5 mmol) was added to 2-[2-(2-acetoxyphenyl)vinyl]-1-methylpyrrole prepared in the step 1, the mixture was stirred at 180°C for 30 minutes and the reaction mixture was purified by a silica gel column chromatography (hexane/ethyl acetate = 1/1) to give 13.71 g (84% throughout the two steps) of the compound 1.
$^1$H-NMR(270MHz, $CDCl_3$, δ); 2.31(s, 3H), 2.79(dd, J=4.3Hz, 15.2Hz, 1H), 3.12(m, 1H), 3.38(m, 1H), 3.52(s, 3H), 3.65 (dd, J=3.5Hz, 7.6Hz, 1H), 4.08(d, J=7.6Hz, 1H), 6.27(d, J=3.0Hz, 1H), 6.59(d, J=3.0Hz, 1H), 7.05(m, 1H), 7.22-7.45 (m, 3H), 7.68(m, 1H).
Fab-MS(m/z); 339[M+1]$^+$

### Example 2: Compound 2

In an argon atmosphere, 136 mL (0.888 mmol) of phosphoryl chloride was added to 0.65 mL (8.9 mmol) of DMF, the mixture was stirred at room temperature for 20 minutes, 105.0 mg (0.310 mmol) of the compound 1 prepared in Example 1 were added and the mixture was stirred at room temperature for 1 hour. After ice water was added to the reaction solution, potassium carbonate was added thereto and the mixture was extracted with ethyl acetate. After the organic layer was washed with water and a saturated saline solution, it was dried over magnesium sulfate. The solventwas evaporated from the organic layer and the residue was purified by a thin-layer chromatography (hexane/ethyl acetate = 2/3) to give 90 mg (79%) of the compound 2.
$^1$H-NMR(270MHz, $CDCl_3$, δ); 2.33(s, 3H), 2.91(m, 1H), 3.23(m, 1H), 3.43(m, 1H), 3.71(dd, J=3.6Hz, 7.9Hz, 1H), 3.87 (s, 3H), 4.08(d, J=7.9Hz, 1H), 7.06(s, 1H), 7.09(m, 1H), 7.26-7.39(m, 3H), 7.60(m, 1H), 9.51(s, 1H).
Fab-MS(m/z); 367$[M+1]^+$

Example 3: Compound 3

After 13.82 g (69.36 mmol) of 2-[2-(3-hydroxyphenyl)-vinyl]-1-methylpyrrole were treated with 6.8 mL (72.07 mmol) of acetic anhydride and 428.0 mg (3.503 mmol) of dimethylaminopyridine according to the step 1 of Example 1 and then made to react with 13.46 g (138.7 mmol) of maleimide according to the step 2 of Example 1 to give 18.78 g (80%) of the compound 3.
$^1$H-NMR(270MHz, $CDCl_3$, δ); 2.28 and 2.31(2s, total 3H), 2.76-3.27(m, 2H), 3.35-3.89(m, 2H), 3.47 and 3.52(2s, total 3H), 3.97 and 4.11(2d, J=7.6Hz, total 1H), 6.26(d, J=2.6Hz, 1H), 6.58(d, J=2.6Hz, 1H), 6.86-7.38(m, 4H), 7.52 and 7.76(2brs, total 1H).
Fab-MS(m/z); 339$[M+1]^+$

Example 4: Compound 4

A compound 4 (5.90 g, 35%) was prepared from 17.8 g (45.90 mmol) of the compound 3 prepared in Example 3, 42.7 mL (551 mmol) of DMF and 12.8 mL (137 mmol) of phosphoryl chloride in a similar manner to that in Example 2.
$^1$H-NMR(274MHz, $CDCl_3$, δ); 2.28 and 2.30(2s, total 3H), 2.91-3.12(m, 2H), 3.47(m, 1H), 3.67 and 3.91(2m, total 1H), 3.83 and 3.88(2s, total 3H), 4.01 and 4.11(2d, J=7.8Hz, total 1H), 6.81-7.38(m, 5H), 7.76 and 8.01(2brs, total 1H), 9.50 and 9.51(2s, total 1H).
Fab-MS(m/z); 367$[M+1]^+$

Example 5: Compound 5

A compound 5 (20.70 g, 83%) was prepared from 18.76 g (64.83 mmol) of 2-[2-(4-benzyloxyphenyl)vinyl]-1-methylpyrrole and 18.91 g (194.81 mmol) of maleimide in a similar manner to that in the step 2 of Example 1.
$^1$H-NMR(270MHz, $CDCl_3$, δ); 2.72-3.06(m, 2H), 3.39(m, 1H), 3.47 and 3.52(2s, total 3H), 3.58 and 3.78(2m, total 1H), 3.96 and 4.12(2d, J=7.4Hz, total 1H), 5.02 and 5.05(2s, total 2H), 6.27(m, 1H), 6.59(d, J=2.8Hz, 1H), 6.85-7.45(m, 9H).
Fab-MS(m/z); 387$[M+1]^+$

Example 6: Compound 6

The compound 4 (5.90 g, 16.10 mmol) prepared in Example 4 was dissolved in 300 mL of dioxane, 36.55 g (161.0 mmol) of DDQ were added thereto and the mixture was heated to reflux for 1.5 hours. The reaction solution was vacuum filtered to remove the precipitate, the solvent was evaporated and the residue was triturated with ethyl acetate to give 4.36 g (75%) of the compound 6.
$^1$H-NMR(270MHz, $CDCl_3$, δ); 2.26(s, 3H), 4.11 (s, 3H), 7.09-7.51(m, 4H), 7.25(s, 1H), 7.53(s, 1H), 7.71(s, 1H), 9.97 (s, 1H).
Fab-MS(m/z); 363$[M+1]^+$

Example 7: Compound 7

The compound 7 (69.8 mg, 70%) was prepared from 100.0 mg (0.273 mmol) of the compound 2 prepared in Example 2 and 576.0 mg (2.537 mmol) of DDQ in the similar manner as in Example 6.
$^1$H-NMR(270MHz, DMSO-$d_6$, δ); 1.95(s, 3H), 4.12(s, 3H), 7.34(d, J=8.3Hz, 1H), 7.45(d, J=7.6Hz, 1H), 7.53-7.59(m, 2H), 7.86(s, 1H), 7.87(s, 1H), 10.15(s, 1H), 11.15(brs, 1H).
Fab-MS(m/z); 363$[M+1]^+$

Example 8: Compound 8

The compound 6 (11.0 mg, 0.030 mmol) prepared in Example 6 was dissolved in 5 mL of 1,2-dichloroethane, 14.0 mg (0.042 mmol) of methyl triphenylphosphoranylideneacetate were added thereto in an argon atmosphere and the mixture was heated to reflux for 3.5 hours. The reaction was stopped by addition of water to the reaction solution and, after diluting with dichloromethane, extraction was carried out. An organic layer was washed with a saturated aqueous saline solution, dried over anhydrous magnesium sulfate. After the solvent was evaporated from the organic

layer, the residue was purified by a thin-layer chromatography (chloroform/methanol = 40/1) and triturated with hexane to give 12.8 mg (quantitatively) of the compound 8.
$^1$H-NMR(270MHz, $CDCl_3$, δ); 2.33(s, 3H), 3.86(s, 3H), 3.92(s, 3H), 6.69(d, J=15.8Hz, 1H), 7.16-7.70(m, 7H), 7.80(d, J=15.8Hz, 1H).
Fab-MS(m/z); 419[M+1]$^+$

Example 9: Compound 9 and compound 10

The compound 6 (50.4 mg, 0.139 mmol) prepared in Example 6 was dissolved in 80 mL of 1,2-dichloroethane, then 0.12 mL (1.05 mmol) of cyclohexylamine, 222.5 mg (1.050 mmol) of sodium triacetoxyborohydride and 0.063 mL (1.1 mmol) of acetic acid were added thereto and the mixture was stirred at room temperature through one night. The reaction was stopped by addition of water to the reaction solution and, after neutralizing with a saturated aqueous solution of sodium hydrogen carbonate, separation of the liquid was carried out. An organic layer was washed with a saturated aqueous saline solution, dried over anhydrous magnesium sulfate and the solvent was evaporated. The residue was purified by a thin-layer chromatography (chloroform/methanol = 15/1) to give 46.6 mg (75%) of the compound 9 and 14.0 mg (28%) of the compound 10.

Compound 9

$^1$H-NMR(270MHz, DMSO-$d_6$, δ); 1.02-1.29(m, 6H), 1.68-1.77(m, 2H), 1.88-2.06(m, 2H), 2.30(s, 3H), 2.45(m, 1H), 3.86(s, 3H), 3.98(s, 2H), 6.77(s, 1H), 7.16(dt, J=2.3Hz, 6.9Hz, 1H), 7.35(t, J=1.3Hz, 1H), 7.43-7.52(m, 2H), 7.71 (s, 1H), 10.83(brs, 1H).
Fab-MS(m/z); 446[M+1]$^+$

Compound 10

$^1$H-NMR(270MHz, DMSO-$d_6$, δ); 2.30(s, 3H), 3.85(s, 3H), 4.74(brd, J=4.3Hz, 2H), 4.51(brm, 1H), 6.79(s, 1H), 7.17(dt, J=2.3Hz, 6.9Hz, 1H), 7.36(brs, 1H), 7.44-7.53(m, 2H), 7.74(s, 1H), 10.86(brm, 1H).
Fab-MS(m/z); 365[M+1]$^+$

Example 10: Compound 11

The compound 6 (1.10 g, 3.04 mmol) prepared in Example 6 was dissolved in a mixed solvent of 250 mL of tetrahydrofuran and 125 mL of methanol, then 4.24 g (30.7 mmol) of potassium carbonate were added thereto with ice cooling and the mixture was stirred at room temperature for 5 minutes. The reaction solution was poured over ice water, the mixture was acidified with 1 mol/L hydrochloric acid and the resulting precipitate was filtered and vacuum dried at 50°C. The resulting crystals were triturated with isopropyl ether to give 851.2 mg (88%) of the compound 11.
$^1$H-NMR(270MHz, DMSO-$d_6$, δ); 4.13(s, 3H), 6.83(d, J=7.3Hz, 1H), 6.99-7.02(m, 2H), 7.25(t, J=7.3Hz, 1H), 7.78(s, 1H), 7.88(s, 1H), 9.50(s, 1H), 10.07(s, 1H), 11.08(s, 1H).
Fab-MS(m/z); 321[M+1]$^+$

Example 11: Compound 12

The compound 11 (201.2 mg, 0.06282 mmol) prepared in Example 10 was dissolved in 150 mL of dichloromethane, then 1.75 mL (12.6 mmol) of triethylamine and 0.19 mL (1.6 mmol) of valeryl chloride were added thereto and the mixture was stirred at room temperature through one night. An aqueous solution of potassium carbonate was added to the reaction solution, the mixture was extracted with dichloromethane, the organic layer was washed with a saturated saline solution, dried over anhydrous sodium sulfate and the solvent was evaporated. The residue was purified by a preparative thin-layer chromatography (chloroform/acetone = 20/1) to give 117.1 mg (46%) of the compound 12.
$^1$H-NMR(270MHz, DMSO-$d_6$, δ); 0.93(t, J=7.4Hz, 3H), 1.39(m, 2H), 1.63(m, 2H), 2.61(t, J=7.4Hz, 2H), 4.15(s, 3H), 7.20(dt, J=2.3Hz, 9.2Hz, 1H), 7.39(brs, 1H), 7.47-7.52(m, 2H), 7.80(s, 1H), 7.96(s, 1H), 10.09(s, 1H), 11.13(brs, 1H).
Fab-MS(m/z); 405[M+1]$^+$

Example 12: Compound 13

The compound 6 (569.2 mg, 1.234 mmol) prepared in Example 6 was treated with 1.4 mL (11 mmol) of N,N,N'-trimethylethylenediamine, 2.40 g (11.4 mmol) of sodium triacetoxyborohydrate and 0.71 mL (11 mmol) of acetic acid

in a similar manner to that in Example 9 and then treated with 1.72 mg (12.4 mmol) of potassium carbonate in a similar manner as that in Example 10 to give 411.9 mg (62%) of the compound 13.
$^1$H-NMR(270MHz, DMSO-$d_6$, δ); 2.15(s, 6H), 2.20(s, 3H), 2.40-2.56(m, 4H), 3.74(s, 2H), 3.88(s, 3H), 6.76(s, 1H), 6.80 (ddd, J=1.0Hz, 1.7Hz, 7.9Hz, 1H), 6.96(d, J=1.7Hz, 1H), 6.98(m, 1H), 7.22(t, J=7.9Hz, 1H), 7.66(s, 1H), 9.44(s, 1H), 10.82(brs, 1H).
Fab-MS(m/z); 407[M+1]$^+$

Example 13: Compound 14

The compound 13 (30.6 mg, 0.0753 mmol) prepared in Example 12 was dissolved in 60 mL of dichloromethane, 0.10 mL (0.75 mmol) of triethylamine and 16 mL (0.2 mmol) of ethyl isocyanate were added thereto in an argon atmosphere and the mixture was stirred through one night. The reaction was stopped by addition of water to the reaction solution, extraction was carried out with dichloromethane and the organic layer was washed with a saturated saline solution, dried over sodium sulfate. The solvent was evaporated from the organic layer and the residue was purified by a thin-layer chromatography (chloroform/methanol = 6/1) to give 26.7 mg (74%) of the compound 14.
$^1$H-NMR(270MHz, $CDCl_3$, δ); 1.22(t, J=7.3Hz, 3H), 2.27(s, 6H), 2.28(s, 3H), 2.52(m, 2H), 2.63(m, 2H), 3.33(m, 2H), 3.72(s, 2H), 3.88(s, 3H), 5.13(brm, 1H), 6.90(s, 1H), 7.21(m, 1H), 7.34(d, J=2.0Hz, 1H), 7.38-7.44(m, 3H), 7.66(brm, 1H).
Fab-MS(m/z); 478[M+1]$^+$

Example 14: Compound 15

The compound 15 (933.2 mg, 96%) was prepared from 1.10 g (3.05 mmol) of the compound 7 prepared in Example 7 and 4.24 g (30.6 mmol) of potassium carbonate in a similar manner to that in Example 10.
$^1$H-NMR(270MHz, DMSO-$d_6$, δ); 4.12(s, 3H), 6.86-6.92(m, 2H), 7.21-7.30(m, 2H), 7.78(d, J=0.7Hz, 1H), 7.83(brs, 1H), 9.47(brm, 1H), 10.08(s, 1H), 11.00(brm, 1H).
Fab-MS(m/z); 321[M+1]$^+$

Example 15: Compound 16

The compound 16 (114.1 mg, 27%) was prepared from 308.3 mg (0.9625 mmol) of the compound 15 prepared in Example 14, 1.34 mL (9.61 mmol) of triethylamine and 0.13 mL (1.2 mmol) of phenyl isocyanate in a similar manner to that in Example 13.
$^1$H-NMR(270MHz, DMSO-$d_6$, δ); 4.04(s, 3H), 6.88-7.55(m, 9H), 7.76(d, J=0.7Hz, 1H), 7.83(d, J=0.7Hz, 1H), 9.81(brs, 1H), 10.05(s, 1H), 11.06(brs, 1H).
Fab-MS(m/z); 440[M+1]$^+$

Example 16: Compound 17

The compound 17 (37.2 mg, 82%) was prepared from 41.0 mg (0.093 mmol) of the compound 16 prepared in Example 15, 82 mL (1.4 mmol) of ethanolamine, 200.0 mg (0.944 mmol) of sodium triacetoxyborohydride and 0.056 mL (0.93 mmol) of acetic acid in a similar manner to that in Example 9.
$^1$H-NMR(270MHz, DMSO-$d_6$, δ); 2.63(t, J=5.8Hz, 2H), 3.48(m, 2H), 3.73(s, 3H), 3.94(s, 2H), 4.52(t, J=5.8Hz, 1H), 6.75(s, 1H), 6.97(m, 1H), 7.18-7.42(m, 6H), 7.45-7.51(m, 2H), 7.58(s, 1H), 9.82(brs, 1H), 10.77(brm, 2H).
Fab-MS(m/z); 485[M+1]$^+$

Example 17: Compound 18

The compound 17 (16.0 mg, 0.0331 mmol) prepared in Example 16 was dissolved in 1.0 mL of dimethyl sulfoxide and allowed to stand at room temperature for 120 hours. The reaction solution was freeze-dried and the residue was purified by a preparative thin-layer chromatography (chloroform/methanol = 4/1) to give 10.0 mg (62%) of the compound 18.
$^1$H-NMR(270MHz, DMSO-$d_6$, δ); 3.51(s, 4H), 3.79(s, 3H), 4.89(s, 2H), 5.36(brs, 1H), 6.75(s, 1H), 6.80-7.00(m, 3H), 7.16-7.30(m, 4H), 7.36-7.46(m, 2H), 7.61(s, 1H), 8.81(s, 1H), 9.29(s, 1H), 10.70(brs, 1H).
Fab-MS(m/z); 485[M+1]$^+$

Example 18: Compound 19

The compound 19 (4.82 g, 57%) was prepared from 8.53 g (25.2 mmol) of the compound 3 prepared in Example 3 and 11.47 g (50.52 mmol) of DDQ in a similar manner to that in Example 6.
$^1$H-NMR(270MHz, DMSO-$d_6$, δ); 2.30(s, 3H), 3.92(s, 3H), 6.83(d, J=3.0Hz, 1H), 7.18(m, 1H), 7.35(d, J=1.0Hz, 1H), 7.44-7.49(m, 2H), 7.75-7.75(m, 2H), 10.89(s, 1H).
Fab-MS(m/z); 335$[M+1]^+$

Example 19: Compound 20

The compound 20 (5.29 g, 92%) was prepared from 5.29 g (15.8 mmol) of the compound 19 prepared in Example 18, 49 mL (630 mmol) of DMF and 14.80 mL (158.8 mmol) of phosphoryl chloride in a similar manner to that in Example 2.
$^1$H-NMR(270MHz, DMSO-$d_6$, δ); 2.30(s, 3H), 4.00(s, 3H), 7.22(m, 1H), 7.37(m, 1H), 7.46-7.53(m, 2H), 7.89(s, 1H), 8.53(s, 1H), 10.87(s, 1H), 11.21(brm, 1H).
Fab-MS(m/z); 363$[M+1]^+$

Example 20: Compound 21

The compound 21 (773.2 mg, 87%) was prepared from 1.00 g (2.77 mmol) of the compound 20 prepared in Example 19 and 3.85 g (27.8 mmol) of potassium carbonate in a similar manner to that in Example 10.
$^1$H-NMR(270MHz, DMSO-$d_6$, δ); 3.99(s, 3H), 6.83(m, 1H), 6.97-7.00(m, 2H), 7.24(dd, J=7.6Hz, 8.3Hz, 1H), 7.86(s, 1H), 8.53(s, 1H), 9.51(brs, 1H), 10.85(s, 1H), 11.21(brm, 1H).
Fab-MS(m/z); 321$[M+1]^+$

Example 21: Compound 22

The compound 22 (81.7 mg, 12%) was prepared from 504.5 mg (1.575 mmol) of the compound 21 prepared in Example 20, 8.80 mL (63.1 mmol) of triethylamine and 0.52 mL (4.7 mmol) of butyl isocyanate in a similar manner to that in Example 13.
$^1$H=NMR(270MHz, DMSO-$d_6$, δ); 0.97(t, J=7.3Hz, 3H), 1.29-1.51(m, 4H), 2.92-3.08(m, 2H), 4.01(s, 3H), 7.17(m, 1H), 7.34(s, 1H), 7.40-7.46(m, 2H), 7.78(t, J=5.6Hz, 1H), 7.92(s, 1H), 8.55(s, 1H), 10.85(s, 1H), 11.25(brm, 1H).
Fab-MS(m/z); 420$[M+1]^+$

Example 22: Compound 23

The compound 23 (97.3 mg, 94%) was prepared from 103.4 mg (0.3228 mmol) of the compound 21 prepared in Example 20, 0.60 mL (4.7 mmol) of N,N,N'-trimethylethylenediamine, 1.03 g (4.88 mmol) of sodium triacetoxyborohydride and 0.3 mL (4.8 mmol) of acetic acid in a similar manner to that in Example 9.
$^1$H-NMR(270MHz, DMSO-$d_6$, δ); 2.12(s, 6H), 2.20(s, 3H), 2.36(m, 2H), 2.50(m, 2H), 3.87(s, 3H), 4.04(s, 2H), 6.79(m, 1H), 6.93-6.96(m, 2H), 7.21(t, J=7.9Hz, 1H), 7.62(s, 1H), 7.63(s, 1H), 9.43(s, 1H), 10.85(brm, 1H).
Fab-MS(m/z); 407$[M+1]^+$

Example 23: Compound 24

The compound 24 (2.37 g, 71%) was prepared from 2.2 g (11.8 mmol) of 2-(2-hydroxymethylvinyl)-1-methylindole and 2.3 g (23.5 mmol) of maleimide in a similar manner to that in the step 2 of Example 1.
$^1$H-NMR(270MHz, DMSO-$d_6$, δ); 2.05-2.09(m, 1H), 2.31-2.42(m, 1H), 2.92-3.00(m, 1H), 3.61(s, 3H), 3.83-4.02(m, 2H), 4.16(d, J=7.6Hz, 1H), 4.79(t, J=5.3Hz, 1H), 7.00(dd, J=6.9Hz, 7.5Hz, 1H), 7.09(dd, J=6.9Hz, 7.9Hz, 1H), 7.37(d, J=7.9Hz, 1H), 7.73(d, J=7.5Hz, 1H), 11.44(s, 1H).
Fab-MS(m/z); 285$[M+1]^+$

Example 24: Compound 25

The compound 25 (73.6 mg, 68%) was prepared from 75 mg (0.37 mmol) of 2-[2-(2-hydroxyethyl)vinyl]-1-methylindole and 390 mg (0.75 mmol) of maleimide in a similar manner to that in the step 2 of Example 1.
$^1$H-NMR(270MHz, DMSO-$d_6$, δ); 2.05-2.09(m, 1H), 2.31-2.42(m, 1H), 2.92-3.00(m, 1H), 3.33(t, J=6.7Hz, 2H), 3.61(s, 3H), 3.83-4.02(m, 2H), 4.16(d, J=7.6Hz, 1H), 4.79(t, J=5.3Hz, 1H), 7.00(dd, J=6.9Hz, 7.6Hz, 1H), 7.09(dd, J=6.9Hz,

7.9Hz, 1H), 7.37(d, J=7.9Hz, 1H), 7.73(d, J=7.6Hz, 1H), 11.54(s, 1H).
Fab-MS(m/z); 299[M+1]$^+$

Example 25: Compound 26

The compound 26 (62.5 mg, 94%) was prepared from 50 mg (0.16 mmol) of 1-benzyl-2-[2-(1,3-dioxolan-2-ylmethyl)vinyl]indole and 32 mg (0.32 mmol) of maleimide in a similar manner to that in the step 2 of Example 1.
$^1$H-NMR(270MHz, DMSO-$d_6$, δ); 1.03(d, J=6.3Hz, 2H), 3.57-3.85(m, 5H), 4.15-4.20(m, 1H), 4.92-4.95(m, 1H), 5.36(s, 2H), 7.00-7.09(m, 4H), 7.21-7.31(m, 3H), 7.39(d, J=7.3Hz, 1H), 7.76(d, J=8.9Hz, 1H), 10.91(s, 1H).
Fab-MS(m/z); 202[M+1]$^+$

Example 26: Compound 27

The compound 27 (1.03 g, 76%) was prepared by the reaction of 1.38 g (4.4 mmol) of 2-(2-methoxycarbonylvinyl)-1-methylindole with 850 mg (8.76 mmol) of maleimide in a similar manner to that in the step 2 of Example 1 and then treated with 4.80 g (21.2 mmol) of DDQ in a similar manner to that in Example 6.
$^1$H-NMR(270MHz, DMSO-$d_6$, δ); 3.93(s, 3H), 7.38(dd, J=7.2Hz, 7.8Hz, 1H), 7.62(dd, J=7.2Hz, 8.1Hz, 1H), 7.73(d, J=8.1Hz, 1H), 8.13(s, 1H), 8.87(d, J=7.8Hz, 1H), 11.31(s, 1H).
Fab-MS(m/z); 309[M+1]$^+$

Example 27: Compound 28

The compound 27 (9.85 g, 32 mmol) prepared in Example 26 was dissolved in 200 mL of 1,4-dioxane, 5 mL of 6 mol/L hydrochloric acid were added thereto and the mixture was heated to reflux for 2.5 hours. The reaction solution was cooled to room temperature, 50 mL of methanol were added thereto and the crystals separated out therefrom were filtered and dried to give 7.94 g (84%) of the compound 28.
$^1$H-NMR(270MHz, DMSO-$d_6$, δ); 3.99(s, 3H), 7.38(dd, J=7.3Hz, 7.9Hz, 1H), 7.64(dd, J=7.3Hz, 7.9Hz, 1H), 7.76(d, J=7.9Hz, 1H), 8.17(s, 1H), 8.85(d, J=7.9Hz, 1H), 11.46(s, 1H).
Fab-MS(m/z); 295[M+1]$^+$

Example 28: Compound 29

Step 1

The compound 28 (800 mg, 2.7 mmol) prepared in Example 27 was dissolved in 10 mL of 1,4-dioxane, 5.0 mL (69 mmol) of thionyl chloride were added thereto and the mixture was heated to reflux for 2 hours. The reaction solution was vacuum concentrated to give 812 mg (96%) of an acid chloride.

Step 2

2-Amino-2-methylpropanol (0.050 mL, 0.52 mmol) was dissolved in 0.5 mL of 1,2-dichloroethane, 0.10 mL (0.72 mmol) of triethylamine was added thereto, then 50 mg (0.16 mmol) of the acid chloride prepared in the step 1 were added thereto and the mixture was stirred for 1 hour. The solvent was evaporated from the reaction solution and the residue was subjected to a trituration using chloroform to give 42.3 mg (67%) of the compound 29.
$^1$H-NMR(270MHz, DMSO-$d_6$, δ); 0.92(s, 6H), 3.89(s, 2H), 3.97(s, 3H), 7.32(dd, J=7.3Hz, 7.9Hz, 1H), 7.70(dd, J=7.3Hz, 7.9Hz, 1H), 7.82(d, J=7.9Hz, 1H), 8.18(s, 1H), 8.80(d, J=7.9Hz, 1H), 9.82-9.85(m, 1H), 11.45(s, 1H).
Fab-MS(m/z); 366[M+1]$^+$

Example 29: Compound 30

The compound 30 (42.3 mg, 67%) was prepared from 50 mg (0.16 mmol) of the acid chloride prepared in the step 1 of Example 28, 0.10 mL (0.72 mmol) of triethylamine and 37.3 mg (0.24 mmol) of D,L-serine methyl ester in a similar manner to that in the step 2 of Example 28.
Fab-MS(m/z); 396[M+1]$^+$

Example 30: Compound 31

The compound 31 (24.8 mg, 41%) was prepared from 50 mg (0.16 mmol) of the acid chloride prepared in the

step 1 of Example 28, 0.10 mL (0.72 mmol) of triethylamine and 0.05 mL (0.8 mmol) of 1-aminomorpholine in a similar manner to that in the step 2 of Example 28.
$^1$H-NMR(270MHz, DMSO-$d_6$, δ); 3.71-3.87(m, 4H), 3.89-4.00(m, 4H), 7.32(dd, J=7.3Hz, 7.9Hz, 1H), 7.64(dd, J=7.3Hz, 7.9Hz, 1H), 7.76(d, J=7.9Hz, 1H), 8.17(s, 1H), 8.74(d, J=7.9Hz, 1H), 9.81-9.85(m, 1H), 11.45(s, 1H).
Fab-MS(m/z); 379[M+1]$^+$

Example 31: Compound 32

The compound 32 (42.3 mg, 76%) was prepared from 50 mg (0.16 mmol) of the acid chloride prepared in the step 1 of Example 28, 0.10 mL (0.72 mmol) of triethylamine and 17.8 mg (0.240 mmol) of acetohydrazide in a similar manner to that in the step 2 of Example 28.
$^1$H-NMR(270MHz, DMSO-$d_6$, δ); 3.79(s, 3H), 3.96(s, 3H), 7.32(dd, J=7.3Hz, 7.9Hz, 1H), 7.70(t, J=7.3Hz, 1H), 7.82(d, J=7.3Hz, 1H), 8.20(s, 1H), 8.80(d, J=7.9Hz, 1H), 9.80-9.82(m, 1H), 11.45(s, 1H).
Fab-MS(m/z); 351[M+1]$^+$

Example 32: Compound 33

The compound 33 (39.7 mg, 69%) was prepared from 50 mg (0.16 mmol) of the acid chloride prepared in the step 1 of Example 28, 0.10 mL (0.72 mmol) of triethylamine and 20.2 mg (0.240 mmol) of 4-amino-1,2,4-triazole in a similar manner to that in the step 2 of Example 28.
Fab-MS(m/z); 361[M+1]$^+$

Example 33: Compound 34

The compound 34 (2.42 g, quantitatively) was prepared from 2.36 g (8.3 mmol) of the compound 24 prepared in Example 23 and 3.5 g (15 mmol) of DDQ in a similar manner to that in Example 6.
$^1$H-NMR(270MHz, DMSO-$d_6$, δ); 3.93(s, 3H), 5.09(s, 2H), 7.33(dd, J=7.3Hz, 7.6Hz, 1H), 7.65(dd, J=7.3Hz, 8.6Hz, 1H), 7.70(d, J=8.6Hz, 1H), 8.05(s, 1H), 8.82(d, J=7.6Hz, 1H), 11.30(s, 1H).
Fab-MS(m/z); 281[M+1]$^+$

Example 34: Compound 35

The compound 35 (22.7 mg, 45%) was prepared from 50 mg (0.17 mmol) of the compound 25 prepared in Example 24 and 80 mg (0.35 mmol) of DDQ in a similar manner to that in Example 6.
$^1$H-NMR(270MHz, DMSO-$d_6$, δ); 3.31(t, J=6.77Hz, 2H), 3.73(t, J=6.93Hz, 2H), 3.87(s, 3H), 7.30(dd, J=6.93Hz, 7.26Hz, 1H), 7.54-7.64(m, 2H), 7.68(s, 1H), 8.78(d, J=7.92Hz, 1H), 11.05(s, 1H).
Fab-MS(m/z); 295[M+1]$^+$

Example 35: Compound 36

Triphenyl phosphine (8.42 g, 32.2 mmol) was dissolved in 200 mL of DMF in an argon atmosphere and 1. 66 mL (32.2 mmol) of bromine were dropped thereinto at -10°C. After the reaction solution was stirred for 10 minutes, 6.00 g (21.4 mmol) of the compound 34 prepared in Example 33 was added to the reaction solution and the mixture was raised to room temperature and stirred for 18 hours. The solvent was evaporated from the reaction solution and the residue was subjected to trituration with methanol to give 2.57 g (23%) of the compound 36.
$^1$H=NMR(270MHz, DMSO-$d_6$, δ); 3.96(s, 3H), 5.20(s, 2H), 7.36(dd, J=7.3Hz, 7.6Hz, 1H), 7.65(dd, J=7.3Hz, 8.6Hz, 1H), 7.71(d, J=8.6Hz, 1H), 8.04(s, 1H), 8.82(d, J=7.6Hz, 1H), 11.27(s, 1H).
Fab-MS(m/z); 343[M+1]$^+$

Example 36: Compound 37

The compound 36 (50 mg, 0.15 mmol) prepared in Example 35 was dissolved in 1 mL of DMF, 1. 0 mL (17 mmol) of ethanolamine was added thereto and the mixture was stirred at room temperature for 30 minutes. The solvent was evaporated from the reaction solution and the residue was subjected to a trituration with methanol to give 33.5 mg (71%) of the compound 37.
Fab-MS(m/z); 324[M+1]$^+$

Example 37: Compound 38

The compound 38 (44.9 mg, 83%) was prepared from 50 mg (0.15 mmol) of the compound 36 prepared in Example 35 and 0.10 mL (0.98 mmol) of 3-aminomethylpyridine in a similar manner to that in Example 36.
Fab-MS(m/z); 371[M+1]$^+$

Example 38: Compound 39

The compound 39 (10.7 mg, 19%) was prepared from 50 mg (0.15 mmol) of the compound 36 prepared in Example 35 and 0.10 mL (0.83 mmol) of 1-amino-4-methylpiperazine in a similar manner to that in Example 36.
$^1$H-NMR(270MHz, DMSO-$d_6$, δ); 2.12-2.25(m, 4H), 2.36-2.39(m, 4H), 3.94(s, 3H), 3.98(s, 3H), 4.07(s, 2H), 7.34(t, J=7.3Hz, 1H), 7.62(t, J=7.3Hz, 1H), 7.69(d, J=7.3Hz, 1H), 7.87(s, 1H), 8.84(d, J=7.3Hz, 1H), 11.13(s, 1H). Fab-MS(m/z); 378[M+1]$^+$

Example 39: Compound 40

The compound 40 (6.2 g, 24%) was prepared when 18.8 g (73.7 mmol) of 1-allyl-2-(2-ethoxycarbonylvinyl) indole was reacted with 7.15 g ( 147 mmol ) of maleimide in the similar manner to that in the step 2 of Example 1 and then treated with 8.4 g (37 mmol) of DDQ in the similar manner to that in Example 6.
$^1$H-NMR(270MHz, DMSO-$d_6$, δ); 1.36(t, J=7.2Hz, 3H), 4.39(q, J=7.2Hz, 2H), 4.88(d, J=17.0Hz, 1H), 5.11(d, J=10.2Hz, 1H), 5.20(d, J=4.0Hz, 2H), 6.02-6.06(m, 1H), 7.39(dd, J=7.1Hz, 7.8Hz, 1H), 7.65(dd, J=7.1Hz, 8.3Hz, 1H), 7.72(d, J=8.3Hz, 1H), 8.10(s, 1H), 8.91(d, J=7.8Hz, 1H), 11.33(s, 1H).
Fab-MS(m/z); 349[M+1]$^+$

Example 40: Compound 41

The compound 41 (4.04 g, 93%) was prepared in the similar manner to that in Example 27 from 4.70 g (13.6 mmol) of the compound 40 prepared in Example 39.
$^1$H-NMR(270MHz, DMSO-$d_6$, δ); 4.89(d, J=17.2Hz, 1H), 5.12(d, J=10.2Hz, 1H), 5.20(d, J=3.6Hz, 2H), 5.97-6.07(m, 1H), 7.40(dd, J=6.9Hz, 7.8Hz, 1H), 7.65(dd, J=6.9Hz, 8.2Hz, 1H), 7.75(d, J=8.2Hz, 1H), 8.17(s, 1H), 8.92(d, J=7.8Hz, 1H), 11.48(s, 1H). Fab-MS(m/z); 321[M+1]$^+$

Example 41: Compound 42

The compound 42 (1.08 g, 96%) was prepared in the similar manner to that in the step 1 and the step 2 of Example 28 from 1.0 g (3.1 mmol) of the compound 41 prepared in Example 40 and 0.21 mL (3.4 mmol) of ethanolamine.
$^1$H-NMR(270MHz, DMSO-$d_6$, δ); 3.45(t, J=6.4Hz, 2H), 3.60(t, J=6.4Hz, 2H), 4.87(d, J=17.2Hz, 1H), 5.12(d, J=10.2Hz, 1H), 5.20 (d, J=4.0Hz, 2H), 5.95-6.09 (m, 1H), 7.39(dd, J=7.1Hz, 7.9Hz, 1H), 7.65(dd, J=7.1Hz, 8.3Hz, 1H), 7.73(d, J=8.3Hz, 1H), 8.24(s, 1H), 8.94(d, J=7.9Hz, 2H), 9.43(t, J=5.3Hz, 1H), 11.33(brs, 1H).
Fab-MS(m/z); 364[M+1]$^+$

Example 42: Compound 43

The compound 42 (500 mg, 1.38 mmol) prepared in Example 41 was dissolved in 10 mL of tetrahydrofuran, 31.5 mg (0.13 mmol) of Burgess reagent were added and the mixture was stirred at 70°C for 50 minutes. Water was added to the reaction solution, the mixture was extracted with chloroform, the organic layer was washed with a saturated saline solution, dried over anhydrous sodium sulfate and the solvent was evaporated. The residue was purified by a preparative thin-layer chromatography (chloroform/methanol = 9/1) to give 27.2 mg (73%) of the compound 43.
$^1$H-NMR(270MHz, DMSO-$d_6$, δ); 4.03(t, J=9.5Hz, 2H), 4.48(t, J=9.5Hz, 2H), 4.87(d, J=17.2Hz, 1H), 5.11(d, J=10.2Hz, 1H), 5.23(brs, 2H), 5.96-6.06(m, 1H), 7.41(dd, J=7.1Hz, 7.8Hz, 1H), 7.66(dd, J=7.1Hz, 8.1Hz, 1H), 7.75(d, J=8.1Hz, 1H), 8.08(s, 1H), 8.94(d, J=7.8Hz, 1H).
Fab-MS(m/z); 346[M+1]$^+$

Example 43: Compound 44

The compound 41 (100 mg, 0.3 mmol) prepared in Example 40 was suspended in 2 mL of tetrahydrofuran, then 0.14 mL (0.63 mmol) of diphenylphosphoric azide, 0.15 mL of ethanol and 0.08 mL (0.6 mmol) of triethylamine were added thereto and the mixture was heated to reflux for 4 hours. After the reaction solution was cooled to room

temperature, the crystals separated out therefrom were filtered to give 32.5 mg (30%) of the compound 44.
$^1$H-NMR(270MHz, DMSO-$d_6$, δ); 1.29(t, J=7.0Hz, 3H), 4.21(q, J=7.0Hz, 2H), 4.88(d, J=17.2Hz, 1H), 5.00-5.01(m, 2H), 5.14(d, J=10.2Hz, 1H), 5.95-6.05(m, 1H), 7.30(dd, J=7.1Hz, 7.6Hz, 1H), 7.54(dd, J=7.1Hz, 8.3Hz, 1H), 7.63(d, J=8.3Hz, 1H), 8.26(s, 1H), 8.68(d, J=7.6Hz, 1H), 9.17(s, 1H), 11.33(s, 1H).
Fab-MS(m/z)364[M+1]$^+$

Example 44: Compound 45

The compound 41 (1.0 g, 3.1 mmol) prepared in Example 40 was suspended in 15 mL of tetrahydrofuran, then 1. 4 mL (6.3 mmol) of diphenylphosphoric azide and 0.80 mL (6.3 mmol) of triethylamine were added thereto, the mixture was heated to reflux for 1 hour, then 1.0 mL of water was added thereto and the mixture was further heated to reflux for 2 hours. After the reaction solution was cooled to roomtemperature, the solvent was evaporated and the residue was subjected to trituration with methanol to give 942.1 mg (quantitatively) of the compound 45.
$^1$H-NMR(270MHz, DMSO-$d_6$, δ); 4.89-4.94(m, 3H), 5.14(d, J=10.2Hz, 1H), 5.82-6.20(m, 1H), 6.38(s, 2H), 6.86(s, 1H), 7.21(dd, J=7.1Hz, 7.6Hz, 1H), 7.42(dd, J=7.1Hz, 7.9Hz, 1H), 7.48(d, J=7.9Hz, 1H), 8.63(d, J=7.6Hz, 1H), 10.88(s, 1H).
Fab-MS(m/z)292[M+1]$^+$

Example 45: Compound 46

The compound 46 (13.0 g, 53%) was prepared when 18.3 g (99.0 mmol) of 1-allyl-2-(2-hydroxymethylvinyl) indole was made to react with 19.0 g (198 mmol) of maleimide in the similar manner to that in the step 2 of Example 1 and then treated with 36.6 g (160 mmol) of DDQ in the similar manner to that in Example 6.
$^1$H-NMR(270MHz, DMSO-$d_6$, δ); 4.87(d, J=17.2Hz, 1H), 5.10(d, J=10.2Hz, 1H), 5.16-5.17(m, 4H), 5.92-6.05(m, 1H), 7.36(m, 1H), 7.63-7.71(m, 2H), 8.15(s, 1H), 8.83(d, J=7.9Hz, 1H), 10.91(s, 1H).
Fab-MS(m/z); 307[M+1]$^+$

Example 46: Compound 47

The compound 46 (1.53 g, 5 mmol) prepared in Example 45 was suspended in 50 mL of dichloromethane, then 190.2 mg (1.0 mmol) of p-toluenesulfonic acid and 1.37 mL (15 mmol) of 3,4-dihydro-2H-pyran were added thereto and the mixture was stirred at room temperature for 30 minutes in an argon atmosphere. A saturated aqueous solution of sodium hydrogen carbonate was added to the reaction solution and the mixture was extracted with chloroform. The organic layer was washed with a saturated aqueous saline solution, dried over anhydrous sodium sulfate and the solvent was evaporated. The residue was purified by a silica gel column chromatography (chloroform/methanol = 20/1) to give 1.45 g (75%) of the compound 47.
Fab-MS(m/z); 391[M+1]$^+$

Example 47: Compound 48

The compound 46 (71.9 mg, 0.235 mmol) prepared in Example 45 was dissolved in 5 mL of tetrahydrofuran, then 0.140 mL (0.936 mmol) of DBU and 341.8 mg (0.936 mmol) of bis(p-nitrophenyl) phosphoric azide were added thereto and the mixture was heated to reflux for 1 hour. After cooling the reaction solution to room temperature, ice water was added thereto with stirring. The resulting precipitate was filtered, subjected to trituration with methanol and recrystallized from ethyl acetate to give 35.8 mg (46%) of the compound 48.
$^1$H-NMR(270MHz, DMSO-$d_6$, δ); 4.94(dd, J=1.3Hz, 17.2Hz, 1H), 5.02(s, 2H), 5.14(dd, J=1.3Hz, 10.6Hz, 1H), 5.19(d, J=5.0Hz, 2H), 6.02 (ddt, J=5.0Hz, 10.6Hz, 17.2Hz, 1H), 7.38(dd, J=6.9Hz, 7.8Hz, 1H), 7.63(ddd, J=1.2Hz, 6.9Hz, 8.2Hz, 1H), 7.73(d, J=8.2Hz, 1H), 7.99(s, 1H), 8.89(d, J=7.8Hz, 1H), 11.27(brs, 1H).
Fab-MS(m/z); 332[M+1]$^+$

Example 48: Compound 49

The compound 48 (10.8 mg, 0.0326 mmol) prepared in Example 47 was dissolved in 1 mL of tetrahydrofuran, then 13.1 mg (0.0499 mmol) of triphenylphosphine were added thereto and the mixture was stirred for 20 minutes. Water (0.080 mL) was added to the reaction solution and the mixture was stirred at 60°C for 2 hours and then stirred through one night at room temperature. The reaction solution was purified by a preparative thin-layer chromatography (chloroform/methanol = 4/1) to give 2.9 mg (29%) of the compound 49.
$^1$H-NMR(270MHz, DMSO-$d_6$, δ); 4.24(s, 2H), 4.96(dd, J=1.3Hz, 16.8Hz, 1H), 5.15(m, 3H), 6.03(m, 1H), 7.35(dd, J=6.9Hz, 7.6Hz, 1H), 7.59(ddd, J=1.1Hz, 6.9Hz, 8.2Hz, 1H), 7.68(d, J=8.2Hz, 1H), 8.00(s, 1H), 8.87(d, J=7.6Hz, 1H).

Fab-MS(m/z); 306[M+1]+

Example 49: Compound 50

The compound 50 (3.69 g, 82%) was prepared when 3.51 g (16.6 mmol) of 1-allyl-2-(2-formylvinyl)indole was made to react with 3.24 g (33.4 mmol) of maleimide in the similar manner to that in the step 2 of Example 1 and then treated with 6.72 g (29.6 mmol) of DDQ in the similar manner to that in Example 6.
$^1$H-NMR(270MHz, DMSO-$d_6$, δ); 4.90(dd, J=1.5Hz, 17.2Hz, 1H), 5.13(dd, J=1.5Hz, 10.2Hz, 1H), 5.26(d, J=5.0Hz, 2H), 6.02(m, 1H), 7.42(ddd, J=1.2Hz, 6.7Hz, 7.9Hz, 1H), 7.70(m, 2H), 8.28(s, 1H), 8.94(d, J=7.9Hz, 1H), 11.00(s, 1H), 11.50 (brs, 1H).
Fab-MS(m/z); 305[M+1]+

Example 50: Compound 51

The compound 50 (50.8 mg, 0.167 mmol) prepared in Example 49 was dissolved in 3 mL of tetrahydrofuran, then 32. 8 mg (0.472 mmol) of hydroxylamine hydrochloride and 0.050 mL (0.18 mmol) of triethylamine were added thereto and the mixture was stirred at room temperature for one day. The resulting precipitate was filtered and subjected to trituration with a mixed solvent of tetrahydrofuran and water to give 12.5 mg (23%) of the compound 51.
$^1$H-NMR(270MHz, DMSO-$d_6$, δ); 4.91(dd, J=1.1Hz, 17.0Hz, 1H), 5.16(m, 3H), 6.03(m, 1H), 7.38(t, J=7.4Hz, 1H), 7.64 (ddd, J=1.0Hz, 6.9Hz, 7.3Hz, 1H), 7.73(d, J=8.3Hz, 1H), 8.16(s, 1H), 8.90(d, J=7.9Hz, 1H), 9.12(s, 1H), 11.72(brs, 1H).
Fab-MS(m/z); 320[M+1]+

Example 51: Compound 52

The compound 52 (17.6 mg, 69%) was prepared from 20.9 mg (0.069 mmol) of the compound 50 prepared in Example 49 and 13.5 mg (0.162 mmol) of O-methylhydroxylamine hydrochloride in a similar manner to that in Example 50.
$^1$H-NMR(270MHz, DMSO-$d_6$, δ); 3.95(s, 3H), 4.90(d, J=17.2Hz, 1H), 5.15(m, 3H), 6.04(m, 1H), 7.38(t, J=7.3Hz, 1H), 7.64(dd, J=7.3Hz, 8.3Hz, 1H), 7.75(d, J=8.3Hz, 1H), 8.90(s, 1H), 8.90(d, J=7.3Hz, 1H), 9.76(s, 1H), 11.36(brs, 1H).
Fab-MS(m/z); 334[M+1]+

Example 52: Compound 53

The compound 53 (12.8 mg, 45%) was prepared from 20.0 mg (0.066 mmol) of the compound 50 prepared in Example 49 and 11.3 mg (0.074 mmol) of p-hydrazinobenzoic acid in a similar manner to that in Example 50.
$^1$H-NMR(270MHz, DMSO-$d_6$, δ); 5.03(dd, J=1.7Hz, 17.2Hz, 1H), 5.20(dd, J=1.3Hz, 10.2Hz, 1H), 5.26(m, 2H), 6.07(m, 1H), 7.26(d, J=8.8Hz, 2H), 7.36(ddd, J=1.0Hz, 6.9Hz, 7.9Hz, 1H), 7.62(ddd, J=1.0Hz, 6.9Hz, 8.3Hz, 1H), 7.70(d, J=8.3Hz, 1H), 7.86(d, J=8.8Hz, 2H), 8.40(s, 1H), 8.90(d, J=7.9Hz, 1H), 9.11(s, 1H), 11.28(brs, 1H), 11.36(s, 1H).
Fab-MS(m/z); 439[M+1]+

Example 53: Compound 54

The compound 54 (16.9 mg, 71%) was prepared from 20.1 mg (0.0661 mmol) of the compound 50 prepared in Example 49 and 0.0050 mL (0.074 mmol) of 2-hydroxyethylhydrazine in a similar manner to that in Example 50.
$^1$H-NMR(270MHz, DMSO-$d_6$, δ); 3.32(m, 2H), 3.62(m, 2H), 4.71(t, J=5.3Hz, 1H), 4.86 (dd, J=1.3Hz, 17.2Hz, 1H), 5.13 (m, 3H), 6.03(m, 1H), 7.33(ddd, J=1.2Hz, 6.9Hz, 7.8Hz, 1H), 7.58(ddd, J=1.3Hz, 6.9Hz, 8.3Hz, 1H), 7.65(d, J=8.3Hz, 1H), 7.97(t, J=4.8Hz, 1H), 8.09(s, 1H), 8.61(s, 1H), 8.87(d, J=7.8Hz, 1H), 11.15(brs, 1H).
Fab-MS(m/z); 363[M+1]+

Example 54: Compound 55

The compound 55 (12.4 mg, 47%) was prepared from 20.1 mg (0.0661 mmol) of the compound 50 prepared in Example 49 and one drop of 2-hydrazinopyridine in a similar manner to that in Example 50.
Fab-MS(m/z): 396[M+1]+

Example 55: Compound 56

The compound 56 (14.7 mg, 66%) was prepared from 21.2 mg (0.070 mmol) of the compound 50 prepared

in Example 49 , 34.7 mg (0.331 mmol) of hydrazine dihydrochloride and 0.118 mL (0.847 mmol) of triethylamine in a similar manner to that in Example 50.
$^1$H-NMR(270MHz, DMSO-$d_6$, δ); 4.87(dd, J=1.3Hz, 17.2Hz, 1H), 5.15 (m, 3H), 6.02(m, 1H), 7.34(ddd, J=1.0Hz, 6.9Hz, 7.9Hz, 1H), 7.59(ddd, J=1.3Hz, 6.9Hz, 8.2Hz, 1H), 7.67(d, J=8.2Hz, 1H), 8.12(s, 1H), 8.77(s, 1H), 8.88(d, J=7.9Hz, 1H), 11.17(brs, 1H).
Fab-MS(m/z); 319[M+1]$^+$

Example 56: Compound 57

The compound 46 (1.0 g, 3.2 mmol) prepared in Example 45 was dissolved in 100 mL of tetrahydrofuran, and 3.9 g (16 mmol) of a 9-borabicyclononane dimer was added thereto with ice cooling, the mixture was stirred at room temperature for 16 hours and cooled with ice again, 11 mL of a 1 mol/L aqueous solution of sodium hydroxide and 11 mL of an aqueous solution of hydrogen peroxide were added thereto and the mixture was stirred at room temperature for 15 minutes. A saturated aqueous saline solution was added to the reaction solution, the mixture was extracted with tetrahydrofuran, the organic layer was washed with a saturated aqueous saline solution, dried over anhydrous sodium sulfate and the solvent was evaporated. The residue was purified by a column chromatography (chloroform/methanol = 9/1) to give 123.8 mg (12%) of the compound 57.
Fab-MS(m/z); 325[M+1]$^+$

Example 57: Compound 58

The compound 58 (99.6 mg, 74%) was prepared from 100 mg (0.3 mmol) of the compound 57 prepared in Example 56, 243 mg (0.9 mmol) of triphenylphosphine and 0.04 mL (0.9 mmol) of bromine in a similar manner to that in Example 35.
$^1$H-NMR(270MHz, DMSO-$d_6$, δ); 2.28-2.35(m, 2H), 3.54(t, J=6.5Hz, 2H), 4.57(t, J=7.3Hz, 2H), 5.20(s, 2H), 7.36 (dd, J=7.3Hz, 7.9Hz, 1H), 7.65(dd, J=7.3Hz, 8.3Hz, 1H), 7.75(d, J=8.3Hz, 1H), 8.09(s, 1H), 8.84(d, J=7.9Hz, 1H), 11.29(s, 1H).
Fab-MS(m/z); 449[M+1]$^+$

Example 58: Compound 59

The compound 59 (44.9 mg, 88%) was prepared from 50 mg (0.11 mmol) of the compound 58 prepared in Example 57 and 0.025 mL (0.28 mmol) of morpholine in a similar manner to that in Example 36.
Fab-MS(m/z); 463[M+1]$^+$

Example 59: Compound 60

The compound 60 (343.2 mg, 84%) was prepared when 390 mg (1.0 mmol) of the compound 47 prepared in Example 46 was made to react with 487 mg (2.0 mmol) of 9-borabicyclononane and the reaction solution was treated with an aqueous solution of sodium hydroxide and an aqueous solution of hydrogen peroxide in a similar manner to that in Example 56.
Fab-MS(m/z); 391[M+1]$^+$

Example 60: Compound 61

The compound 61 (2.27 g, 56%) was prepared from 3.82 g (9.4 mmol) of the compound 60 prepared in Example 59, 2.1 mL (14 mmol) of DBU and 5.15 g (14.1 mmol) of bis(p-nitrophenyl)phosphoric azide in a similar manner to that in Example 47.
$^1$H-NMR(270MHz, DMSO-$d_6$, δ); 1.53-1.80(m, 8H), 1.84-2.10(m, 2H), 3.52-3.65(m, 1H), 3.80-4.00(m, 1H), 4.40-4.58 (m, 2H), 4.82-4.90(m, 1H), 5.05(d, J=14.2Hz, 1H), 5.21(d, J=14.2Hz, 1H), 7.35(dd, J=7.1Hz, 7.86Hz, 1H), 7.62(dd, J=7.1Hz, 8.3Hz, 1H), 7.74(d, J=8.3Hz, 1H), 7.96(s, 1H), 8.85(d, J=7.8Hz, 1H), 11.15(brs, 1H).
Fab-MS(m/z); 434[M+1]$^+$

Example 61: Compound 62

The compound 61 (1.6 g, 3.8 mmol) prepared in Example 60 was dissolved in a mixed solvent of 24 mL of tetrahydrofuran and 96 mL of ethanol and stirred at room temperature for 5 hours in a hydrogen atmosphere in the presence of 1 g of 10% palladium-carbon. The reaction solution was filtered through Celite, the filtrate was vacuum

concentrated and the residue was subjected to trituration with methanol to give 1. 53 g (99%) of the compound 62.
Fab-MS(m/z); 408[M+1]$^+$

Example 62: Compound 63

The compound 62 (120 mg, 0.1 mmol) prepared in Example 61 was dissolved in 2 mL of dichloromethane, then 0.07 mL (0.2 mmol) of benzoyl chloride was added thereto in the presence of triethylamine and the mixture was stirred at room temperature for 30 minutes. Aminomethyl resin (250 mg, 0.8 mmol/g; manufactured by Kokusan Kagaku) was added to the reaction solution, the mixture was stirred for 2 hours, 200mg (1.6 mmol/g; Bio-Rad) of AG1-X8 resin was added thereto and the mixture was stirred for 30 minutes. The reaction solution was filtered, the filtrate was vacuum concentrated and the residue was purified by a preparative thin-layer chromatography (chloroform/methanol = 9/1) to give 137.0 mg (89%) of the compound 63.
Fab-MS(m/z); 512[M+1]$^+$

Example 63: Compound 64

Step 1

2-(2-Acetoxymethylvinyl)indole (12.94 g, 60.12 mmol) was reacted with 23.47 g (69.15 mmol) of N-triphenylmethylmaleimide in a similar manner to that in the step 2 of Example 1 and then reaction product was treated with 18.11 g (79.78 mmol) of DDQ in a similar manner to that in Example 6 to give 18.28 g (56%) of 4-acetoxymethyl-1,3-dioxo-1,2,3,6-tetrahydro-2-triphenylme thylpyrrolo[3,4-c]carbazole.
$^1$H-NMR(270MHz, $CDCl_3$, δ); 2.13(s, 3H), 5.45(s, 2H), 7.17-7.33(m, 11H), 7.43(s, 1H), 7.46(ddd, J=1.2Hz, 7.2Hz, 8.4Hz, 1H), 7.58(dd, J=1.7Hz, 7.3Hz, 6H), 8.08(brs, 1H), 8.80(d, J=7.6Hz, 1H).

Step 2

4-Acetoxymethyl-1,3-dioxo-1,2,3,6-tetrahydro-2-tri-p henylmethylpyrrolo[3,4-c]carbazole (18.28 g, 33.82 mmol) prepared in the step 1 of Example 63 was dissolved in a mixed solvent of 300 mL of tetrahydrofuran and 100 mL of methanol, then 4.70 g (34.0 mmol) of potassium carbonate was added thereto and the mixture was stirred at room temperature for 1 hour. Ice water was added to the reaction solution and the resulting precipitate was filtered. After that, the resulting precipitate was treated with 8.13 g (35.8 mmol) of DDQ in a similar manner to that in Example 6 to give 5.40 g (35%) of 1,3-dioxo-4-formyl-1,2,3,6-tetrahydro-2-triphenylmethyl-pyrrolo[3,4-c]carbazole.
$^1$H-NMR(270MHz, $CDCl_3$, δ); 7.20-7.35(m, 10H), 7.41(d, J=8.2Hz, 1H), 7.56(ddd, J=1.2Hz, 7.3Hz, 8.2Hz, 1H), 7.59 (dd, J=1.5Hz, 7.1Hz, 6H), 8.14(s, 1H), 8.48(brs, 1H), 8.91(d, J=7.6Hz, 1H), 10.94(s, 1H).
Fab-MS(m/z); 507[M+1]$^+$

Step 3

1,3-dioxo-4-formyl-1,2,3,6-tetrahydro-2-triphenyl-me thylpyrrolo[3,4-c]carbazole (405.3 mg, 0.800 mmol) prepared in the step 2 of Example 63 was dissolved in 10 mL of DMF and then 386.0 mg (2.44 mmol) of 3-dimethylaminopropyl chloride hydrochloride was added thereto. A solution (9.6 mL, 4.8 mmol) of 0.5 mol/L potassium tert-butoxide in 2-methyl-2-propanol was dropped over 30 minutes into the mixture at 100°C. The mixture was stirred for 2 0 minutes at that temperature and cooled to room temperature, ice water was added thereto and the resulting precipitate was filtered to give 175.7 mg (37%) of 6-(3-dimethylaminopropyl)-1,3-dioxo-4-formyl-1,2,3,6-tetra hydro-2-triphenylmethylpyrrolo[3,4-c]carbazole.
$^1$H-NMR(270MHz, $CDCl_3$, δ); 1.92(m, 2H), 2.12(s, 6H), 2.50(m, 2H), 4.63(m, 2H), 7.19(t, J=7.6Hz, 3H), 7.30(t, J=7.6Hz, 6H), 7.37(m, 1H), 7.58(d, J=7.6Hz, 6H), 7.70(m, 1H), 7.81(d, J=8.2Hz, 1H), 8.45(s, 1H), 8.78(d, J=7.9Hz, 1H), 10.89 (s, 1H).
Fab-MS(m/z); 592[M+1]$^+$

Step 4

Trifluoroacetic acid (2 mL) and 1 mL of dichloromethane were added to 169.0 mg (0.286 mmol) of 6-(3-dimethylaminopropyl)-1,3-dioxo-4-formyl-1,2,3,6-tetra hydro-2-triphenylmethylpyrrolo[3,4-c]carbazole prepared in the step 3 of Example 63 and the mixture was stirred at room temperature for 2.5 hours. The solvent was evaporated and the residue was subjected to trituration with water and purified by a preparative thin-layer chromatography (chloroform/methanol/aqueous ammonia = 9/1/1) to give 73.2 mg (73%) of a trifluoroacetate of the compound 64.

$^1$H-NMR(270MHz, $CDCl_3$, δ); 2.04(m, 2H), 2.41(s, 6H), 2.64(m, 2H), 4.63(t, J=6.8Hz, 2H), 7.44(dd, J=7.3Hz, 8.1Hz, 1H), 7.74(dd, J=7.3Hz, 8.3Hz, 1H), 7.84(d, J=8.3Hz, 1H), 8.41(s, 1H), 8.96(d, J=8.1Hz, 1H), 11.05(s, 1H), 11.51(brs, 1H).
Fab-MS(m/z); 350[M+1]$^+$

Example 64: Compound 65

The compound 65 (8.6 mg, 82%) was prepared from 10.0 mg (0.029 mmol) of the compound 64 prepared in Example 63, 3.3 mg (0.047 mmol) of hydroxylamine hydrochloride and 0.075 mL (0.075 mmol) of a 1 mol/L triethylamine solution in tetrahydrofuran in a similar manner to that in Example 50.
$^1$H-NMR(270MHz, DMSO-$d_6$, δ); 1.92(m, 2H), 2.12(s, 6H), 2.14(t, J=6.9Hz, 2H), 4.53(t, J=6.3Hz, 2H), 7.37(t, J=7.4Hz, 1H), 7.65(m, 1H), 7.75(d, J=8.3Hz, 1H), 8.28(s, 1H), 8.89(d, J=7.4Hz, 1H), 9.13(s, 1H), 11.28(brs, 1H), 11.72(s, 1H).
Fab-MS(m/z); 365[M+1]$^+$

Example 65: Compound 66

Step 1

6-(2-Dimethylaminopropyl)-1,3-dioxo-4-formyl-1,2,3,6-tetrahydro-2-triphenylmethylpyrrolo[3,4-c]carbazole (313.2 mg, 66%) and 6-(2-dimethylamino-1-methylethyl)-1,3-dioxo-4-formyl-1,2,3,6-tetrahydro-2-triphenylmethylpyrrolo[3,4-c]carbazole (64.9 mg, 14%) were prepared in a similar manner to that in the step 3 of Example 63 from 405.8 mg (0.801 mmol) of 1,3-dioxo-4-formyl-1,2,3,6-tetrahydro-2-triphenylmethylpyr rolo [3,4-c]carbazole prepared in the step 2 of Example 63, 379.0 mg (2.40 mmol) of 2-dimethylaminopropyl chloride hydrochloride and 9.6 mL (4.8 mmol) of a 0.5 mol/L solution of potassium tert-butoxide in 2-methyl-2-propanol.
6-(2-Dimethylaminopropyl)-1,3-dioxo-4-formyl-1,2,3,6-tetra hydro-2-triphenylmethylpyrrolo[3,4-c]carbazole
$^1$H-NMR(270MHz, DMSO-$d_6$, δ); 0.91(d, J=6.6Hz, 3H), 2.14(s, 6H), 3.08(m, 1H), 4.45(dd, J=6.1Hz, 14.9Hz, 1H), 4.67 (dd, J=8.1Hz, 14.9Hz, 1H), 7.19(t, J=7.5Hz, 3H), 7.30(t, J=7.5Hz, 6H), 7.36(m, 1H), 7.58(d, J=7.5Hz, 6H), 7.69(dd, J=7.3Hz, 8.2Hz, 1H), 7.81(d, J=8.2Hz, 1H), 8.37(s, 1H), 8.79(d, J=7.6Hz, 1H), 10.88(s, 1H). Fab-MS(m/z); 592[M+1]$^+$
6-(2-Dimethylamino-1-methylethyl)-1,3-dioxo-4-formyl-1,2,3,6-tetrahydro-2-triphenylmethylpyrrolo[3,4-c]carbazole
$^1$H-NMR(270MHz, DMSO-$d_6$, δ); 1.62(d, J=6.6Hz, 3H), 2.01(s, 6H), 2.65(dd, J=5.3Hz, 13.2Hz, 1H), 3.21(m, 1H), 5.34 (m, 1H), 7.19(t, J=7.5Hz, 3H), 7.30(t, J=7.5Hz, 6H), 7.36(dd, J=7.6Hz, 8.2Hz, 1H), 7.58 (d, J=7.5Hz, 6H), 7.66 (dd, J=7.6Hz, 8.2Hz, 1H), 7.94 (d, J=8.2Hz, 1H), 8.41(brs, 1H), 8.86(d, J=8.2Hz, 1H), 10.88(s, 1H).
Fab-MS(m/z); 592[M+1]$^+$

Step 2

A trifluoroacetate (161.1 mg, 89%) of the compound 66 was prepared in a similar manner to that in the step 4 of Example 63 from 307.4 mg (0.520 mmol) of 6-(2-dimethylaminopropyl)-1,3-dioxo-4-formyl-1,2,3,6-tetra hydro-2-triphenylmethylpyrrolo[3,4-c]carbazole prepared in the step 1 of Example 65 and 2 mL of trifluoroacetic acid.
$^1$H-NMR(270MHz, DMSO-$d_6$, δ); 1.10(d, J=6.3Hz, 3H), 2.89(m, 6H), 3.90(brm, 1H), 4.87(brm, 2H), 7.48(m, 1H), 7.78 (dd, J=7.3Hz, 8.3Hz, 1H), 7.94(d, J=8.3Hz, 1H), 8.53(brs, 1H), 9.01(d, J=8.3Hz, 1H), 10.00(brs, 1H), 11.06(s, 1H), 11.58(brs, 1H).
Fab-MS(m/z); 350[M+1]$^+$

Example 66: Compound 67

A trifluoroacetate (7.1 mg, 14%) of the compound 67 was prepared in a similar manner to that in the step 4 of Example 63 from 64.9 mg (0.110 mmol) of 6-(2-dimethylamino-1-methylethyl)-1,3-dioxo-4-formyl-1,2,3,6-tetrahydro-2-triphenylmethylpyrrolo[3,4-c]carbazole prepared in the step 1 of Example 65 and 1 mL of trifluoroacetic acid.
$^1$H-NMR(270MHz, DMSO-$d_6$, δ); 1.63(d, J=6.9Hz, 3H), 2.04(s, 6H), 2.67(dd, J=5.3Hz, 12.9Hz, 1H), 3.21(dd, J=9.9Hz, 12.9Hz, 1H), 5.34(m, 1H), 7.41(dd, J=7.3Hz, 7.9Hz, 1H), 7.68(ddd, J=1.0Hz, 7.3Hz, 8.4Hz, 1H), 7.94(d, J=8.4Hz, 1H), 8.39(s, 1H), 9.06(d, J=7.9Hz, 1H), 11.03(s, 1H), 11.51(br, 1H).

Example 67: Compound 68

The compound 68 (11.2 mg, 80%) was prepared from 13.5 mg (0.039 mmol) of the compound 66 prepared in Example 65, 3.5 mg (0.050 mmol) of hydroxylamine hydrochloride and 0.077 mL (0.077 mmol) of a 1 mol/L triethylamine solution in tetrahydrofuran in a similar manner to that in Example 50.

$^1$H-NMR(270MHz, DMSO-$d_6$, δ); 0.92(d, J=6.6Hz, 3H), 2.21(s, 6H), 3.12(m, 1H), 4.36(dd, J=6.6Hz, 14.9Hz, 1H), 4.57 (dd, J=7.3Hz, 14.9Hz, 1H), 7.36(dd, J=7.3Hz, 7.9Hz, 1H), 7.64(dd, J=7.3Hz, 8.4Hz, 1H), 7.74(d, J=8.4Hz, 1H), 8.21 (s, 1H), 8.90(d, J=7.9Hz, 1H), 9.13(s, 1H), 11.26(brs, 1H), 11.75(s, 1H).
Fab-MS(m/z); 365$[M+1]^+$

Example 68: Compound 69

The compound 69 (7.7 mg, 72%) was prepared from 10.3 mg (0.029 mmol) of the compound 66 prepared in Example 65 and 0.002 mL of hydrazine hydrate in a similar manner to that in Example 50.
$^1$H-NMR(270MHz, DMSO-$d_6$, δ); 0.91(d, J=6.6Hz, 3H), 2.22(s, 6H), 3.12(m, 1H), 4.31(dd, J=7.3Hz, 14.8Hz, 1H), 4.51 (dd, J=6.8Hz, 14.8Hz, 1H), 7.32(dd, J=6.9Hz, 7.9Hz, 1H), 7.48(s, 2H), 7.59(m, 1H), 7.68(d, J=8.3Hz, 1H), 8.16(s, 1H), 8.79(s, 1H), 8.87(d, J=7.9Hz, 1H), 11.14(brs, 1H).
Fab-MS(m/z); 364$[M+1]^+$

Example 69: Compound 70

The compound 70 (3.8 mg, 71%) was prepared from 5.1 mg (0.015 mmol) of the compound 67 prepared in Example 66, 15.3 mg (0.22 mmol) of hydroxylamine hydrochloride and 0.061 mL (0.44 mmol) of triethylamine in a similar manner to that in Example 50.
$^1$H-NMR(270MHz, DMSO-$d_6$, δ); 1.62(d, J=6.9Hz, 3H), 2.06(s, 6H), 2.69(dd, J=5.1Hz, 12.5Hz, 1H), 3.15(dd, J=9.6Hz, 12.5Hz, 1H), 5.19(m, 1H), 7.35(dd, J=7.3Hz, 7.8Hz, 1H), 7.61(dd, J=7.3Hz, 8.3Hz, 1H), 7.86(d, J=8.3Hz, 1H), 8.28(s, 1H), 8.98(d, J=7.8Hz, 1H), 9.13(s, 1H), 11.71(br, 1H), 11.74(brs, 1H).

Example 70: Compound 71

2-Formylindole (6.60 g, 45.5 mmol) was dissolved in a mixed solvent of 40 mL of DMF and 80 mL of toluene, then 3.0 g (75 mmol) of 60% sodium hydride was added with ice cooling, the mixture was stirred for 45 minutes, 14.4 mL (75.1 mmol) of benzyl 2-bromoacetate was dropped thereinto with ice cooling and the mixture was stirred at room temperature for 30 minutes. Water was added to the reaction solution, the mixture was extracted with chloroform, the organic layer was washed with a saturated saline solution, dried over anhydrous sodium sulf ate and the solvent was evaporated. The residue was purified by a silica gel column chromatography (chloroform/methanol = 9/1). After that, the resulting compound was dissolved in 100 mL of toluene, 23.5 g (67.5 mmol) of ethyl triphenylphosphoranylideneacetate was added and the mixture was heated to reflux for 1 hour. The solvent was evaporated from the reaction solution, 300 mL of ethyl acetate was added, insoluble matters were filtered off and the filtrate was vacuum concentrated. The residue was purified by a silica gel column chromatography (hexane/ethyl acetate = 9/1). After that, the above-prepared compound was reated with 8.73 g (90 mmol) of N-triphenylmethylmaleimide in a similar manner to that in the step 2 of Example 1 and then the reaction product was treated with 20.4 g (90 mmol) of DDQ in a similar manner to that in Example 6 to give 6.02 g (30%) of the compound 71.
Fab-MS(m/z); 457$[M+1]^+$

Example 71: Compound 72

The compound 72 (235 mg, 70%) was prepared from 456 mg (1 mmol) of the compound 71 prepared in Example 70 in a similar manner to that in Example 27.
$^1$H-NMR(270MHz, DMSO-$d_6$, δ); 5.47(s, 2H), 7.39(dd, J=7.1Hz, 7.8Hz, 1H), 7.67(dd, J=7.1Hz, 8.4Hz, 1H), 7.74(d, J=8.4Hz, 1H), 8.22(s, 1H), 8.93(d, J=7.8Hz, 1H), 11.49(s, 1H).
Fab-MS(m/z); 339$[M+1]^+$

Example 72: Compound 73

The compound 40 (2.09 g, 6 mmol) prepared in Example 39 was dissolved in 30 mL of tetrahydrofuran, then 3 mL of pyridine, 1.0 mL of a 2.5% by weight solution of osmium tetraoxide in 2-methyl-2-propanol and 3.51 g (30 mmol) of N-methylmorpholine N-oxide were added thereto and the mixture was stirred at room temperature for 12 hours in an argon atmosphere shielding from the light. An aqueous solution of sodium sulfite was added to the reaction solution, the mixture was extracted with a mixed solvent of chloroform and methanol, the organic layer was washed with a saturated aqueous saline solution, dried over anhydrous sodium sulfate and the solvent was evaporated. The residue was subjected to trituration with methanol to give 1.33 g (87%) of the compound 73.
Fab-MS(m/z); 383$[M+1]^+$

Example 73: Compound 74

The compound 74 (1.01 g, 84%) was prepared from 1.3 g (1 mmol) of the compound 73 prepared in Example 72 in a similar manner to that in Example 27.
Fab-MS(m/z); 355[M+1]$^+$

Example 74: Compound 75

The compound 74 (1.01 g, 2.85 mmol) prepared in Example 73 was dissolved in 20 mL of DMF, then 0.62 mL (5.05 mmol) of 2,2-dimethoxypropane and 108 mg (0.57 mmol) of p-toluenesulfonic acid were added thereto and the mixture was stirred at room temperature for 5 hours. Water was added to the reaction solution and the crystals separated out therefrom were filtered to give 1.21 g (quantitatively) of the compound 75.
$^1$H-NMR(270MHz, DMSO-$d_6$, δ); 1.16(s, 3H), 1.24(s, 3H), 3.72-3.75(m, 1H), 4.13-4.18(m, 1H), 4.51-4.78(m, 3H), 7.40 (dd, J=7.3Hz, 7.9Hz, 1H), 7.67(dd, J=7.3Hz, 8.2Hz, 1H), 7.85(d, J=8.2Hz, 1H), 7.95(s, 1H), 8.31(s, 1H), 8.93(d, J=7.9Hz, 1H), 11.48(brs, 1H).
Fab-MS(m/z); 395[M+1]$^+$

Example 75: Compound 76

The compound 75 (80 mg, 0.2 mmol) prepared in Example 74 was suspended in 2 mL of dichloromethane, then 0.05 mL of thiomorpholine, 80 mg of water-soluble carbodiimide and 0.05 mL of triethylamine were added thereto and the mixture was stirred at 50°c for 2 hours. After cooling the mixture to room temperature, the solvent was evaporated and the residue was subjected to trituration with water to give 97.8 mg (quantitatively) of the compound 76.
Fab-MS(m/z); 480[M+1]$^+$

Example 76: Compound 77

The compound 77 (20.6 mg, 71%) was prepared from 30 mg (0.07 mmol) of the compound 26 prepared in Example 25 and 60 mg (0.26 mmol) of DDQ in a similar manner to that in Example 6.
$^1$H-NMR(270MHz, DMSO-$d_6$, δ); 3.48(d, J=5.0Hz, 2H), 3.73-3.79(m, 2H), 3.83-3.88(m, 2H), 5.16(m, 1H), 5.75(s, 2H), 7.18(d, J=7.6Hz, 2H), 7.22-7.30(m, 3H), 7.35(dd, J=7.3Hz, 7.9Hz, 1H), 7.56(dd, J=7.3Hz, 8.3Hz, 1H), 7.73(d, J=8.3Hz, 1H), 7.93(s, 1H), 8.89(d, J=7.9Hz, 1H), 11.17(s, 1H).
Fab-MS(m/z); 413[M+1]$^+$

Example 77: Compound 78

1-Benzyl-2-[2-(1,3-dioxolan-2-yl)vinyl]indole (40 mg, 0.13 mmol) was reacted with 26 mg (0.26 mmol) of maleimide in a similar manner to that in the step 2 of Example 1 and then the reaction product was treated with 60 mg (0.26 mmol) of DDQ in a similar manner to that in Example 6 to give 44.1 mg (85%) of the compound 78.
Fab-MS(m/z); 399[M+1]$^+$

Example 78: Compound 79

Step 1

2-(2-Hydroxymethylvinyl)indole (2.66 g, 15.4 mmol) was made to react with 6.25 g (29.6 mmol) of N-(tert-butyldimethylsilyl)maleimide in a similar manner to that in the step 2 of Example 1 to give 1.07 g (18%) of 2-(tert-butyldimethylsilyl)-1,3-dioxo-4-hydroxymethyl-1,2, 3,3a,4,5,6,10c-octahydropyrrolo[3,4-c]carbazole.
$^1$H-NMR(270MHz, $CDCl_3$, δ); 0.30 and 0.38(2s, total 6H), 0.70(s, 9H), 2.32(m, 1H), 2.71(dd, J=4.6Hz, 16.2Hz, 1H), 2.92(m, 1H), 3.52(dd, J=4.0Hz, 7.6Hz, 1H), 3.99-4.27(m,3H), 4.30(d, J=7.6Hz, 1H), 7.09-7.19(m, 2H), 7.28(m, 1H), 7.89(d, J=8.2Hz, 1H), 7.96(brs, 1H).

Step 2

2-(tert-Butyldimethylsilyl)-1,3-dioxo-4-hydroxy-meth yl-1,2,3,3a,4,5,6,10c-octahydropyrrolo[3,4-c]carbazole (732.9 mg, 1.776 mmol) prepared in the step 1 of Example 78 was dissolved in 100 mL of dichloromethane, then 0.245 mL (2.69 mmol) of 3,4-dihydro-2H-pyran and 47.3 mg (0.188 mmol) of pyridinium p-toluenesulfonate were added thereto and the mixture was stirred at room temperature through one night. Water was added to the reaction solution and

the mixture was extracted with chloroform. The organic layer was washed with a saturated aqueous saline solution, dried over anhydrous sodium sulfate and the solvent was evaporated. The residue was purified by a silica gel column chromatography (hexane/ethyl acetate = 2/1) to give 511.6 mg (61%) of 2-(tert-butyldimethylsilyl)-1,3-dioxo-1,2,3,3a,4,5,6,10c-octahydro-4-(tetrahydropyran-2-yloxymethyl)pyrrolo[3,4-c]carbazole.
$^1$H-NMR(270MHz, $CDCl_3$, δ); 0.30 and 0.37(2s, total 6H), 0.68 and 0.69(2s, total 9H), 1.44-1.91(brm, 6H), 2.42(m, 1H), 2.69(m, 1H), 2.89(m, 1H), 3.54(m, 3H), 3.89(m, 1H), 3.93(m, 1H), 4.24(d, J=7.6Hz, 1H), 4.67(m, 1H), 7.14(m, 2H), 7.29(m, 1H), 7.90(brm, 2H).
Fab-MS(m/z); 468[M]$^+$

Step 3

2-(tert-Butyldimethylsilyl)-1,3-dioxo-1,2,3,6-tetrah ydro-4-(tetrahydropyran-2-yloxymethyl)pyrrolo[3,4-c]carbaz ole (389.4 mg, 92%) was prepared in a similar manner to that in Example 6 from 453.4 mg (0.913 mmol) of 2-(tert-butyldimethylsilyl)-1,3-dioxo-1,2,3,3a,4,5,6,10c-o ctahydro-4-(tetrahydropyran-2-yloxymethyl)pyrrolo[3,4-c]ca rbazole prepared in the step 2 of Example 78 and 627.0 mg (2.762 mmol) of DDQ.
$^1$H-NMR(270MHz, $CDCl_3$, δ); 0.58(s, 6H), 1.03(s, 9H), 1.60-1.98(m, 6H), 3.60(m, 1H), 3.99(m, 1H), 4.87(m, 1H), 5.25 (dd, J=1.0Hz, 14.8Hz, 1H), 5.40(dd, J=1.0Hz, 14.8Hz, 1H), 7.34(ddd, J=1.3Hz, 6.,6Hz, 7.9Hz, 1H), 7.48(dd, J=6.8Hz, 8.1Hz, 1H), 7.55(dd, J=1.3Hz, 8.1Hz, 1H), 7.91(s, 1H), 8.65(brs, 1H), 9.01(d, J=7.9Hz, 1H).

Step 4

2-(tert-Butyldimethylsilyl)-1,3-dioxo-1,2,3,6-tetrah ydro-4-(tetrahydropyran-2-yloxymethyl)pyrrolo[3,4-c]carbaz ole (20.2 mg, 0.043 mmol) prepared in the step 3 of Example 78 was dissolved in 1 ml of DMF, then 12.0 mg (0.052 mmol) of methyl 4-bromomethylbenzoate and 19.9 mg (0.144 mmol) of potassium carbonate were added thereto and the mixture was stirred at room temperature for 4 hours. Ice water was added to the reaction solution and the resulting precipitate was filtered and purified by a silica gel column chromatography (chloroform/acetonitrile = 9/1) to give 9.2 mg (43%) of the compound 79.
$^1$H-NMR(270MHz, DMSO-$d_6$, δ); 1.52-1.86(m, 6H), 3.49(m, 1H), 3.828(s, 3H), 3.829(m, 1H), 4.85(m, 1H), 4.89(s, 2H), 5.08(d, J=14.3Hz, 1H), 5.24(d, J=14.3Hz, 1H), 7.31(ddd, J=1.3Hz, 6.6Hz, 7.9Hz, 1H), 7.51(d, J=8.6Hz, 2H), 7.56(ddd, J=1.3Hz, 6.6Hz, 7.9Hz, 1H), 7.62(brd, J=7.9Hz, 1H), 7.92(s, 1H), 7.94(d, J=8.6Hz, 2H), 8.80(brd, J=7.9Hz, 1H), 12.05 (brs, 1H).
Fab-MS(m/z); 499[M+1]$^+$

Example 79: Compound 80

The compound 80 (10.8 mg, 51%) was prepared in a similar manner to that in the step 4 of Example 78 from 20.0 mg (0.043 mmol) of 2-(tert-butyldimethylsilyl)-1,3-dioxo-1,2,3,6-tetrahydro-4-(tetrahydropyran-2-yloxymethyl)pyrrolo[3,4-c]carbazole prepared from the step 3 of Example 78, 10.8 mg (0.049 mmol) of 2-bromomethylnaphthalene and 23.9 mg (0.173 mmol) of potassium carbonate.
$^1$H-NMR(270MHz, DMSO-$d_6$, δ); 1.53-1.79(m, 6H), 3.51(m, 1H), 3.84(m, 1H), 4.85(brs, 1H), 4.99(s, 2H), 5.09(d, J=14.5Hz, 1H), 5.26(d, J=14.5Hz, 1H), 7.30(m, 1H), 7.47-7.63(m, 5H), 7.88-7.95(m, 5H), 8.83(d, J=7.9Hz, 1H), 12.07 (brs, 1H).
Fab-MS(m/z); 491[M+1]$^+$

Example 80: Compound 81

The compound 81 (17.6 mg, 42%) was prepared in a similar manner to that in the step 4 of Example 78 from 40.2 mg (0.087 mmol) of 2-(tert-butyldimethylsilyl)-1,3-dioxo-1,2,3,6-tetrahydro-4-(tetrahydropyran-2-yloxymethyl)pyrrolo[3,4-c]carbazole prepared from the step 3 of Example 78, 17.8 mg (0.082 mmol) of 2-nitrobenzyl bromide and 11.4 mg (0.082 mmol) of potassium carbonate.
$^1$H-NMR(270MHz, DMSO-$d_6$, δ); 1.50-2.00(m, 6H), 3.64(m, 1H), 4.04(m, 1H), 4.90(m, 1H), 5.19(dd, J=1.0Hz, 14.2Hz, 1H), 5.25(s, 2H), 5.37(dd, J=1.0Hz, 14.2Hz, 1H), 7.27-7.59(m, 6H), 7.83(s, 1H), 8.11(dd, J=1.5Hz, 8.1Hz, 1H), 8.81 (brs, 1H), 8.89(d, J=7.9Hz, 1H).
Fab-MS(m/z); 486[M+1]$^+$

Example 81: Compound 82

The compound 81 (5.1 mg, 0.011 mmol) prepared in Example 80 was dissolved in a mixed solvent of 1 mL

of ethanol and 0.6 mL of tetrahydrofuran, then 3.0 mg (0.012 mmol) of pyridinium p-toluenesulfonate were added thereto and the mixture was stirred at 50°C for 3 hours. After the reaction solution was concentrated, the residue was purified by a thin-layer chromatography (chloroform/methanol = 9/1) to give 5.1 mg (quantitatively) of the compound 82.
$^1$H-NMR(270MHz, DMSO-$d_6$, δ); 5.09(d, J=5.2Hz, 2H), 5.14(s, 2H), 5.56(t, J=5.2Hz, 1H), 7.30(ddd, J=1.2Hz, 6.8Hz, 7.9Hz, 1H), 7.69(dt, J=1.3Hz, 7.6Hz, 1H), 7.48-7.63(m, 4H), 7.98(s, 1H), 8.12(dd, J=1.3Hz, 8.2Hz, 1H), 8.77(d, J=7.9Hz, 1H), 12.09(brs, 1H).
Fab-MS(m/z); 402[M+1]$^+$

Example 82: Compound 83

The compound 40 (10.0 g, 28.7 mmol) prepared in Example 39 reacted with 14.0 g (57.5 mmol) of a 9-borabicyclononane dimer in a similar manner to that in Example 56 and the reaction product was treated with an aqueous solution of sodium hydroxide and an aqueous solution of hydrogen peroxide to give 8.56 g (82%) of the compound 83.
Fab-MS(m/z); 367[M+1]$^+$

Example 83: Compound 84

The compound 84 (1.47 g, 79%) was prepared in a similar manner to that in Example 47 from 1.74 g (4.75 mmol) of the compound 83 prepared in Example 82, 1.1 mL (7.1 mmol) of DBU and 2.6 g (7.1 mmol) of bis(p-nitrophenyl) phosphoric azide.
Fab-MS(m/z); 392[M+1]$^+$

Example 84: Compound 85

The compound 84 (1.47 g, 3.76 mmol) prepared in Example 83 was dissolved in a mixed solvent of tetrahydrofuran and ethanol and subjected to a catalytic reduction in the presence of 700 mg of 10% palladium carbon in a similar manner to that in Example 61 to give 1.34 g (98%) of the compound 85.
Fab-MS(m/z); 366[M+1]$^+$

Example 85: Compound 86

The compound 71 (1.94 g, 4.25 mmol) prepared in Example 7 0 was dissolved in DMF and stirred for 5 hours at roomtemperature in a hydrogen atmosphere in the presence of 210 mg of 10% palladium carbon. The reaction solution was filtered through Celite, the filtrate was vacuum concentrated and the residue was subjected to trituration with methanol to give 1.69 g (quantitatively) of the compound 86.
Fab-MS(m/z); 367[M+1]$^+$

Example 86: Compound 87

The compound 86 (1.43 g, 3.91 mmol) prepared in Example 85 was dissolved in 100 mL of tetrahydrofuran, then 1.11 mL (11.7 mmol) of a boran-dimethyl sulfide complex were dropped thereinto with ice cooling and the mixture was stirred at room temperature for 2 days. Ice water was added to the reaction solution, the mixture was extracted with a mixed solvent of tetrahydrofuran and ethyl acetate, the organic layer was washed with a saturated aqueous saline solutio dried over anhydrous sodium sulfate and the solvent was evaporated. The residue was purified by a silica gel column chromatography (chloroform/methanol = 40/1) to give 735 mg (53%) of the compound 87.
Fab-MS(m/z); 353[M+1]$^+$

Example 87: Compound 88

The compound 88 (176 mg, 83%) was prepared in a similar manner to that in Example 47 from 199 mg (0.565 mmol) of the compound 87 prepared in Example 86, 0.13 mL (0.85 mmol) of DBU and 310 mg (0.849 mmol) of bis(p-nitrophenyl)phosphoric azide.
Fab-MS(m/z); 378[M+1]$^+$

Example 88: Compound 89

The compound 88 (170 mg, 0.452 mmol) prepared in Example 87 was dissolved in a mixed solvent of tetrahydrofuran and ethanol and subjected to a catalytic reduction in the presence of 106 mg of 10% palladium-carbon

in a similar manner to that in Example 61 to give 67 mg (42%) of the compound 89.
Fab-MS(m/z); 352[M+1]$^+$

Example 89: Compound 90

Step 1

Magnesium (317.2 mg, 13.05 mmol) was suspended in 5 mL of ether, a solution of 2.30 mL (13.0 mmol) of 3-benzyloxypropyl bromide in 7 mL of ether was dropped into the suspension over 10 minutes, a flake of iodine was added thereto and the mixture was heated to reflux for 1 hour. The reaction mixture was cooled with an ice-methanol bath, a solution of 5.1 mg (0.011 mmol) of N-methoxy-N-methyl-(1-methylindole)-2-carboxamide in tetrahydrofuran was added thereto and the mixture was stirred at 0°C for 2 hours. Ice water was added to the reaction solution and the mixture was acidified with 6 mol/L hydrochloric acid and extracted with ether. The organic layer was washed with water and a saturated aqueous saline solutio dried over anhydrous sodium sulfate and the solvent was evaporated. The residue was purified with a silica gel column chromatography (hexane/ethyl acetate = 8/1) to give 0.79 g (54%) of 2-(4-benzyloxy-1-oxobutyl)-1-methylindole.
$^1$H-NMR(270MHz, DMSO-$d_6$, δ); 2.09(m, 2H), 3.10(t, J=7.3Hz, 2H), 3.58(t, J=6.1Hz, 2H), 4.05(s, 3H), 4.50(s, 2H), 7.15(m, 1H), 7.26-7.39(m, 8H), 7.69(dd, J=1.0Hz, 8.3Hz, 1H).
Fab-MS(m/z); 308[M+1]$^+$

Step 2

Ethyl diethylphosphonoacetate (5.14 mL, 25.7 mmol) was dissolved in 100 mL of tetrahydrofuran, 1.0 g (25 mmol) of 60% sodium hydride was added thereto, the mixture was stirred at room temperature for 20 minutes, then 0.79 g (2.6 mmol) of 2-(4-benzyloxy-1-oxobutyl)-1-methylindole prepared in the step 1 of Example 89 was added thereto and the mixture was heated under reflux for 8 hours. The reaction solution was cooled to room temperature, water was added thereto and the mixture was extracted with ether. The organic layer was washed with water and a saturated aqueous saline solutio dried over anhydrous sodium sulfate and the solvent was evaporated. The residue was purified by a silica gel column chromatography (hexane/ethyl sulfate = 8/1). After that, the above-prepared product was reacted with 314.3 mg (3.238 mmol) of maleimide in a similar manner to that in the step 2 of Example 1 and then the reaction product was treated with 561.7 mg (2.474 mmol) of DDQ in a similar manner to that in Example 6 to give 258.7 mg (22%) of the compound 90.
$^1$H-NMR(270MHz, DMSO-$d_6$, δ); 1.42(t, J=7.3Hz, 3H), 2.08(m, 2H), 3.35(m, 2H), 3.63(t, J=5.6Hz, 2H), 4.18(s, 3H), 4.53(q, J=7.3Hz, 2H), 4.56(s, 2H), 7.31-7.49(m, 7H), 7.53(brs, 1H), 7.65(m, 1H), 9.12(d, J=7.9Hz, 1H).
Fab-MS(m/z); 471[M+1]$^+$

Example 90: Compound 91

The compound 90 (108.3 mg, 0.230 mmol) prepared in Example 89 was dissolved in a mixed solvent of ethyl acetate and ethanol and subjected to a catalytic reduction in the presence of 52.9 mg of 10% palladium-carbon to give 60.4 mg (69%) of the compound 91.
$^1$H-NMR(270MHz, DMSO-$d_6$, δ); 1.35(t, J=7.1Hz, 3H), 1.79-1.86(m, 2H), 3.16-3.24(m, 2H), 3.53-3.59(m, 2H), 4.21(s, 3H), 4.43(q, J=7.1Hz, 2H), 4.75(t, J=5.1Hz, 1H), 7.38(t, J=7.3Hz, 1H), 7.67(ddd, J=1.3Hz, 7.3Hz, 8.6Hz, 1H), 7.81(d, J=8.6Hz, 1H), 8.99(brd, J=7.3Hz, 1H).
Fab-MS(m/z); 381[M+1]$^+$

Example 91: Compound 92

The compound 92 (186.6 mg, 94%) was prepared in a similar manner to that in Example 35 from 0.17 g (0.45 mmol) of the compound 91 prepared in Example 90, 357.03 mg (1.361 mmol) of triphenylphosphine and 0.063 mL (1.2 mmol) of bromine.
$^1$H-NMR(270MHz, $CDCl_3$, δ); 1.46(t, J=7.3Hz, 3H), 2.34(m, 2H), 3.39(m, 2H), 3.58(t, J=5.9Hz, 2H), 4.22(s, 3H), 4.57 (q, J=7.3Hz, 2H), 7.39-7.70(m, 4H), 9.13(d, J=7.9Hz, 1H).
Fab-MS(m/z); 443[M+1]$^+$

Example 92: Compound 93

The compound 92 (148.1 mg, 0.334 mmol) prepared in Example 91 was suspended in 1 mL of 1,2-dichlorobenzene, then 0.055 mL (0.50 mmol) of benzylamine was added thereto and the mixture was heated under reflux for 30 minutes. After cooling to room temperature, dichloromethane and 1 mol/L aqueous solution of sodium hydroxide were added thereto followed by extracting. The organic layer was washed with a saturated aqueous saline solutio dried over anhydrous sodium sulfate and the solvent was evaporated. The residue was purified by a thin-layer chromatography (chloroform/methanol = 7/1) to give 96.8 mg (62%) of the compound 93.
Fab-MS(m/z); 470$[M+1]^+$

Example 93: Compound 94 and compound 95

The compound 40 (20 mg, 0.056 mmol) prepared in Example 39 was dissolved in 0.5 mL of tetrahydrofuran, then 2 mg (0.06 mmol) of sodium borohydride was added thereto at -78°C and the mixture was stirred at the same temperature for 1.5 hours. Further, 21 mg (0.56 mmol) of sodium borohydride was added thereto, the mixture was stirred for one hour by raising up to -18°C, then 20 mg (0.53 mmol) of sodium borohydride was added and the mixture was stirred for 15.5 hours at room temperature. Water and methanol were added to the reaction solution, the mixture was extracted with chloroform, the organic layer was washed with a saturated aqueous saline solutio dried over anhydrous sodium sulfate and the solvent was evaporated. The residue was purified by a preparative thin-layer chromatography (chloroform/methanol = 9/1) to give 8.7 mg (44%) of the compound 94 and 1.0 mg (5%) of the compound 95.

Compound 94

$^1$H-NMR(270MHz, DMSO-$d_6$, δ); 1.38(t, J=7.3Hz, 3H), 4.38(q, J=7.3Hz, 2H), 4.81(d, J=17.1Hz, 1H), 5.10(d, J=9.2Hz, 1H), 5.21(d, J=5.6Hz, 2H), 6.02 (m, 1H), 6.12 (d, J=8.6Hz, 1H), 6.33(d, J=8.6Hz, 1H), 7.30(dd, J=7.6Hz, 7.6Hz, 1H), 7.57(dd, J=7.2Hz, 7.2Hz, 1H), 7.67(d, J=8.2Hz, 1H), 8.21(s, 1H), 9.06(s, 1H), 9.16(d, J=7.6Hz, 1H).
APCI-MS(m/z); 351$[M+1]^+$

Compound 95

$^1$H-NMR(270MHz, DMSO-$d_6$, δ); 1.32(t, J=7.2Hz, 3H), 4.33(q, J=7.2Hz, 2H), 4.87(d, J=18.8Hz, 1H), 5.10(d, J=11.5Hz, 1H), 5.17(d, J=5.0Hz, 2H), 6.00(m, 1H), 6.32(d, J=11.2Hz, 1H), 6.57(d, J=9.9Hz, 1H), 7.32(dd, J=7.5Hz, 7.5Hz, 1H), 7.56(dd, J=7.8Hz, 7.8Hz, 1H), 7.68(d, J=8.3Hz, 1H), 7.79(s, 1H), 8.34(d, J=7.6Hz, 1H), 8.81(s, 1H).
APCI-MS(m/z); 351$[M+1]^+$

Example 94: Compound 96, compound 97 and compound 98

The compound 85 (101 mg, 0.277 mmol) prepared in Example 84 was dissolved in a mixed solvent of 2.5 mL of tetrahydrofuran and 2.5 mL of methanol, then 54 mg (1.4 mmol) of sodium borohydride was added thereto at -18°C and the mixture was stirred at -18°C for 50 minutes. Sodium borohydride (55 mg, 1.5 mmol) was further added to the reaction solution and the mixture was stirred for 2 hours. Water and 2 mol/L hydrochloride acid were added to the reaction solution, the mixture was stirred for 1 hour with ice cooling, a saturated aqueous solution of sodium hydrogen carbonate was added thereto and the mixture was extracted with a mixed solvent of chloroform and methanol. The organic layer was washed with a saturated aqueous saline solution, dried over anhydrous sodium sulfate and the solvent was evaporated. The residue was purified by a preparative thin-layer chromatography [ammonia-containing chloroform (a chloroform layer after mixing chloroform with 28% aqueous ammonia in a ratio of 6/1)/methanol = 6/1] to give 29 mg (28%) of the compound 96, 10 mg (10%) of the compound 97 and 15 mg (14%) of the compound 98.

Compound 96

$^1$H-NMR(270MHz, DMSO-$d_6$, δ); 1.48(t, J=7.0Hz, 3H), 2.04(m, 2H), 2.76(t, J=6.7Hz, 2H), 3.20(s, 3H), 4.50 (m, 2H), 4.55(t, J=6.9Hz, 2H), 6.50(m, 2H), 7.35(dd, J=7.4Hz, 7.4Hz, 1H), 7.52(d, J=8.2Hz, 1H), 7.60(dd, J=6.9Hz, 6.9Hz, 1H), 8.32(s, 1H), 9.28(d, J=7.9Hz, 1H).
ESI-MS(m/z); 382$[M+1]^+$

Compound 97

$^1$H-NMR(270MHz, DMSO-$d_6$, δ); 1.40(t, J=7.3Hz, 3H), 1.88(m, 2H), 2.57(t, J=6.9Hz, 2H), 4.40(q, J=7.3Hz, 2H), 4.60(t, J=6.8Hz, 2H), 6.12(br, 1H), 6.35(s, 1H), 7.30(dd, J=7.3Hz, 7.3Hz, 1H), 7.60(dd, J=7.3Hz, 7.9Hz, 1H), 7.76 (d, J=8.6Hz, 1H), 8.31(s, 1H), 9.06(s, 1H), 9.17(d, J=7.9Hz, 1H).
ESI-MS(m/z); 368$[M+1]^+$

Compound 98

$^1$H-NMR(270MHz, DMSO-$d_6$, δ); 1.34(t, J=7.3Hz, 3H), 2.01(m, 2H), 2.79(t, J=7.4Hz, 2H), 4.35(q, J=7.3Hz, 2H), 4.60(t, J=6.9Hz, 2H), 6.34(d, J=9.9Hz, 1H), 6.60(d, J=9.9Hz, 1H), 6.89(br, 2H), 7.34(dd, J=7.3Hz, 7.3Hz, 1H), 7.60(dd, J=7.3Hz, 7.3Hz, 1H), 7.80(d, J=8.2Hz, 1H), 7.91(s, 1H), 8.35(d, J=7.6Hz, 1H), 8.83(s, 1H).
ESI-MS(m/z); 368$[M+1]^+$

Example 95: Compound 99

1-Allyl-2-(2-acetylvinyl)indole (1.61 g, 7.16 mmol) was reacted with 2.08 g (21.5 mmol) of maleimide in a similar manner to that in the step 2 of Example 1 and then the reaction product was treated with 2.50 g (11.0 mmol) of DDQ at room temperature in a similar manner to that in Example 6 to give 1.25 g (71%) of the compound 99.
$^1$H-NMR(270MHz, $CDCl_3$-$CD_3OD$, δ); 2.86(s, 3H), 5.01(m, 3H), 5.23(dd, J=0.7Hz, 10.2Hz, 1H), 6.00(m, 1H), 7.41(ddd, J=0.9Hz, 7.2Hz, 8.1Hz, 1H), 7.48(d, J=8.4Hz, 1H), 7.65(m, 1H), 7.67(s, 1H), 9.06(ddd, J=0.9Hz, 0.9Hz, 8.1Hz, 1H), 9.93(br, 1H).
Fab-MS(m/z); 319$[M+1]^+$

Example 96: Compound 100

The compound 99 (1.15 g, 3.60 mmol) prepared in Example 95 was reacted with 1.76 g (7.21 mmol) of a 9-borabicyclononane dimer in a similar manner to that in Example 56 and then the reaction product was treated with a 1 mol/L aqueous solution of sodium hydroxide and an aqueous solution of hydrogen peroxide to give 1.21 g (99%) of the compound 100.
Fab-MS(m/z); 339$[M+1]^+$

Example 97: Compound 101

The compound 100 (1.19 g, 3.52 mmol) prepared in Example 96 was dissolved in 100 mL of 1,4-dioxane, 1.60 g (7.05 mmol) of DDQ were added thereto and the mixture was heated to reflux for 2 hours with stirring. The reaction solution was cooled with ice and a saturated aqueous solution of sodium hydrogen carbonate was added thereto. The reaction solution was extracted with ethyl acetate, the organic layer was washed with a saturated saline solution, dried over anhydrous sodium sulfate and the solvent was evaporated. The residue was purified by a silica gel column chromatography (chloroform/methanol = 20/1) to give 0.40 g (34%) of the compound 101.
$^1$H-NMR(270MHz, $CD_3OD$, δ); 2.01(m, 2H), 2.73(s, 3H), 3.51(t, J=5.8Hz, 2H), 4.84(m, 2H), 7.28(ddd, J=1.6Hz, 6.4Hz, 8.0Hz, 1H), 7.53-7.63(m, 2H), 7.82(s, 1H), 8.91(d, J=8.0Hz, 1H).

Example 98: Compound 102

The compound 102 (343 mg, 81%) was prepared in a similar manner to that in Example 47 from 392 mg (1.17 mmol) of the compound 101 prepared in Example 97, 0.44 mL (2.9 mmol) of DBU and 1.07 g (2.93 mmol) of bis(p-nitrophenyl)phosphoric azide.
APCI-MS (m/z); 362$[M+1]^+$

Example 99: Compound 103

The compound 102 (250 mg, 0.691 mmol) prepared in Example 98 was dissolved in a mixed solvent of 25 mL of ethanol and 15 mL of tetrahydrofuran, then 249 mg of lead-poisoned 5% palladium-calcium carbonate (Lindlar catalyst) was added thereto and the mixture was stirred at room temperature for 6 hours in a hydrogen atmosphere. After the catalyst was filtered off, the solvent was evaporated. The residue was subjected to trituration with chloroform to give 157 mg (68%) of the compound 103.
$^1$H-NMR(270MHz, DMSO-$d_6$, δ); 1.85(m, 2H), 2.54(t, J=7.1Hz, 2H), 2.73(s, 3H), 4.58(t, J=7.2Hz, 2H), 7.39(dd, J=7.1Hz, 7.8Hz, 1H), 7.66(m, 1H), 7.81(d, J=8.2Hz, 1H), 8.00(s, 1H), 8-94(d, J=7.8Hz, 1H).
APCI-MS(m/z); 334$[M-1]^-$

Example 100: Compound 104

1-Allyl-2-(2-butyrylvinyl)indole (571 mg, 2.25 mmol) was reacted with 689 mg (6.79 mmol) of maleimide in a similar manner to that in the step 2 of Example 1 and then treated with 730 mg (3.22 mmol) of DDQ at room temperature

in a similar manner to that in Example 6 to give 361 mg (65%) of the compound 104.
$^1$H-NMR(270MHz, DMSO-$d_6$, δ); 0.96(t, J=7.3Hz, 3H), 1.67(m, 2H), 3.05(t, J=7.1Hz, 2H), 4.95(d, J=17.1Hz, 1H), 5.13 (d, J=10.2Hz, 1H), 5.20(m, 2H), 6.01(m, 1H), 7.40(dd, J=7.0Hz, 8.1Hz, 1H), 7.66(dd, J=7.0Hz, 8.5Hz, 1H), 7.74(d, J=8.5Hz, 1H), 7.94(s, 1H), 8.93(d, J=8.1Hz, 1H), 11.34(s, 1H).
APCI-MS(m/z); 347[M+1]$^+$

Example 101: Compound 105 and compound 106

The compound 104 (356 mg, 1.03 mmol) prepared in Example 100 was reacted with 502 mg (2.24 mmol) of 9-borabicyclononane dimer in a similar manner to that in Example 56 and then the reaction product was treated with a 1 mol/L aqueous solution of sodium hydroxide and an aqueous solution of hydrogen peroxide to give 121 mg (32%) of the compound 105 and 117 mg (31%) of the compound 106.

Compound 105

APCI-MS (m/z); 365[M+1]$^+$

Compound 106

APCI-MS (m/z); 365[M+1]$^+$

Example 102: Compound 107

The compound 107 (122 mg, 65%) was prepared in a similar manner to that in Example 47 from 174 mg (0.477 mmol) of the compound 105 prepared in Example 101, 0.178 mL (1.19 mmol) of DBU and 440 mg (1.21 mmol) of bis(p-nitrophenyl)phosphoric azide.
APCI-MS (m/z); 388 [M+ 1]$^+$

Example 103: Compound 108 and a succinate of the compound 108

The compound 107 (119 mg, 0.307 mmol) prepared in Example 102 was subjected to a catalytic reduction in a hydrogen atmosphere using 123 mg of lead-poisoned 5% palladium-calcium carbonate (Lindlar catalyst) in a similar manner to that in Example 99 to give 62 mg (55%) of the compound 108.

The resulting compound 108 (27 mg, 0.073 mmol) and 8.6 mg (0.073 mmol) of succinic acid were dissolved in 1 mL of a mixed solvent of chloroform, methanol and water (8/3/0.3) and then the solvents were evaporated. The residue was subjected to trituration with chloroform to give 22 mg (51%) of a succinate of the compound 108.
$^1$H-NMR(270MHz, DMSO-$d_6$, δ); 0.98(t, J=7.4Hz, 3H), 1.69(m, 2H), 2.04(m, 2H), 2.30(s, 7.2H), 2.82(m, 2H), 3.06(t, J=7.2Hz, 2H), 4.63(t, J=7.0Hz, 2H), 7.43(dd, J=7.2Hz, 7.5Hz, 1H), 7.70(dd, J=7.2Hz, 8.2Hz, 1H), 7.85(d, J=8.2Hz, 1H), 8.04(s, 1H), 8.94(d, J=7.5Hz, 1H).
APCI-MS(m/z); 362[M-1]$^-$

Example 104: Compound 109

The compound 102 (10 mg, 0.028 mmol) prepared in Example 98 was dissolved in 0.5 mL of tetrahydrofuran, then 8 mg (0.03 mmol) of triphenylphosphine dissolved in 0.5 mL of tetrahydrofuran was added thereto and the mixture was stirred at room temperature for 3.5 hours. Water (0.75 mL) was added to the reaction solution and the mixture was stirred at room temperature for 3 hours. A solution of 15 mg (0.057 mmol) of triphenylphosphine dissolved in 1 mL of tetrahydrofuran and 1.5 mL of water were further added to the reaction solution, the mixture was stirred at room temperature for 15.5 hours and the solvent was evaporated. The residue was purified by a preparative thin-layer chromatography [ammonia-containing chloroform (a chloroform layer after mixing chloroform with a 28% aqueous ammonia in a ratio of 8/1)/methanol = 8/1] to give 5.4 mg (62%) of the compound 109.
$^1$H-NMR(270MHz, DMSO-$d_6$, δ); 2.08(s, 6H), 2.10(m, 4H), 3.28(m, 4H), 4.33(m, 4H), 7.16(s, 2H), 7.33(m, 2H), 7.42 (d, J=8.1Hz, 2H), 7.56(m, 2H), 8.73(d, J=7.4Hz, 2H).
APCI-MS(m/z); 635[M+1]$^+$

Example 105: Tablets

A tablet having the following ingredients was prepared by a conventional method.

| | |
|---|---|
| Compound 68 | 5 mg |
| Lactose | 60 mg |
| Potato starch | 30 mg |
| Polyvinyl alcohol | 2 mg |
| Magnesium stearate | 1 mg |
| Tar dye | small amount |

Example 106: Granules

A granular preparation having the following ingredients was prepared by a conventional method.

| | |
|---|---|
| Compound 66 | 5 mg |
| Lactose | 280 mg |

Example 107: Syrup

A syrup preparation having the following ingredients was prepared by a conventional method.

| | |
|---|---|
| Compound 69 | 1 mg |
| Pure sucrose | 4000 mg |
| Ethyl p-hydroxybenzoate | 40 mg |
| Propyl p-hydroxybenzoate | 10 mg |
| Strawberry flavor | 0.1 cc |

Water was added thereto so as to make the total volume 100 cc.

Industrial Applicability

In accordance with the present invention, there is provided an indole derivative or a pharmaceutically acceptable salt thereof which is useful for the treatment of a malignant tumor or a brain neurodegenerative disease.

## Claims

**1.** An indole derivative represented by the formula (I) or a pharmaceutically acceptable salt thereof:

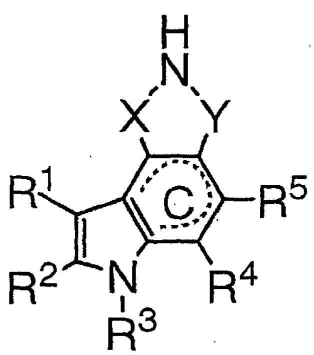

(I)

<<wherein

C ring represents a benzene ring or a cyclohexene ring;
X and Y are the same or different and each represents $-CH_2-$, -CH(OH)-, $-CH(OR^X)-$ (wherein $R^X$ represents

lower alkyl), -CH($SR^Y$)- (wherein $R^Y$ represents lower alkyl) or carbonyl;
$R^1$ and $R^2$ are the same or different and each represents a hydrogen atom, substituted or unsubstituted lower alkyl, substituted or unsubstituted lower alkenyl or substituted or unsubstituted lower alkanoyl or $R^1$ and $R^2$ form a benzene ring together with two carbon atoms, each being adjacent thereto, respectively; and
$R^3$ represents a hydrogen atom, substituted or unsubstituted lower alkyl, substituted or unsubstituted lower alkenyl, substituted or unsubstituted aralkyl, substituted or unsubstituted lower alkanoyl, substituted or unsubstituted aroyl, or is combined with $R^5$ to form, the formula (T)

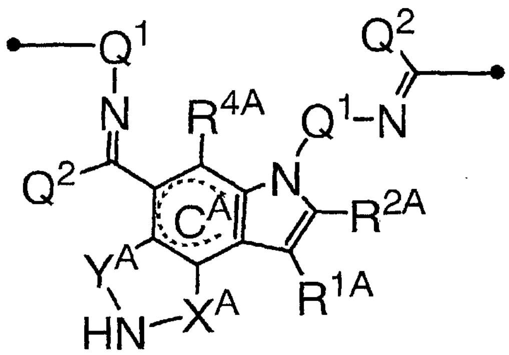

(T)

<wherein

$C^A$ ring represents a benzene ring or a cyclohexene ring;
$X^A$ and $Y^A$ are the same or different and each represents $-CH_2-$, -CH(OH)-, -CH($OR^{XA}$)- (wherein $R^{XA}$ represents lower alkyl), -CH($SR^{YA}$)- (wherein $R^{YA}$ represents lower alkyl) or carbonyl;
$R^{1A}$ and $R^{2A}$ are the same or different and each represents a hydrogen atom, substituted or unsubstituted lower alkyl, substituted or unsubstituted lower alkenyl or substituted or unsubstituted lower alkanoyl or $R^{1A}$ and $R^{2A}$ form a benzene ring together with two carbon atoms, each being adjacent thereto, respectively;
$R^{4A}$ represents a hydrogen atom, substituted or unsubstituted lower alkyl, substituted or unsubstituted lower alkenyl, substituted or unsubstituted lower alkanoyl, substituted or unsubstituted aroyl, substituted or unsubstituted aryl, a substituted or unsubstituted heterocyclic group, $-NR^6R^7$ [wherein $R^6$ and $R^7$ are the same or different and each represents a hydrogen atom, substituted or unsubstituted lower alkyl, lower alkoxycarbonyl or $-CONR^8R^9$ (wherein $R^8$ and $R^9$ are the same or different and each represents a hydrogen atom, substituted or unsubstituted lower alkyl, substituted or unsubstituted aryl or a substituted or unsubstituted heterocyclic group)], $-COR^{10}$ {wherein $R^{10}$ represents hydroxy, lower alkoxy or $-NR^{11}R^{12}$ [wherein $R^{11}$ and $R^{12}$ are the same or different and each represents a hydrogen atom, substituted or unsubstituted lower alkyl, cycloalkyl, substituted or unsubstituted aralkyl, substituted or unsubstituted aryl, a substituted or unsubstituted heterocyclic group, lower alkylthio or an amino acid residue (wherein an amino group of the amino acid residue may be protected with a protective group) or $R^{11}$ and $R^{12}$ form a heterocyclic group together with the adjacent nitrogen atom]}, $-CO(NH)NR^{13}R^{14}$ [wherein $R^{13}$ and $R^{14}$ are the same or different and each represents a hydrogen atom or $-COR^{15}$ (wherein $R^{15}$ represents substituted or unsubstituted lower alkyl, substituted or unsubstituted aryl or a substituted or unsubstituted heterocyclic group) or $R^{13}$ and $R^{14}$ form a heterocyclic group together with the adjacent nitrogen atom], $-CH=NOR^{16}$ (wherein $R^{16}$ represents a hydrogen atom, substituted or unsubstituted lower alkyl, substituted or unsubstituted lower alkenyl, substituted or unsubstituted aralkyl, substituted or unsubstituted aryl or a substituted or unsubstituted heterocyclic group) or $-CH=NNR^{17}R^{18}$ [wherein $R^{17}$ and $R^{18}$ are the same or different and each represents a hydrogen atom, substituted or unsubstituted lower alkyl, substituted or unsubstituted aralkyl, substituted or unsubstituted aryl, a substituted or unsubstituted heterocyclic group or $-CONR^{11A}R^{12A}$ (wherein $R^{11A}$ and $R^{12A}$ have the same meanings as the above $R^{11}$ and $R^{12}$, respectively) or $R^{17}$ and $R^{18}$ form a heterocyclic group together with the adjacent nitrogen atom];
$Q^1$ represents substituted or unsubstituted lower alkylene; and
$Q^2$ represents a hydrogen atom or substituted or unsubstituted lower alkyl>, provided that

(A): when $R^3$ represents a hydrogen atom, substituted or unsubstituted lower alkyl, substituted or unsubstituted lower alkenyl, substituted or unsubstituted aralkyl, substituted or unsubstituted lower alkanoyl or substituted or unsubstituted aroyl, and also $R^1$ and $R^2$ are the same or different and each represents a hydrogen atom, substituted or unsubstituted lower alkyl, substituted or unsubstituted lower alkenyl or substituted or unsubstituted lower alkanoyl,

then $R^4$ and $R^5$ are the same or different and each represents a hydrogen atom, substituted or unsubstituted lower alkyl, substituted or unsubstituted lower alkenyl, substituted or unsubstituted lower alkanoyl, substituted or unsubstituted aroyl, substituted or unsubstituted aryl, a substituted or unsubstituted heterocyclic group, $-NR^{6A}R^{7A}$ (wherein $R^{6A}$ and $R^{7A}$ have the same meanings as the above $R^6$ and $R^7$, respectively), $-COR^{10A}$ (wherein $R^{10A}$ has the same meaning as the above $R^{10}$), $-CO(NH)NR^{13A}R^{14A}$ (wherein $R^{13A}$ and $R^{14A}$ have the same meanings as the above $R^{13}$ and $R^{14}$, respectively), $-CH=NOR^{16A}$ (wherein $R^{16A}$ has the same meaning as the above $R^{16}$) or $-CH=NNR^{17A}R^{18A}$ (wherein $R^{17A}$ and $R^{18A}$ have the same meanings as the above $R^{17}$ and $R^{18}$, respectively);

(B): when $R^3$ represents a hydrogen atom, substituted or unsubstituted lower alkyl, substituted or unsubstituted lower alkenyl, substituted or unsubstituted aralkyl, substituted or unsubstituted lower alkanoyl or substituted or unsubstituted aroyl and also when $R^1$ and $R^2$ form a benzene ring together with the two carbon atoms, each being adjacent thereto, respectively,

then $R^4$ and $R^5$ are the same or different and each represents a hydrogen atom, substituted or unsubstituted lower alkyl, substituted or unsubstituted lower alkenyl, substituted or unsubstituted lower alkanoyl, substituted or unsubstituted aroyl, a substituted or unsubstituted aliphatic heterocyclic group, oxazolinyl, $-NR^{6A}R^{7A}$ (wherein $R^{6A}$ and $R^{7A}$ have the same meanings as the above $R^6$ and $R^7$, respectively), $-COR^{10A}$ (wherein $R^{10A}$ has the same meaning as the above $R^{10}$), $-CO(NH)NR^{13A}R^{14A}$ (wherein $R^{13A}$ and $R^{14A}$ have the same meanings as the above $R^{13}$ and $R^{14}$, respectively), $-CH=NOR^{16A}$ (wherein $R^{16A}$ has the same meaning as the above $R^{16}$) or $-CH=NNR^{17A}R^{18A}$ (wherein $R^{17A}$ and $R^{18A}$ have the same meanings as the above $R^{17}$ and $R^{18}$, respectively); and

(C): when $R^3$ and $R^5$ are combined to form the formula (T), then $R^4$ represents a hydrogen atom, substituted or unsubstituted lower alkyl, substituted or unsubstituted lower alkenyl, substituted or unsubstituted lower alkanoyl, substituted or unsubstituted aroyl, substituted or unsubstituted aryl, a substituted or unsubstituted heterocyclic group, $-NR^{6A}R^{7A}$ (wherein $R^{6A}$ and $R^{7A}$ have the same meanings as the above $R^6$ and $R^7$, respectively), $-COR^{10A}$ (wherein $R^{10A}$ has the same meaning as the above $R^{10}$), $-CO(NH)NR^{13A}R^{14A}$ (wherein $R^{13A}$ and $R^{14A}$ have the same meanings as the above $R^{13}$ and $R^{14}$, respectively) , $-CH=NOR^{16A}$ (wherein $R^{16A}$ has the samemeaning as the above $R^{16}$) or $-CH=NNR^{17A}R^{18A}$ (wherein $R^{17A}$ and $R^{18A}$ have the same meanings as the above $R^{17}$ and $R^{18}$, respectively)>>.

**2.** The indole derivative or the pharmaceutically acceptable salt thereof according to claim 1, wherein $R^3$ is a hydrogen atom, substituted or unsubstituted lower alkyl, substituted or unsubstituted lower alkenyl, substituted or unsubstituted aralkyl, substituted or unsubstituted lower alkanoyl or substituted or unsubstituted aroyl.

**3.** The indole derivative or the pharmaceutically acceptable salt thereof according to claim 1, wherein $R^3$ is substituted or unsubstituted lower alkyl.

**4.** The indole derivative or the pharmaceutically acceptable salt thereof according to any of claims 1 to 3, wherein $R^1$ and $R^2$ form a benzene ring together with the two carbon atoms , each being adjacent thereto, respectively.

**5.** The indole derivative or the pharmaceutically acceptable salt thereof according to any of claims 1 to 3, wherein $R^1$ and $R^2$ are the same or different and each is a hydrogen atom, substituted or unsubstituted lower alkyl, substituted or unsubstituted lower alkenyl or substituted or unsubstituted lower alkanoyl.

**6.** The indole derivative or the pharmaceutically acceptable salt thereof according to any of claims 1 to 5, wherein the C ring is a benzene ring.

**7.** The indole derivative or the pharmaceutically acceptable salt thereof according to claim 1, wherein $R^3$ and $R^5$ are combined to form, the formula (T).

**8.** An anti-tumor agent, which comprises the indole derivative or the pharmaceutically acceptable salt thereof according to any of claims 1 to 7 as an active ingredient.

**9.** A therapeutic agent for a brain neurodegenerative disease, which comprises the indole derivative or the pharmaceutically acceptable salt thereof according to any of claim 1 to 7 as an active ingredient.

**10.** An enhancer for activity of an anti-tumor agent, which comprises the indole derivative or the pharmaceutically acceptable salt thereof according to any of claims 1 to 7 as an active ingredient.

**11.** An inhibitor for a cyclin-dependent kinase 2 (CDK2), which comprises the indole derivative or the pharmaceutically acceptable salt thereof according to any of claims 1 to 7 as an active ingredient.

**12.** An agent for abrogating accumulation action at G2 phase and/or S phase, which comprises the indole derivative or the pharmaceutically acceptable salt thereof according to any of claims 1 to 7 as an active ingredient.

**13.** A pharmaceutical composition comprising the indole derivative or the pharmaceutically acceptable salt thereof according to any of the claims 1 to 7 as an active ingredient.

**14.** Use of the indole derivative or the pharmaceutically acceptable salt thereof according to any of claims 1 to 7 for the manufacture of an anti-tumor agent.

**15.** Use of the indole derivative or the pharmaceutically acceptable salt thereof according to any of claims 1 to 7 for the manufacture of an enhancer for activity of an anti-tumor agent.

**16.** Use of the indole derivative or the pharmaceutically acceptable salt thereof according to any of claims 1 to 7 for the manufacture of a therapeutic agent for a brain neurodegenerative disease.

**17.** Use of the indole derivative or the pharmaceutically acceptable salt thereof according to any of claims 1 to 7 for the manufacture of an inhibitor for CDK2.

**18.** Use of the indole derivative or the pharmaceutically acceptable salt thereof according to any of claims 1 to 7 for the manufacture of an agent for abrogating accumulation action at G2 phase and/or S phase.

**19.** Amethod of treating amalignant tumor, which comprises administering an effective amount of the indole derivative or the pharmaceutically acceptable salt thereof according to any of claims 1 to 7.

**20.** Amethod of treating a brain neurbdegenerative disease, which comprises administering an effective amount of the indole derivative or the pharmaceutically acceptable salt thereof according to any of claims 1 to 7.

**21.** A method of treating a disease in which CDK2 is concerned, which comprises administering an effective amount of the indole derivative or the pharmaceutically acceptable salt thereof according to any of claims 1 to 7.

**22.** Amethod of enhancing activity for an anti-tumor agent, which comprises administering an effective amount of the indole derivative or the pharmaceutically acceptable salt thereof according to any of claims 1 to 7.

**23.** A method of abrogating accumulation action at G2 phase and/or S phase, which comprises administering an effective amount of the indole derivative or the pharmaceutically acceptable salt thereof according to any of claims 1 to 7.

**INTERNATIONAL SEARCH REPORT**

International application No.
PCT/JP02/13172

A. CLASSIFICATION OF SUBJECT MATTER
Int.Cl$^7$ C07D487/04, 487/22, A61K31/407, 31/409, 31/4196, 31/422, 31/4439, 31/497, 31/5377, 31/541, A61P25/00, 35/00, 43/00

According to International Patent Classification (IPC) or to both national classification and IPC

B. FIELDS SEARCHED

Minimum documentation searched (classification system followed by classification symbols)
Int.Cl$^7$ C07D487/04, 487/22, A61K31/407, 31/409, 31/4196, 31/422, 31/4439, 31/497, 31/5377, 31/541, A61P25/00, 35/00, 43/00

Documentation searched other than minimum documentation to the extent that such documents are included in the fields searched

Electronic data base consulted during the international search (name of data base and, where practicable, search terms used)
CAPLUS(STN), CAOLD(STN), REGISTRY(STN), WPI/L(DIALOG)

C. DOCUMENTS CONSIDERED TO BE RELEVANT

| Category* | Citation of document, with indication, where appropriate, of the relevant passages | Relevant to claim No. |
|---|---|---|
| X<br><br>Y | WO 01/85686 A2 (CEPHALON, INC.), 15 November, 2001 (15.11.01), Claims; compounds stated in examples; page 74, lines 12 to 16; page 77, line 23 to page 78, line 9<br>& US 2002/0028815 A1 | 1-2,4,6, 8-9,13-14,16<br>1-6,8-9, 11-14,16-18 |
| X<br><br>Y | US 5721267 A (SYNTEX INC.), 24 February, 1998 (24.02.98), All pages<br>& EP 695755 A1 & JP 8-59666 A | 1-4,6,8, 13-14<br>1-6,8-9, 11-14,16-18 |
| X<br><br>Y | US 4912107 A (GOEDECKE AG), 27 March, 1990 (27.03.90), Claims; compounds stated in examples; column 10, lines 37 to 49<br>& EP 362695 A1 & JP 2-142791 A | 1-4,6,9,13, 16<br>1-6,8-9, 11-14,16-18 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 January, 2003 (30.01.03) | 04 March, 2003 (04.03.03) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/13172

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 5-202048 A (Toyama Chemical Co., Ltd.),<br>10 August, 1993 (10.08.93),<br>Compound 4 stated in Par. No. [0011]; Par. Nos. [0030] to [0034]<br>(Family: none) | 1-2,5-6<br>8,13-14 |
| Y | JP 4-230385 A (Toyama Chemical Co., Ltd.),<br>19 August, 1992 (19.08.92),<br>Claims; Par. Nos. [0017] to [0020]<br>(Family: none) | 1-4,6,8,<br>13-14 |
| X | US 6063803 A (ORTHO-MCNEIL PHARMACEUTICAL INC.),<br>16 May, 2000 (16.05.00),<br>Examples 84, 85 stated in column 32<br>& WO 99/65911 A1 | 1-2,4,13 |
| Y | WO 99/15500 A1 (GLAXO GROUP LTD.),<br>01 April, 1999 (01.04.99),<br>Claims; compound 66 stated in tables on page 20; table 2 stated on page 103<br>& EP 1009738 A1 & JP 2001-517652 A | 11-12,17-18 |
| X | Marta Adeva et al., Open analogues of arcyriaflavin A. Synthesis through Diels-Alder reaction between maleimides and 1-aryl-3-tert-butyldimetylsiloxy-1,3-butadienes, Journal of Organic Chemistry, 2000, Vol.65, pages 3387 to 3394<br>Compounds 6c, 8c stated in scheme 2 on page 3389; page 3387, right column | 1-2,4,6,8,<br>13-14 |
| X | David P. Rotella et al., The effect of pyrrolo [3,4-c] carbazole derivatives on spinal cord ChAT activity, Bioorganic & Medicinal Chemistry Letters, 1995, Vol.5, No.11, pages 1167 to 1170<br>Compounds 3, 5, 6, 9 stated on page 1168; table 1 stated on page 1169 | 1-4,6,9,13,<br>16 |
| X | Christopher J. Moody, et al., Synthesis of the staurosporine aglycone, Journal of Organic Chemistry, 1992, Vol.57, pages 2105 to 2114<br>Compounds 20, 21 stated on page 2106; compounds 36, 37 stated on page 2108 | 1-2,4,6,13 |
| X | Mahboobi, von Siavosh et al., Synthesis of carbazole derivatives. I. Reaction of 3-(2-nitroethenyl)indole -2-malonic acid esters with Michael-acceptors, Archive der Pharmazie (Weinheim, Germany), 1995, Vol.328, No.1, pages 29 to 38<br>Compound 33 stated on page 33 | 1-2,4,13 |
| A | Yukimasa SHIOTSU et al., Chemoprotective effects of KF41399, a derivative of carbazole compounds, on nimustine-induced thrombocytopenia, Blood, 2000, Vol.95, No.12, pages 3771 to 3780 | 8,10-15,<br>17-18 |

Form PCT/ISA/210 (continuation of second sheet) (July 1998)

**INTERNATIONAL SEARCH REPORT**

International application No.
PCT/JP02/13172

**Box I Observations where certain claims were found unsearchable (Continuation of item 2 of first sheet)**

This international search report has not been established in respect of certain claims under Article 17(2)(a) for the following reasons:

1. [X] Claims Nos.: 19 to 23
because they relate to subject matter not required to be searched by this Authority, namely:
Claims 19 to 23 pertain to a method for treatment of the human body by therapy and thus relate to a subject matter which this International Searching Authority is not required, under the provisions of Article 17(2)(a)(i) of
(continued to extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)

**INTERNATIONAL SEARCH REPORT**

International application No.
PCT/JP02/13172

Continuation of Box No.I-1 of continuation of first sheet(1)

the PCT and Rule 39.1(iv) of the Regulations under the PCT, to make an international search.

Form PCT/ISA/210 (extra sheet) (July 1998)